# EUROPEAN PATENT SPECIFICATION

(11) **EP 4 204 397 B1**
(45) Date of publication and mention of the grant of the patent: **03.12.2025**
(21) Application number: 21766627.0
(22) Date of filing: 24.08.2021
(51) Int. Cl.: C07C 323/60, C07D 277/46, C07D 235/30, A61P 31/04, A61K 31/4184, A61K 31/4406, A61K 31/428, A61K 31/426, A61K 31/167, A61K 31/10, A61K 31/095, C07F 9/40, C07F 9/38, C07D 333/36, C07D 277/82, C07D 213/74, C07C 235/80, C07C 259/06, C07F 9/58, C07F 9/59

(54) **INHIBITORS OF PSEUDOMONAS AERUGINOSA VIRULENCE FACTOR LASB**
N-ARYL UND N-HETEROARYL- SUBSTITUIERTE AMIDEN ALS INHIBITOREN DES PSEUDOMONAS-AERUGINOSA-VIRULENZFAKTORS LASB
LES AMIDES N-ARYLES- ET N-HÉTÉROARYLES COMME INHIBITEURS DE LASB FACTEUR DE VIRULENCE PSEUDOMONAS AERUGINOSA

(30) Priority: 25.08.2020 EP 20192608
(43) Date of publication of application: 05.07.2023
(73) Proprietor: Helmholtz-Zentrum für Infektionsforschung GmbH, 38124 Braunschweig (DE); Universität des Saarlandes, 66123 Saarbrücken (DE)
(72) Inventor: DUCHO, Christian, 66123 Saarbrücken (DE); HARTMANN, Rolf W., 38124 Braunschweig (DE); HAUPENTHAL, Jörg, 38124 Braunschweig (DE); HIRSCH, Anna K. H., 38124 Braunschweig (DE); KANY, Andreas, 38124 Braunschweig (DE); KAYA, Cansu, 38124 Braunschweig (DE); KONSTANTINOVIC, Jelena, 38124 Braunschweig (DE); VOOS, Katrin, 66123 Saarbrücken (DE); WALTER, Isabell, 38124 Braunschweig (DE); YAHIAOUI, Samir, 38124 Braunschweig (DE); ABDELSAMINE, Ahmed Saad, 38124 Braunschweig (DE); SCHÜTZ, Christian, 38124 Braunschweig (DE); JUMDE, Ravindra, 38124 Braunschweig (DE); KIEFER, Alexander, 38124 Braunschweig (DE)
(74) Representative: Forstmeyer, Dietmar
(86) International application number: PCT/EP2021/073381
(87) International publication number: WO 2022/043322

(56) References cited:
- EP-A1- 0 475 531
- WO-A1-2008/153857
- WO-A1-99/11608
- WO-A2-2008/133896
- DE-C- 356 912
- US-A- 5 801 274
- US-A1- 2010 048 513
- US-B2- 7 977 367
- S. SINGH ET AL: "Synthesis, characterization and biocidal activities of thioamides and their mixedligand copper complexes", CHEMICAL ABSTRACTS AN: 2008:117817, vol. 150, 1 January 2008 (2008-01-01), XP055770281
- S SINGH ET AL: "Synthesis, characterization and biological studies of some N, O/S containing compounds", CHEMICAL ABSTRACTS AN: 2005:1201316, 1 January 2005 (2005-01-01), XP055770296
- ANNA INYUTINA ET AL: "A Novel Approach to Substituted [alpha]-Carbamoyl Phosphonates: Useful Reagents for the Horner-Wadsworth-Emmons Olefination", SYNLETT, vol. 31, no. 15, 20 July 2020 (2020-07-20), DE, pages 1487 - 1490, XP055769650, ISSN: 0936-5214, DOI: 10.1055/s-0040-1707200
- ANNA INYUTINA ET AL: "A Novel Approach to Substituted [alpha]-Carbamoyl Phosphonates: Useful Reagents for the Horner-Wadsworth-Emmons Olefination", SUPPLEMENTARY INFORMATION TO SYNLETT VOL 31(15) PP 1487-1490 (20.07.2020), 20 July 2020 (2020-07-20), pages 1 - 44, XP055769653, Retrieved from the Internet <URL:https://www.thieme-connect.de/media/synlett/202015/supmat/sup_st-2020-b0288-l_10-1055_s-0040-1707200.pdf> [retrieved on 20210128], DOI: 10.1055/s-0040-1707200
- CHRISTIANA M. ADEYEMI ET AL: "Synthesis and anti-parasitic activity of C -benzylated ( N -arylcarbamoyl)alkylphosphonate esters", TETRAHEDRON, vol. 73, no. 13, 1 March 2017 (2017-03-01), AMSTERDAM, NL, pages 1661 - 1667, XP055769682, ISSN: 0040-4020, DOI: 10.1016/j.tet.2017.01.045
- MISRA R N ET AL: "The use of some thiolactic anilides in inorganic analysis", JOURNAL OF THE INDIAN CHEMICAL SOCIETY, INDIAN CHEMICAL SOCIETY, IN, vol. 33, no. 7, 1 January 1956 (1956-01-01), pages 523 - 526, XP009525218, ISSN: 0019-4522
- GILM KAMAI AND E SH BASMANOV: "Some new phosphonic esters and their derivatives", JOURNAL OF GENERAL CHEMISTRY USSR, CONSULTANTS BUREAU, NEW YORK, NY, US, vol. 21, no. 10, 1 October 1951 (1951-10-01), pages 2449 - 2453, XP009525219, ISSN: 0022-1279
- H BECKURTS ET AL: "Über die Toluidide der Thiooxybuttersäuren", ARCHIV DER PHARMAZIE, vol. 253, 1 January 1915 (1915-01-01), pages 155 - 181, XP055769722
- ANDREAS M. KANY ET AL: "Binding Mode Characterization and Early in Vivo Evaluation of Fragment-Like Thiols as Inhibitors of the Virulence Factor LasB from Pseudomonas aeruginosa", ACS INFECTIOUS DISEASES, vol. 4, no. 6, 27 February 2018 (2018-02-27), US, pages 988 - 997, XP055770127, ISSN: 2373-8227, DOI: 10.1021/acsinfecdis.8b00010
- JIE ZHU ET AL: "Disarming Pseudomonas aeruginosa Virulence Factor LasB by Leveraging a Caenorhabditis elegans Infection Model", CHEMISTRY AND BIOLOGY., vol. 22, no. 4, 1 April 2015 (2015-04-01), GB, pages 483 - 491, XP055770141, ISSN: 1074-5521, DOI: 10.1016/j.chembiol.2015.03.012
- OYA M ET AL: "THIOL COMPOUNDS. I. SYNTHESIS AND ANTIHYPERTENSIVE ACTIVITY OF MERCAPTOACYLAMINO ACIDS", CHEMICAL AND PHARMACEUTICAL BULLETIN, PHARMACEUTICAL SOCIETY OF JAPAN, JP, vol. 29, no. 1, 1 January 1981 (1981-01-01), pages 63 - 70, XP002019775, ISSN: 0009-2363
- FLIPO ET AL: "A library of novel hydroxamic acids targeting the metallo-protease family: Design, parallel synthesis and screening", BIOORGANIC, ELSEVIER, AMSTERDAM, NL, vol. 15, no. 1, 15 November 2006 (2006-11-15), pages 63 - 76, XP005764588, ISSN: 0968-0896, DOI: 10.1016/J.BMC.2006.10.010

## Description

The present invention relates to novel inhibitors of the *Pseudomonas aeruginosa* virulence factor LasB. These compounds are useful in the treatment of bacterial infections, especially caused by *P. aeruginosa.*

*P. aeruginosa* is a Gram-negative bacterium, which is ranked by the WHO as one of the most critical pathogens today (World Health Organization. Global Priority List of Antibiotic-Resistant Bacteria to Guide Research, Discovery, and Development of New Antibiotics. WHO **2017**). This opportunistic bacterium causes around 10% of hospital-acquired infections and has a high occurrence among immunocompromised and cystic-fibrosis patients (Magill, S. S.; Edwards, J. R.; Bamberg, W.; Beldavs, Z. G.; Dumyati, G.; Kainer, M. A.; Lynfield, R.; Maloney, M.; McAllister-Hollod, L.; Nadle, J.; et al. N. Engl. J. Med. 2014, 370, 1198-1208; Richards, M. J.; Edwards, J. R.; Culver, D. H.; Gaynes, R. P. Pediatrics 1999, 103, e39; Valenza, G.; Tappe, D.; Turnwald, D.; Frosch, M.; König, C.; Hebestreit, H.; Abele-Horn, M. J. Cyst. Fibros. 2008, 7, 123-127; Sordé, R.; Pahissa, A.; Rello, J. Infect. Drug Resist. 2011, 4, 31-41). The development of potent antibiotics is urgently needed due to the lack of efficient therapeutics on the market (Mesaros, N.; Nordmann, P.; Plésiat, P.; Roussel-Delvallez, M.; Eldere, J. Van; Glupczynski, Y.; Laethem, Y. Van; Jacobs, F.; Lebecque, P.; Malfroot, A.; et al. Clin. Microbiol. Infect. 2007, 13, 560-578; Taubes, G. Science 2008, 321, 356-361). This task is complicated by the high intrinsic resistance of the pathogen (Hancock, R. E. W.; Speert, D. P. Drug Resist. Updat. 2000, 3, 247-255; Strateva, T.; Yordanov, D. J. Med. Microbiol. 2009, 58, 1133-1148).

*P. aeruginosa* has a particularly low permeability of the outer membrane, preventing the entrance of antibiotics into the cell (Nikaido, H.; Yoshimura, F. J. Bacteriol. 1982, 152, 636-642). Additionally, its efflux pumps efficiently transport undesired antimicrobials out of the cell and its inducible chromosomal β-lactamases are able to inactivate the corresponding β-lactam antibiotics (Pos, K. M. Biochim. Biophys. Acta - Proteins Proteomics 2009, 1794, 782-793; Moreira, M. A. S.; Souza, E. C. de; Moraes, C. A. de. Brazilian J. Microbiol. 2004, 35, 19-28; Hancock, R. E. W.; Woodruff, W. A. Clin. Infect. Dis. 1988, 10, 770-775; Li, X. Z.; Livermore, D. M.; Nikaido, H. Antimicrob. Agents Chemother. 1994, 38, 1732-1741). An additional difficulty is the rising mutational resistance rate of *P. aeruginosa* strains (Thomson, J. M.; Bonomo, R. A. Curr. Opin. Microbiol. 2005, 8, 518-524). For example, fluoroquinolone and aminoglycoside resistance have reached up to 30% (Gasink, L. B.; Fishman, N. O.; Weiner, M. G.; Nachamkin, I.; Bilker, W. B.; Lautenbach, E. Am. J. Med. 2006, 119, 19-25; Poole, K. Antimicrob. Agents Chemother. 2005, 49, 479-487). Furthermore, resistances against almost all drugs used for the treatment of infections with P. *aeruginosa* (for example cephalosporins and carbapenems) are described (Obritsch, M. D.; Fish, D. N.; MacLaren, R.; Jung, R. Pharmacotherapy 2005, 25, 1353-1364; ASCP Susceptibility Testing Group. United States Geographic Bacteria Susceptibility Patterns. Am. J. Clin. Pathol. 1996, 106, 275-281). These facts emphasize the urgent need for new therapeutic options.

Besides the traditional strategy to target bacterial viability, recently, special attention has been paid to targeting bacterial virulence as an alternative approach for fighting microbial infections (Dickey, S. W.; Cheung, G. Y. C.; Otto, M. Nat. Rev. Drug Discov. 2017, 16, 457-471; Rasko, D. A.; Sperandio, V. Nat. Rev. Drug Discov. 2010, 9, 117-128). Virulence factors are common among pathogenic bacteria and act by damaging their host or evading its immune response (Strateva, T.; Mitov, I. Ann. Microbiol. 2011, 61, 717-732). Inhibitors of virulence factors reduce bacterial virulence and in this way enable clearance of the pathogen by either the host's immune system or with the help of antibiotics (Heras, B.; Scanlon, M. J.; Martin, J. L. Br. J. Clin. Pharmacol. 2015, 79, 208-215; Clatworthy, A. E.; Pierson, E.; Hung, D. T. Nat. Chem. Biol. 2007, 3, 541-548). Although only a few compounds have reached clinical approval yet, many *in vitro* and *in vivo* studies support the efficacy of this strategy (Wagner, S.; Sommer, R.; Hinsberger, S.; Lu, C.; Hartmann, R. W.; Empting, M.; Titz, A. J. Med. Chem. 2016, 59, 5929-5969). The main advantage of this new approach is the reduced selection pressure on the bacteria and thus the lower risk for resistance development. In addition, these anti-virulence agents do not harm the commensal bacteria.

A well-known anti-virulence target of *P. aeruginosa* is the elastase LasB. This extracellular zinc-containing protease plays a role in the pathogenic invasion of tissues and is thought to be predominantly relevant during acute infections (Liu, P. V. J. Infect. Dis. 1974, 130, S94-S99). It has the ability to break down elastin, which is an important component of lung tissue and blood vessels (Morihara, K.; Tsuzuki, H.; Oka, T.; Inoue, H.; Ebata, M. J. Biol. Chem. 1965, 240, 3295-3304). Additionally, LasB can degrade fibrin, collagen and surfactant proteins in the lung and is also involved in the reduction of the host's immunity by inactivation of human immunoglobulins A and G, cytokines gamma-interferon and tumor necrosis factor α as well as the degradation of the antibacterial peptide LL-37 (Heck, L. W.; Morihara, K.; McRae, W. B.; Miller, E. J. Infect. Immun. 1986, 51, 115-118; Heck, L. W.; Alarcon, P. G.; Kulhavy, R. M.; Morihara, K.; Mestecky, M. W.; Russell, J. F. J. Immunol. 1990, 144, 2253-2257; Holder, I. A.; Wheeler, R. Can. J. Microbiol. 1984, 30, 1118-1124; Galloway, D. R. Mol. Microbiol. 1991, 5, 2315-2321; Parmely, M.; Gale, A.; Clabaugh, M.; Horvat, R.; Zhou, W. Infect. Immun. 1990, 58, 3009-3014; Mariencheck, W. I.; Alcorn, J. F.; Palmer, S. M.; Wright, J. R. Am. J. Respir. Cell Mol. Biol. 2003, 28, 528-537; Schmidtchen, A. et al. Mol. Microbiol. 2002, 46, 157-168).

Since LasB is an attractive anti-virulence target, several LasB inhibitors have been described in the literature up to now: natural products such as streptomyces metalloprotease inhibitor TK-23 (SMPI) from *Streptomyces nigrescens* and phosphoramidon (Oda, K.; Koyama, T.; Murao, S. Biochim. Biophys. Acta 1979, 571, 147-156; Nishino, N.; Powers, J. C. J. Biol. Chem. 1979, 255, 3482-19), small peptides containing metal-chelating motifs such as thiol or hydroxamate groups (Kessler, E.; Israel, M.; Landshman, N.; Chechick, A.; Blumberg, S. Infect. Immun. 1982, 38, 716-723; Cathcart, G. R. A.; Quinn, D.; Greer, B.; Harriott, P.; Lynas, J. F.; Gilmore, B. F.; Walker, B. Antimicrob. Agents Chemother. 2011, 55, 2670-2678; Burns, F. R.; Paterson, C. A.; Gray, R. D.; Wells, J. T. Antimicrob. Agents Chemother. 1990, 34, 2065-2069) and small synthetic molecules with hydroxamate, thiol or mercaptoacetamide groups (Zhu, J.; Cai, X.; Harris, T. L.; Gooyit, M.; Wood, M.; Lardy, M.; Janda, K. D. Chem. Biol. 2015, 22, 483-491; Adekoya, O. A.; Sjøli, S.; Wuxiuer, Y.; Bilto, I.; Marques, S. M.; Santos, M. A.; Nuti, E.; Cercignani, G.; Rossello, A.; Winberg, J. O.; et al. Eur. J. Med. Chem. 2015, 89, 340-348) as well as compounds based on tropolone (Fullagar, J. L.; Garner, A. L.; Struss, A. K.; Day, J. A.; Martin, D. P.; Yu, J.; Cai, X.; Janda, K. D.; Cohen, S. M. Chem. Commun. 2013, 49, 3197-3199).

Recently, a group of *N*-aryl mercaptoacetamides as potent LasB inhibitors has been described (Kany, A. M.; Sikandar, A.; Haupenthal, J.; Yahiaoui, S.; Maurer, C. K.; Proschak, E.; Köhnke, J.; Hartmann, R. W. ACS Infect. Dis. 2018, 4, 988-997). The crystal structure of the most promising compound described therein (compound **36**) revealed the presence of two molecules in the binding pocket. In order to occupy the active site with a single molecule, a series of *N*-benzylamide/*N*-alkylamide derivatives were synthesized. However, this approach failed to improve the inhibitory potency of the initial ligand.

It has been the object of the present invention to provide novel inhibitors of the *P. aeruginosa* virulence factor LasB.

The present invention provides compounds of formula (Ia) 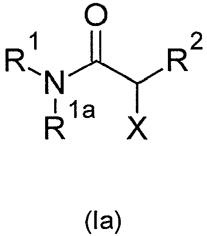 wherein
X is a group of formula -PO(OH)₂, -SH, -C(=O)-NH-OH, an optionally substituted triazolyl group, -SR³, -PO(OH)(OR⁴) or -PO(OR⁴)(OR⁵);
R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group; or a group of formula -CH(R⁶)-C(=O)-NH-R⁷, or a group of formula -C(Me)₂-CH₂-C(=O)-NH-R⁷, or a group of formula -CH(R⁶)-CH₂-C(=O)-NH-R⁷, or a group of formula -CH(R⁶)-R⁸;
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R³ is a group of formula -COR^{3a} or -CON(R^{3b})₂; wherein R^{3a} is an alkyl group, an optionally substituted phenyl group or an optionally substituted benzyl group and R^{3b} is independently selected from hydrogen or an alkyl group, an optionally substituted phenyl group or an optionally substituted benzyl group;
R⁴ is an alkyl group, an optionally substituted phenyl group or an optionally substituted benzyl group;
R⁵ is an alkyl group, an optionally substituted phenyl group or an optionally substituted benzyl group;
R⁶ is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R⁷ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group;
R⁸ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group; and
R^{1a} is hydrogen, or, if R¹ is a group of formula -CH(R⁶)-C(=O)-NH-R⁷, R^{1a} and R⁶ together may be a group of formula -(CH₂)₃- or -(CH₂)₄-;
or a pharmaceutically acceptable salt thereof, for use in the treatment of bacterial infections caused by *P. aeruginosa.*

The present invention further provides compounds of formula (I) 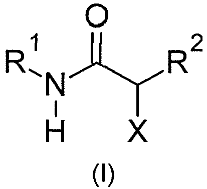 wherein
X is a group of formula -PO(OH)₂, -SH, -C(=O)-NH-OH, an optionally substituted triazolyl group, -SR³, -PO(OH)(OR⁴) or -PO(OR⁴)(OR⁵);
R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group; or a group of formula -CH(R⁶)-C(=O)-NH-R⁷, or a group of formula -C(Me)₂-CH₂-C(=O)-NH-R⁷, or a group of formula -CH(R⁶)-CH₂-C(=O)-NH-R⁷, or a group of formula -CH(R⁶)-R⁸;
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R³ is a group of formula -COR^{3a} or -CON(R^{3b})₂; wherein R^{3a} is an alkyl group, an optionally substituted phenyl group or an optionally substituted benzyl group and R^{3b} is independently selected from hydrogen or an alkyl group, an optionally substituted phenyl group or an optionally substituted benzyl group;
R⁴ is an alkyl group, an optionally substituted phenyl group or an optionally substituted benzyl group;
R⁵ is an alkyl group, an optionally substituted phenyl group or an optionally substituted benzyl group;
R⁶ is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R⁷ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group; and
R⁸ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group;
or a pharmaceutically acceptable salt thereof, for use in the treatment of bacterial infections caused by *P. aeruginosa.*

Preferably, X is a group of formula -PO(OH)₂, -SH, -C(=O)-NH-OH or a triazolyl group.

The present invention moreover provides compounds of formula (I) 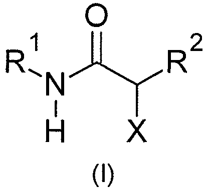 wherein
X is a group of formula -SH, -PO(OH)₂, -SR³, -PO(OH)(OR⁴) or -PO(OR⁴)(OR⁵);
R¹ is an optionally substituted aryl group or an optionally substituted heteroaryl group;
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R³ is a group of formula -COR^{3a} or -CON(R^{3b})₂; wherein R^{3a} is an alkyl group, an optionally substituted phenyl group or an optionally substituted benzyl group and R^{3b} is independently selected from hydrogen or an alkyl group, an optionally substituted phenyl group or an optionally substituted benzyl group;
R⁴ is an alkyl group, an optionally substituted phenyl group or an optionally substituted benzyl group;
R⁵ is an alkyl group, an optionally substituted phenyl group or an optionally substituted benzyl group;
or a pharmaceutically acceptable salt thereof, for use in the treatment of bacterial infections caused by *P. aeruginosa.*

According to a further preferred embodiment, the present invention provides compounds of formula (II) 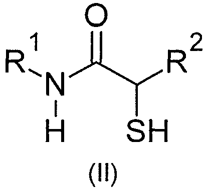 wherein R¹ and R² are as defined above or below; or a pharmaceutically acceptable salt thereof, for use in the treatment of bacterial infections caused by P. aeruginosa.

According to a moreover preferred embodiment, the present invention provides compounds of formula (II) 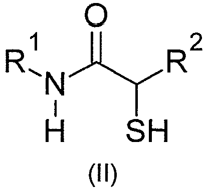 wherein
R¹ is an optionally substituted aryl group or an optionally substituted heteroaryl group; and
R² is an alkyl, alkenyl, alkynyl, heteroalkyl, cycloalkyl, heterocycloalkyl, alkylcycloalkyl,
heteroalkylcycloalkyl, aryl, heteroaryl, aralkyl or heteroaralkyl group, all of which may optionally be substituted;
or a pharmaceutically acceptable salt thereof, for use in the treatment of bacterial infections caused by *P. aeruginosa.*

According to a further preferred embodiment, the present invention provides compounds of formula (III) 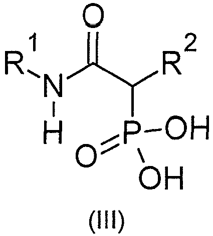 wherein R¹ and R² are as defined above or below; or a pharmaceutically acceptable salt thereof, for use in the treatment of bacterial infections caused by P. aeruginosa.

According to a moreover preferred embodiment, the present invention provides compounds of formula (III) 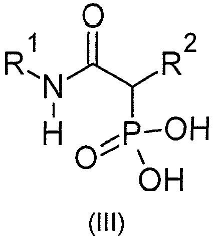 wherein
R¹ is an optionally substituted aryl group or an optionally substituted heteroaryl group; and
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
or a pharmaceutically acceptable salt thereof, for use in the treatment of bacterial infections caused by *P. aeruginosa.*

Furthermore, of these compounds of formula (III), where R² represents a group of formula: -CH₂-CH(CH₃)₂, the compounds *per se* (where R² represents this specific value) also form part of the presently claimed invention, as do the pharmaceutical compositions comprising them and these compounds for use in the treatment of bacterial infections in general.

According to a further preferred embodiment, the present invention provides compounds of formula (IV) 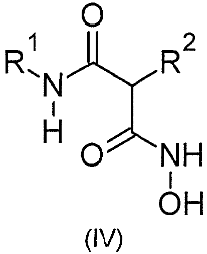 wherein R¹ and R² are as defined above or below; or a pharmaceutically acceptable salt thereof, for use in the treatment of bacterial infections caused by *P. aeruginosa.*

According to a moreover preferred embodiment, the present invention provides compounds of formula (V) 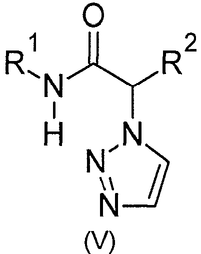 wherein R¹ and R² are as defined above or below; or a pharmaceutically acceptable salt thereof, for use in the treatment of bacterial infections caused by *P. aeruginosa.*

According to a further preferred embodiment, the present invention provides compounds of formula (VI) 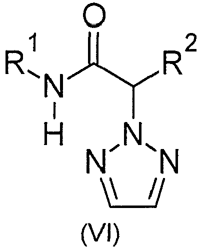 wherein R¹ and R² are as defined above or below; or a pharmaceutically acceptable salt thereof, for use in the treatment of bacterial infections caused by *P. aeruginosa.*

The following preferred embodiments independently apply to compounds of formulas (I), (Ia), (II), (III), (IV), (V) and (VI):
Preferably, R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group.

Further preferably, R¹ is an optionally substituted aryl group or an optionally substituted heteroaryl group.

Moreover preferably, R¹ is an optionally substituted phenyl group, an optionally substituted naphthyl group or an optionally substituted heteroaryl group containing one or two rings and from 5 to 10 ring atoms selected from C, O, N and S.

Especially preferably, R¹ is an optionally substituted phenyl group or an optionally substituted heteroaryl group containing one or two rings and 5, 6, 9 or 10 ring atoms selected from C, O, N and S.

Further preferably, R¹ is an optionally substituted heteroaryl group containing 5 or 6 ring atoms selected from C, O, N and S.

More preferably, R¹ is an optionally substituted phenyl group.

Further preferably, R¹ is a group of formula -Cy¹-L-Cy², wherein Cy¹ is an optionally substituted cycloalkylene group containing 1 or 2 rings and from 3 to 7 carbon ring atoms, an optionally substituted heterocycloalkylene group containing 1 or 2 rings and from 3 to 7 ring atoms selected from C, N, O and S, an optionally substituted phenylene group, or an optionally substituted heteroarylene group containing 5 or 6 ring atoms selected from C, N, O and S; Cy² is a cycloalkyl, heterocycloalkyl, alkylcycloalkyl, heteroalkylcycloalkyl, aryl, heteroaryl, aralkyl or heteroaralkyl group, all of which may optionally be substituted; and L is a bond or -O-, -S-, -NH-, -CH₂-, -CO-, -NHCO-, -CO-NH-, -CH₂-CO-NH-, -NH-CO-CH₂-, -CH₂-O-CO-NH-, -NH-CO-O-CH₂-, -O-CO-NH-, - NH-CO-O-, -NHSO₂-, -SO₂NH-, -CH₂-SO₂-NH-, -NH-SO₂-CH₂-, -S-CH₂-, -CH₂-S-, - NH-CH₂-, -CH₂-NH-, -O-CH₂- or-CH₂-O-.

Preferably, Cy² is an optionally substituted phenyl group, an optionally substituted biphenyl group, an optionally substituted naphthyl group, an optionally substituted heteroaryl group containing one or two rings and 5, 6, 9 or 10 ring atoms selected from C, O, N and S, an optionally substituted cycloalkyl group containing from 3 to 7 ring atoms, an optionally substituted heterocycloalkyl group containing from 3 to 7 ring atoms selected from C, N, O and S, an optionally substituted heterocycloalkylaryl group containing 9 or 10 ring atoms selected from C, N, S and O, or a group of formula -CH(CH₂Ph)Ph.

Further preferably, L is a bond or -NHCO-, -CO-NH-, -CH₂-CO-NH-, -NH-CO-CH₂-, - NHSO₂- or -SO₂NH-.

Moreover preferably, Cy¹ is a 1,4-phenylene group.

Further preferably, R¹ is a group of formula -CH(R⁶)-C(=O)-NH-R⁷.

Moreover preferably, R¹ is a group of formula -CH(R⁶)-R⁸.

Further preferably, R⁶ is hydrogen or a C₁₋₆ alkyl group, a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O, or a group of formula -CH₂-R^{6a}, wherein R^{6a} is a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O.

Especially preferably, R⁶ is a -CH(CH₃)₂ group.

Moreover preferably, R⁷ is an optionally substituted phenyl group or an optionally substituted C₃₋₇ cycloalkyl group; especially an optionally substituted phenyl group.

Further preferably, R⁸ is an optionally substituted benzimidazole group or an optionally substituted triazole group or an optionally substituted imidazole group.

Moreover preferably, R⁸ is a group of the following formula: 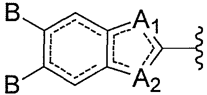 wherein each ".....", independently of one another, represents a single bond or a double bond, wherein at least one "....." in each of the rings is a double bond;
A₁ and A₂ each, independently of one another represents CH, N, NH, O or S;
B is R^{B1} or a group of formula -Y-R^{B2}, wherein
R^{B1} is a hydrogen atom, a halogen atom, CN, CF₃, CH₂-OH; NR^{T1}R^{T2}; or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R^{T1} and R^{T2} each, independently of one another, represents a hydrogen atom or a (C₁-C₃) alkyl group, which may be substituted by one or more, identical or different, group(s) selected from a halogen atom, OH, =O, and NH₂;
Y is -O- or -S-; and
R^{B2} is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted.

Further preferably, R⁸ is a group of the following formula: 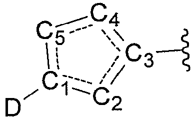 wherein each ".....", independently of one another, represents a single bond or a double bond, wherein at least one "....." is a double bond;
C₁ and C₃ each, independently of one another represents C or N;
C₂, C₄ and C₅ each, independently of one another represents CH, N, NH, O, or S;
D is an optionally substituted aryl group or an optionally substituted heteroaryl group (especially preferably, D is an optionally substituted phenyl group).

Further preferably, R² is a C₁₋₆ alkyl group; a heteroalkyl group containing from 1 to 6 carbon atoms and 1, 2, 3 or 4 heteroatoms selected from O, S and N; a C₄₋₁₀ alkylcycloalkyl group; or a C₇₋₁₂ aralkyl group; all of which may optionally be substituted.

Especially preferably, R² is a C₁₋₆ alkyl group; a heteroalkyl group containing from 1 to 6 carbon atoms and 1, 2, 3 or 4 heteroatoms selected from O, S and N; or a group of formula -CH₂-R²¹, wherein R²¹ is a C₃₋₇ cycloalkyl group, COOH, COOMe or an optionally substituted phenyl group.

Moreover, especially preferably, R² is a C₁₋₄ alkyl group; or a group of formula -CH₂-R²¹, wherein R²¹ is a C₃₋₆ cycloalkyl group, OMe, COOH, COOMe or an optionally substituted phenyl group. Preferably, R²¹ is a phenyl group which is unsubstituted or substituted by one or two substituents which are independently selected from OH, NO₂ and Me; further preferably, R²¹ is an unsubstituted phenyl group.

More preferably, R² is an optionally substituted benzyl group (i.e., a group of formula -CH₂-Ph which may optionally be substituted). Moreover preferably, R² is an unsubstituted benzyl group.

Further preferably, R² is an *iso*-butyl group (i.e., a group of formula -CH₂CH(CH₃)₂).

The term "optionally substituted" refers to a group which is unsubstituted or substituted by one or more (especially by one, two or three; preferably by one or two) substituents.

If group R¹ and/or group R² comprises more than one substituent, these substituents are independently selected, i.e., they may be the same or different.

If group R¹ and/or group R² is substituted by a cyclic group, such as e.g., a cycloalkyl group or a heterocycloalkyl group, this cyclic group may be bonded to group R¹ and/or group R² via a single or double bond or this cyclic group may be annulated or fused to group R¹ and/or group R². Isatin is an example for a substituted phenyl group.

Examples for substituents are fluorine, chlorine, bromine and iodine and OH, SH, NH₂, -SO₃H, -SO₂NH₂, -COOH, -COOMe, -COMe (Ac), -NHSO₂Me, -SO₂NMe₂, -CH₂NH₂, - NHAc, -SO₂Me, -CONH₂, -CN, -NHCONH₂, -NHC(NH)NH₂, -NOHCH₃, -N₃ and -NO₂ groups. Further examples of substituents are C₁-C₁₀ alkyl, C₂-C₁₀ alkenyl, C₂-C₁₀ alkynyl, C₁-C₁₀ heteroalkyl, C₃-C₁₈ cycloalkyl, C₁-C₁₇ heterocycloalkyl, C₄-C₂₀ alkylcycloalkyl, C₁-C₁₉ heteroalkylcycloalkyl, C₆-C₁₈ aryl, C₁-C₁₇ heteroaryl, C₇-C₂₀ aralkyl and C₁-C₁₉ heteroaralkyl groups; especially C₁-C₆ alkyl, C₂-C₆ alkenyl, C₂-C₆ alkynyl, C₁-C₆ heteroalkyl, C₃-C₁₀ cycloalkyl, C₁-C₉ heterocycloalkyl, C₄-C₁₂ alkylcycloalkyl, C₁-C₁₁ heteroalkylcycloalkyl, C₆-C₁₀ aryl, C₁-C₉ heteroaryl, C₇-C₁₂ aralkyl and C₁-C₁₁ heteroaralkyl groups, further preferably C₁-C₆ alkyl and C₁-C₆ heteroalkyl groups.

Preferred substituents are halogen atoms (e.g. F, Cl, Br, I) and groups of formula -OH, -O-C₁₋₆ alkyl (e.g. -OMe, -OEt, -O-*n*Pr, -O-*i*Pr, -O-*n*Bu, -O-*i*Bu and -O-*t*Bu), -NH₂, - NHC₁₋₆ alkyl, -N(C₁₋₆ alkyl)₂, -COOH, -COOMe, -COMe, -COCF₃, -NHSO₂Me, - SO₂NMe₂, -SO₃H, -SO₂NH₂, -CONH₂, -CH₂NH₂, -CN, -C₁₋₆ alkyl (e.g. -Me, -Et, -*n*Pr, - *i*Pr, -*n*Bu, -*i*Bu, -*t*Bu and -CF₃), -SH, -S-CO-C₁₋₆ alkyl, -S-C₁₋₆ alkyl, -NHAc, -NO₂, - C≡CH, -NHCONH₂, -SO₂Me, -SO₂CF₃, phenyl, -C₃₋₆ cycloalkyl (e.g. cyclopropyl, cyclobutyl) and heterocycloalkyl containing 3 to 6 ring atoms selected from C, N, S and O.

Further preferred substituents are halogen atoms (e.g. F, Cl, Br) and groups of formula -OH, -O-C₁₋₆ alkyl (e.g. -OMe, -OEt, -O-*n*Pr, -O-*i*Pr, -O-*n*Bu, -O-*i*Bu and -O-*t*Bu), -NH₂, -NHC₁₋₆ alkyl, -N(C₁₋₆ alkyl)₂, -COOH, -COOMe, -COMe, -NHSO₂Me, -SO₂NMe₂, - SO₃H, -SO₂NH₂, -CONH₂, -CH₂NH₂, -CN, -C₁₋₆ alkyl (e.g. -Me, -Et, -*n*Pr, -*i*Pr, -*n*Bu, - *i*Bu, -*t*Bu and -CF₃), -SH, -S-CO-C₁₋₆ alkyl, -S-C₁₋₆ alkyl, -NHAc, -NO₂, -C=CH, - NHCONH₂, -SO₂Me and cyclopropyl.

The substituent(s) is/are especially preferably independently selected from halogen (especially F and Cl), -Me, -CF₃, -OMe, -OH, -COOH, -CONH₂, -COOMe, -COMe and -NO₂.

The substituent(s) is/are further especially preferably independently selected from halogen (especially F and Cl), -Me, -CF₃, -OMe, -OH, -COOH, -COOMe, -COMe and -NO₂.

The most preferred compounds of the present invention are the compounds disclosed in the examples, or a salt thereof (where such examples relate to compounds other than those of formula (III) where R² represents -CH₂-CH(CH₃)₂, these are part of the present invention only in as far as they are indicated for use in the treatment of bacterial infections caused by *P. aeruginosa*).

It is further preferred to combine the preferred embodiments of the present invention in any desired manner (e.g., any embodiment for R¹ may be combined with any embodiment of R²).

The suffix "-ene" like e.g., in "phenylene" refers to the corresponding divalent group.

The expression alkyl refers to a saturated, straight-chain or branched hydrocarbon group that contains from 1 to 20 carbon atoms, preferably from 1 to 15 carbon atoms, especially from 1 to 10 (e.g. 1, 2, 3 or 4) carbon atoms, for example a methyl (Me, CH₃), ethyl (Et), *n*-propyl (*n*Pr), *iso*-propyl (*i*Pr), *n*-butyl (*n*Bu), *iso*-butyl (*i*Bu), sec-butyl (*s*Bu), *tert*-butyl (*t*Bu), *n*-pentyl, *iso*-pentyl, *n*-hexyl, 2,2-dimethylbutyl or *n*-octyl group. Especially preferred alkyl groups are C₁₋₆ alkyl groups; moreover preferred alkyl groups are C₁₋₄ alkyl groups.

The expression C₁₋₆ alkyl refers to a saturated, straight-chain or branched hydrocarbon group that contains from 1 to 6 carbon atoms. The expression C₁₋₄ alkyl refers to a saturated, straight-chain or branched hydrocarbon group that contains from 1 to 4 carbon atoms. Examples are a methyl, ethyl, *n*-propyl, *iso*-propyl, *n*-butyl, *iso*-butyl, *sec*-butyl or *tert*-butyl group.

The expressions alkenyl and alkynyl refer to at least partially unsaturated, straight-chain or branched hydrocarbon groups that contain from 2 to 20 carbon atoms, preferably from 2 to 15 carbon atoms, especially from 2 to 10 (e.g. 2, 3 or 4) carbon atoms, for example an ethenyl (vinyl), propenyl (allyl), isopropenyl, butenyl, ethynyl (acetylenyl), propynyl (e.g. propargyl), butynyl, isoprenyl or hex-2-enyl group. Preferably, alkenyl groups have one or two (especially preferably one) double bond(s), and alkynyl groups have one or two (especially preferably one) triple bond(s).

The expression heteroalkyl refers to an alkyl, alkenyl or alkynyl group in which one or more (preferably 1 to 8; especially preferably 1, 2, 3 or 4) carbon atoms have been replaced by an oxygen, nitrogen, phosphorus, boron, selenium, silicon or sulfur atom (preferably by an oxygen, sulfur or nitrogen atom) or by a SO or a SO₂ group. The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy. Furthermore, the term heteroalkyl refers to groups in which one or more hydrogen atoms have been replaced by a halogen atom (preferably F or CI).

Preferably, a heteroalkyl group contains from 1 to 12 carbon atoms and from 1 to 8 heteroatoms selected from oxygen, nitrogen and sulfur (especially oxygen and nitrogen). Especially preferably, a heteroalkyl group contains from 1 to 6 (e.g. 1, 2, 3 or 4) carbon atoms and 1, 2, 3 or 4 (especially 1, 2 or 3) heteroatoms selected from oxygen, nitrogen and sulfur (especially oxygen and nitrogen). The term C₁-C₁₀ heteroalkyl refers to a heteroalkyl group containing from 1 to 10 carbon atoms and 1, 2, 3, 4, 5 or 6 heteroatoms selected from O, S and/or N (especially O and/or N). The term C₁-C₆ heteroalkyl refers to a heteroalkyl group containing from 1 to 6 carbon atoms and 1, 2, 3 or 4 heteroatoms selected from O, S and/or N (especially O and/or N). The term C₁-C₄ heteroalkyl refers to a heteroalkyl group containing from 1 to 4 carbon atoms and 1, 2 or 3 heteroatoms selected from O, S and/or N (especially O and/or N).

Further preferably, the expression heteroalkyl refers to an alkyl group as defined above (straight-chain or branched) in which one or more (preferably 1 to 6; especially preferably 1, 2, 3 or 4) carbon atoms have been replaced by an oxygen, sulfur or nitrogen atom or a CO group or a SO group or a SO₂ group; this group preferably contains from 1 to 6 (e.g. 1, 2, 3 or 4) carbon atoms and 1, 2, 3 or 4 (especially 1, 2 or 3) heteroatoms selected from oxygen, nitrogen and sulfur (especially oxygen and nitrogen); this group may preferably be substituted by one or more (preferably 1 to 6; especially preferably 1, 2, 3 or 4) fluorine, chlorine, bromine or iodine atoms or OH, =O, SH, =S, NH₂, =NH, N₃, CN or NO₂ groups.

Examples of heteroalkyl groups are groups of formulae: R^{a}-O-Y^{a}-, R^{a}-S-Y^{a}-, Ra-SO-Y^{a}-, R^{a}-SO₂-Y^{a}-, R^{a}-N(R^{b})-SO₂-Y^{a}-, R^{a}-SO₂-N(R^{b})-Y^{a}-, R^{a-}N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, *n*-propyloxy, *iso*-propyloxy, *n*-butoxy, *tert-*butyloxy*,* methoxymethyl, ethoxymethyl, -CH₂CH₂OH, -CH₂OH, -SO₂Me, -NHAc, methoxyethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, *N*-ethyl-*N*-methylcarbamoyl or *N*-methylcarbamoyl. Further examples of heteroalkyl groups are nitrile (-CN), isonitrile, cyanate, thiocyanate, isocyanate, isothiocyanate and alkylnitrile groups.

The expression cycloalkyl refers to a saturated cyclic group that contains one or more rings (preferably 1 or 2), and preferably contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetraline, cyclopentylcyclohexyl, fluorocyclohexyl or cyclohex-2-enyl group. Preferably, the expression cycloalkyl refers to a saturated cyclic group that contains one or more rings (preferably 1 or 2), and contains from 3 to 14 ring carbon atoms, preferably from 3 to 10 (especially 3, 4, 5, 6 or 7) ring carbon atoms.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulfur atom (preferably by an oxygen, sulfur or nitrogen atom) or a SO group or a SO₂ group. A heterocycloalkyl group has preferably 1 or 2 ring(s) and 3 to 10 (especially 3, 4, 5, 6 or 7) ring atoms (preferably selected from C, O, N and S). The expression heterocycloalkyl refers furthermore to groups that are substituted by fluorine, chlorine, bromine or iodine atoms or by OH, =O, SH, =S, NH₂, =NH, N₃ or NO₂ groups. Examples are a piperidyl, prolinyl, imidazolidinyl, piperazinyl, morpholinyl (e.g. -N(CH₂CH₂)₂O), urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings and from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulfur atom (preferably by an oxygen, sulfur or nitrogen atom) or a SO group or a SO₂ group. A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings and from 6 to 14 ring carbon atoms, preferably from 6 to 10 (especially 6) ring carbon atoms. The expression aryl refers furthermore to groups that are substituted by fluorine, chlorine, bromine or iodine atoms or by OH, SH, NH₂, N₃ or NO₂ groups. Examples are the phenyl (Ph), naphthyl, biphenyl, 2-fluorophenyl, anilinyl, 3-nitrophenyl or 4-hydroxyphenyl group.

The expression heteroaryl refers to an aromatic group that contains one or more rings and from 5 to 14 ring atoms, preferably from 5 to 10 (especially 5 or 6 or 9 or 10) ring atoms, comprising one or more (preferably 1, 2, 3 or 4) oxygen, nitrogen, phosphorus or sulfur ring atoms (preferably O, S or N). The expression heteroaryl refers furthermore to groups that are substituted by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g. 3-phenylpyrrolyl), thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl, pyridazinyl, 4-hydroxypyridyl (4-pyridonyl), 3,4-hydroxypyridyl (3,4-pyridonyl), oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g. 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are phenylcyclopentyl, cyclohexylphenyl as well as groups derived from toluene, xylene, mesitylene, styrene, benzyl chloride, o-fluorotoluene, 1*H*-indene, tetraline, dihydronaphthalene, indanone, cumene, fluorene and indane. An aralkyl group preferably contains one or two aromatic ring systems (especially 1 or 2 rings), each containing from 6 to 10 carbon atoms and one or two alkyl, alkenyl and/or alkynyl groups containing from 1 or 2 to 6 carbon atoms and/or a cycloalkyl group containing 3, 4, 5, 6 or 7 ring carbon atoms.

The expression heteroaralkyl refers to groups containing both aryl and/or heteroaryl groups and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (especially 1 or 2 rings), each containing from 5 or 6 to 9 or 10 ring atoms (preferably selected from C, N, O and S) and one or two alkyl, alkenyl and/or alkynyl groups containing 1 or 2 to 6 carbon atoms and/or one or two heteroalkyl groups containing 1 to 6 carbon atoms and 1, 2 or 3 heteroatoms selected from O, S and N and/or one or two cycloalkyl groups each containing 3, 4, 5, 6 or 7 ring carbon atoms and/or one or two heterocycloalkyl groups, each containing 3, 4, 5, 6 or 7 ring atoms comprising 1, 2, 3 or 4 oxygen, sulfur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroaryl-heterocycloalkyl, heteroarylheterocycloalkenyl, heteroarylalkylcycloalkyl, heteroaryl-alkylheterocycloalkenyl, heteroarylheteroalkylcycloalkyl, heteroaryl heteroalkyl-cycloalkenyl and heteroarylheteroalkylheterocycloalkyl groups, the cyclic groups being saturated or mono-, di- or tri-unsaturated. Specific examples are a tetrahydroisoquinolinyl, benzoyl, phthalidyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups that are substituted by fluorine, chlorine, bromine or iodine atoms or by OH, =O, SH, =S, NH₂, =NH, N₃ or NO₂ groups.

The term halogen refers to F, CI, Br or I.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated or fused or bridged.

Owing to their substitution, the compounds *per se* or for use according to the medicinal indication of the present invention may contain one or more centers of chirality. The present invention therefore includes both all pure enantiomers and all pure diastereomers and also mixtures thereof in any mixing ratio. The present invention moreover also includes all cis/trans-isomers of the compounds of the present invention and also mixtures thereof. The present invention moreover includes all tautomeric forms of the compounds of the present invention.

The present invention further provides pharmaceutical compositions comprising one or more compounds of formula (III), wherein R² represents the group -CH₂-CH(CH₃)₂, or a pharmaceutically acceptable salt, solvate or hydrate thereof, optionally in combination with one or more carrier substances and/or one or more adjuvants. The pharmaceutical composition of the present invention may contain a further antibacterial compound.

The compounds or pharmaceutical compositions of the present invention may be administered in combination with a further antibacterial compound.

The present invention relates to compounds of the various formulae disclosed herein for use in the treatment of bacterial infections caused by *P. aeruginosa.* The present invention also relates to compounds of formula (III) *per se,* where R² represents a group of formula -CH₂-CH(CH₃)₂. In respect of this last group of compounds, the invention also extends to pharmaceutical compositions thereof and these compounds for use in the treatment of bacterial infections.

Examples of pharmacologically acceptable salts of sufficiently basic compounds are salts of physiologically acceptable mineral acids like hydrochloric, hydrobromic, sulfuric and phosphoric acid; or salts of organic acids like methanesulfonic, *p*-toluenesulfonic, lactic, acetic, trifluoroacetic, citric, succinic, fumaric, maleic and salicylic acid. Further, a sufficiently acidic compound may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of the compounds described herein.

The compounds described herein may be solvated, especially hydrated. The solvation/ hydration may occur during the process of production or as a consequence of the hygroscopic nature of the initially water-free compounds. The solvates and/or hydrates may e.g. be present in solid or liquid form.

In general, the compounds and pharmaceutical compositions described herein will be administered by using the established and acceptable modes known in the art.

For oral administration, such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g. liquid aerosol), transdermal, for example via an transdermal drug delivery system (TDDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules, one may use excipients as are e.g. vegetable, petroleum, animal or synthetic oils, wax, fat, and polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations, one may use compressed gases suitable for this purpose, e.g. oxygen, nitrogen and carbon dioxide. The pharmaceutically useful agents may also contain additives for conservation, stabilization, e.g. UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 1 mg to about 10,000 mg, preferably from about 5 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

According to a moreover preferred embodiment, the present invention provides compounds as defined herein for use in a method for inhibiting the *P. aeruginosa* virulence factor LasB in a subject which comprises administering to the subject an effective amount of a compound of formula (I), or a pharmaceutically acceptable salt thereof.

According to a moreover preferred embodiment, the present invention provides compounds of formula (III), wherein R² represents a group of formula -CH₂-CH(CH₃)₂, for use in a method for treating a bacterial infection which comprises administering to a subject in need of such treatment a therapeutically effective amount of such a compound, or a pharmaceutically acceptable salt thereof.

According to a further preferred embodiment, the present invention provides a pharmaceutical composition comprising a compound of formula (III), wherein R² represents the group -CH₂-CH(CH₃)₂, or a pharmaceutically acceptable salt thereof, for use in a method for treating a bacterial infection which comprises administering to a subject in need of such treatment said pharmaceutical composition.

Gram-positive pathogens *Clostridium histolyticum* (recently renamed as *Hathewaya histolytica*), *C. tetani* and *Bacillus cereus* produce collagenases ColH and ColG (*C*. *histolyticum*), ColT (*C. tetani*) and ColQ1 (*B. cereus* strain Q1) as virulence factors, which are attractive targets for the treatment of infections derived from these bacteria (Schönauer, E.; Kany, A. M.; Haupenthal, J.; Hüsecken, K.; Hoppe, I. J.; Voos, K.; Yahiaoui, S.; Elsässer, B.; Ducho, C.; Brandstetter, H.; Hartmann, R. W. J. Am. Chem. Soc. 2017, 139, 12696-12703). The compounds of the present invention are also potent inhibitors of these collagenases.

### EXAMPLES

### I. General Procedures:

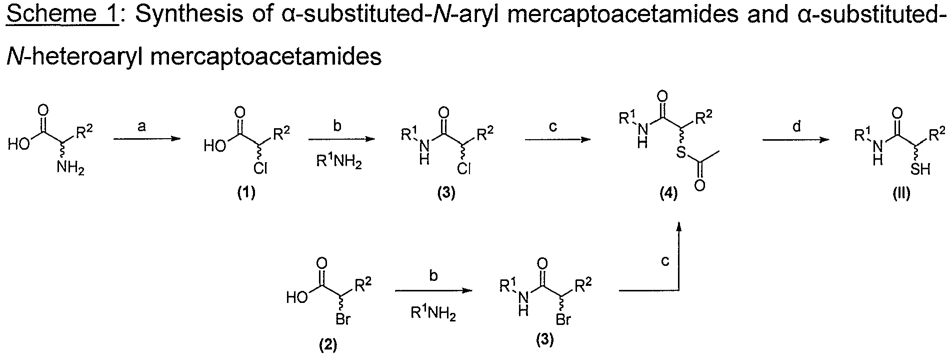

(a) sodium nitrite, 6 M HCl, -5 °C to r.t., (b) EDC·HCl, DCM, r.t. or ClCO₂Et, Et₃N, THF, r.t.; (c) potassium thioacetate, acetone, r.t.; (d) NaOH, MeOH, r.t.

### General procedure A: Synthesis of 2-chloroalkanoic acids (1)

Amino acid (1.0 equiv.) was dissolved in 6 M hydrochloric acid (2 mL/mmol or until mostly dissolved) under nitrogen atmosphere and cooled to -5 °C. Sodium nitrite (3.5 equiv.) was dissolved in water (0.3 mL/mmol amino acid) and slowly added dropwise. The mixture was stirred overnight while warming to rt. The reaction mixture was extracted with EtOAc/THF (3:1, 3 x). The combined organic extracts were washed with saturated aqueous NaCl solution, dried over anhydrous Na₂SO₄ and filtered. The solvent was removed under reduced pressure to afford the crude product, which was used in the next step without further purification.

### General procedure B-1: Synthesis of N-aryl-2-halo-2-alkylacetamide derivatives (3)

2-Haloalkanoic acid (1.2 equiv.) (2-chloroalkanoic acid (**1**) as crude or commercially available 2-bromoalkanoic acid (**2**)) and EDC·HCl (1.2 equiv.) were added to a solution of the corresponding aniline (1.0 equiv.) in DCM. The resultant mixture was stirred at rt, until the starting aniline was consumed (monitored by TLC or LC-MS). The obtained solution was washed with 1 M HCl and saturated aqueous NaCl solution. The organic layer was dried over anhydrous Na₂SO₄, filtered and concentrated under reduced pressure to afford the crude product. The crude product obtained was either used for the next step without further purification or purified using column chromatography.

### General procedure B-2: Synthesis of N-heteroaryl-2-halo-2-alkylacetamide derivatives (3)

2-Haloalkanoic acid (1.0 equiv.) (2-chloroalkanoic acid (**1**) as crude or commercially available 2-bromoalkanoic acid (**2**)) was dissolved in THF. Et₃N (1.0 equiv.) was added to this solution at rt, followed by dropwise addition of ethylchloroformate (1.1 equiv.). A solution of the corresponding heterocyclic amine (0.8 equiv.) was dissolved in THF and added dropwise to this mixture. The reaction was stirred at r.t overnight. THF was evaporated, the crude solid was dissolved in DCM, and the solution was washed with aqueous KHCO₃ (10% wt) and water. The organic layer was dried over anhydrous Na₂SO₄, filtered and concentrated under reduced pressure to afford the crude product. The resultant crude was purified via flash chromatography.

### General procedure C: Synthesis of N-aryl-2-thioacetyl-2-alkylacetamide derivatives and N-heteroaryl-2-thioacetyl-2-alkylacetamide derivatives (4)

*N*-Aryl-2-halo-2-alkylacetamide derivative or *N*-heteroaryl-2-halo-2-alkylacetamide derivative (1.0 equiv.) ((**3**) purified or as crude) was dissolved in acetone, and potassium thioacetate (2.0 equiv.) was added to the solution. The resultant mixture was stirred at rt until full conversion (monitored by TLC or LC-MS). After concentration under vacuum, the resultant residue was diluted with H₂O and extracted with EtOAc. The organic layer was washed with saturated aqueous NaCl solution, dried over anhydrous Na₂SO₄, filtered and evaporated under reduced pressure. The crude residue was purified using column chromatography.

### General procedure D: Synthesis of N-aryl-2-mercapto-2-alkylacetamide derivatives and N-heteroaryl-2-mercapto-2-alkylacetamide derivatives (II)

NaOH (3.0 equiv.) was added to a solution of compound **4** (1.0 equiv.) in MeOH under argon atmosphere. The reaction was stirred at rt. The reaction mixture was acidified with 2 M HCl and extracted with EtOAc. The obtained organic layer was washed with 0.5 M HCl solution and with saturated aqueous NaCl solution, dried over anhydrous Na₂SO₄, filtered and evaporated under reduced pressure. In case of heterocyclic derivatives, instead of HCl, pH was adjusted to acidic values with Amberlite IR-120. The product was obtained as pure or purified using column chromatography or preparative HPLC.

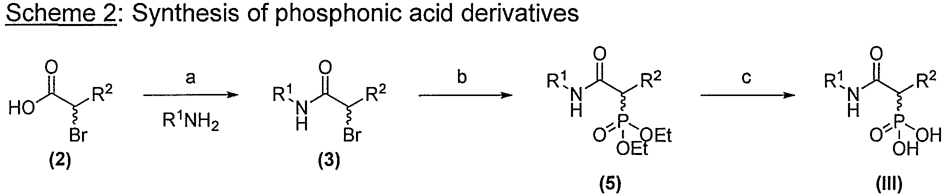

(a) EDC·HCl, DCM, r.t.; (b) P(OEt)₃, neat, 150 °C; (c) i) TMSBr, DCM, r.t.; ii) MeOH, r.t.

### General procedure E: Synthesis of diethyl phosphonate derivatives (5)

*N*-Aryl-2-bromo-2-alkylacetamide derivative (**3**) (1.0 equiv.) was suspended in triethyl phosphite (10 equiv.), equipped with a reflux condenser, heated to 150 °C and stirred for a total of 18 h. Most of unreacted triethyl phosphite was evaporated *in vacuo* and the resultant oil was purified by column chromatography.

### General procedure F: Synthesis of phosphonic acid derivatives (III)

To a solution of diethyl phosphonate (**5**) (1.0 equiv.) in dry DCM, bromotrimethylsilane (5.0 equiv.) was added dropwise over a period of 15 min. The reaction mixture was stirred at r.t. overnight. Then, MeOH was added and stirred for 30 min at r.t. to cleave the previously formed TMS ester. Solvents were concentrated *in vacuo* and the resultant oil was purified by preparative HPLC. 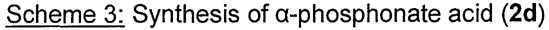 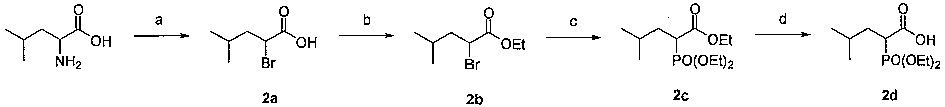 (a) HBr, NaNO₂, H₂O, 0 °C - rt, 2h, quant., (b) EtOH, cat. H₂SO₄, reflux, 2h, 81%, (c) P(OEt)₃, neat, 150 °C; 48h, 44%, (d) NaOH, EtOH, 0 °C-rt, 48h, 98%.

### 2-Bromo-4-methylpentanoic acid (2a)

10.5 g racemic leucine **1** (80.0 mmol, 1.0 equiv.) was dissolved in 48% HBr (80 mL) and 72 mL dist. water. The mixture was cooled to 0 °C and a solution of NaNO₂ (8.82 g, 128.0 mmol, 1.6 equiv.) in 20 mL dist. water was added dropwise over 2 h. The mixture was warmed up to rt and was stirred overnight. After that, the mixture was transferred into a separatory funnel and extracted with acetone (4 × 100 mL). The combined organic layers were washed with dist. water (400 mL) and saturated aqueous NaCl solution (400 mL), dried over MgSO₄, filtered and concentrated under reduced pressure. The compound **2a** (15.06 g, 80.0 mmol, quantitative) was obtained as a pale yellow liquid and was used in the next step without further purification.

### Ethyl 2-bromo-4-methylpentanoate (2b)

To α-bromo acid **2a** (15.06 g, 80.0 mmol, 1.0 equiv.) a solution of concentrated sulphuric acid (30 µL/mmol) in ethanol (2 mL/mmol) was added and the mixture was refluxed for 2 h. After that, the solution was cooled to rt and concentrated under reduced pressure. Et₂O (150 mL) was added and the organic layer was washed with aqueous saturated NaHCO₃ solution (150 mL), followed by saturated aqueous NaCl solution (150 mL). The organic layer was dried over MgSO₄, filtered and concentrated under reduced pressure. The compound **2b** (14.45 g, 64.7 mmol, 81% yield) as a pale yellow liquid and was used in the next step without further purification.

### 2-(Diethoxyphosphoryl)-4-methylpentanoate (2c)

The α-bromo ester **2b** (14.45 g, 64.7 mmol, 1.0 equiv.) and P(OEt)₃ (22.41 mL, 129.4 mmol, 2.0 equiv.) were mixed and heated to 150 °C for 48 h. After that, the mixture was cooled down to rt, and Et₂O (350 mL) was added. The mixture was transferred into a separatory funnel and washed with saturated aqueous NaCl solution (2 × 350 mL), dried over MgSO₄, filtered and concentrated under reduced pressure. The product was purified using flash chromatography (SiO₂, hexanes/EtOAc 1:1), and compound **2c** (7.93 g, 28.3 mmol, 44%) was obtained as pale yellow oil. ¹H NMR (CDCl₃, 500 MHz) δ ppm: 4.18-4.24 (m, 2H), 4.11-4.17 (m, 4H), 3.00-3.07 (m, 1H), 1.95-2.07 (m, 1H), 1.55-1.66 (m, 2H), 1.33 (dt, 6H, *J* = 2.3, 7.0 Hz), 1.28 (t, 3H, *J* = 7.2 Hz), 0.92 (d, 3H, *J* = 6.1 Hz), 0.89 (d, 3H, *J* = 6.3 Hz). ¹³C NMR (CDCl₃, 126 MHz) δ ppm: 169.4 (d, *J* = 5.5 Hz), 62.7 (d, *J* = 6.4 Hz), 62.6 (d, *J* = 6.4 Hz), 61.3, 44.5, 43.4, 35.5 (d, *J* = 5.5 Hz), 26.9 (d, *J* = 14.7 Hz), 22.9, 21.2, 16.4 (d, *J* = 3.7 Hz), 16.3 (d, *J* = 3.7 Hz), 14.1. ³¹ P NMR (CDCl₃, 202 MHz) δ ppm: 23.4.

HRMS (ESI+) calculated for C₁₂H₂₆O₅P [M+1]⁺ 281.1518, found: 281.1503.

### 2-(Diethoxyphosphoryl)-4-methylpentanoic acid (2d)

The compound **2c** (7.93 g, 28.3 mmol, 1.0 equiv.) was dissolved in EtOH (270 mL), and NaOH (2.15 g, 53.88 mmol, 2.0 equiv.) in dist. H₂O (100 mL) was added. The mixture was stirred at rt overnight. The progress was monitored using LC-MS. After completion, the mixture was transferred into a separatory funnel, dist. water (300 mL) and Et₂O (400 mL) were added, and the layers were separated. The aqueous layer was acidified to pH = 1 using HCl (6 M), and extracted with EtOAc (3 × 300 mL). The combined EtOAc-layers were washed with saturated aqueous NaCl solution (2 × 500 mL), dried over MgSO₄, filtered and concentrated under reduced pressure. The compound **2d** (6.63 g, 26.29 mmol, 98%) was obtained as a pale-yellow oil, which was used without further purification. ¹H NMR (CDCl₃, 500 MHz) δ ppm: 8.33 (br s, 2H), 4.13-4.25 (m, 4H), 3.07 (ddd, 1H, *J =* 3.1, 11.3, 23.0 Hz), 1.99 (dddd, 1H, *J =* 4.8, 8.5, 11.4, 13.5 Hz), 1.58-1.70 (m, 1H), 1.49-1.57 (m, 1H), 1.33 (dt, 6H, *J* = 2.7, 7.1 Hz), 0.92 (d, 3H, *J* = 6.6 Hz), 0.89 (d, 3H, *J* = 6.6 Hz). ¹³C NMR (CDCl₃, 126 MHz) δ ppm: 171.9 (d, *J* = 3.7 Hz), 63.7 (d, *J* = 6.4 Hz), 62.9 (d, *J* = 6.4 Hz), 44.4, 43.4, 35.6 (d, *J =* 5.5 Hz), 26.8 (d, *J =* 13.8 Hz), 23.0, 21.2, 16.3 (d, *J =* 2.8 Hz), 16.2 (d, *J =* 2.8 Hz).³¹P NMR (CDCl₃, 202 MHz) δ ppm: 24.3. HRMS (ESI+) calculated for C₁₀H₂₂O₅P [M+1]⁺ 253.1205, found: 253.1191. 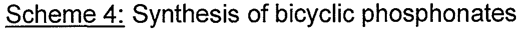 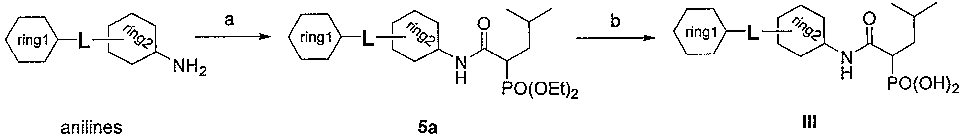
**L (linker):** no linker, O, NH, S, CH₂, CH₂O, CH₂S, SCH₂, NHCH₂, CO, NHCO, CONH, CH₂CONH, NHSO₂, SO₂NH,
CH₂SO₂NH
**ring1:** aromatic or saturated 6-membered ring
**ring2:** aromatic or saturated 6-membered ring or propellane
(a) **2d,** EDC·HCl, DCM, r.t. or TBTU, NMM, DCM (or DMF), 0 °C to r.t.; (b) TMSBr, DCM, r.t.; ii) MeOH, r.t.

### General procedure G: Synthesis of diethyl phosphonate derivatives (5a)

Aniline (commercially available or synthesized according to conventional protocols that can be found in literature, examples given in general procedures G-1, G-2, G-3, G-4 below) (1.0 equiv.), 2-(diethoxyphosphoryl)-4-methylpentanoic acid (**2d**) (1.2 equiv.) and N-methylmorpholine (2.5 equiv.) were dissolved in DCM or DMF. The reaction mixture is cooled in an ice-bath and TBTU (1.5 equiv.) was added. The temperature was maintained for 30 minutes and then allowed to warm up to r.t. As another alternative route, instead of TBTU/NMM, EDC·HCl (2.0 equiv.), HOBt (2.0 equiv.) and DIPEA (2.5 equiv.) were used. In both cases, the reaction mixture was stirred overnight, then washed with water and saturated aqueous NaCl solution. The organic layer was dried over anhydrous Na₂SO₄, filtered and concentrated under reduced pressure to afford the crude product. The obtained crude product was either used for the next step without further purification or purified using column chromatography.

### General procedure G-1: Synthesis of anilines with the amide linker (CONH and CH₂CONH)

The corresponding carboxylic acid (1.2 equiv.) was dissolved in DCM and EDC·HCl (1.2 equiv.) was added, followed by *tert*-butyl (4-aminophenyl)carbamate (1.0 equiv.). The reaction mixture was stirred at rt. In case a precipitate was formed, it was filtered and washed with DCM. When no precipitate was formed, after the consumption of the starting material, the reaction mixture was washed with 1 M HCl (x 2) and saturated aqueous NaCl solution (× 1) and purified on column chromatography. Obtained product was suspended in DCM/TFA mixture (3:1) at 0 °C. The mixture was then stirred at rt for 2 h. Solvents were evaporated. EtOAc was added, washed with 2.5 M NaOH (x 2) and saturated aqueous NaCl solution (× 2). The organic layer was dried over anhydrous Na₂SO₄, filtered and concentrated under reduced pressure to give the desired aniline.

### General procedure G-2: Synthesis of anilines with the sulfonamide linker (SO₂NH and CH₂SO₂NH)

Tert-butyl (4-aminophenyl)carbamate (1.0 equiv.) was dissolved in DCM and cooled to 0 °C. Et₃N (1.2 equiv.) was added, followed by the corresponding sulfonyl chloride (1.1 equiv.). The reaction mixture was stirred at rt for 8 h. Precipitate was filtered and filtrate purified on column chromatography. The product obtained was suspended in DCM/TFA mixture (3:1) at 0 °C. The mixture was then stirred at rt for 2 h. Solvents were evaporated. EtOAc was added, washed with 2.5 M NaOH (× 2) and saturated aqueous NaCl solution (× 2). The organic layer was dried over anhydrous Na₂SO₄, filtered and concentrated under reduced pressure to give the desired aniline.

### General procedure G-3: Synthesis of anilines with the ether linker (CH₂O)

*Tert*-butyl (4-hydroxyphenyl)carbamate (1.0 equiv.) was dissolved in DMF. Potassium carbonate (2.0 equiv.) was added, and the reaction mixture stirred for 15 minutes. The corresponding benzyl bromide was then added drop-wise during 15 minutes and left to stir at rt overnight. Water was added, extracted with EtOAc (× 3), and the organic layer was washed with saturated aqueous NaCl solution. The organic layer was dried over anhydrous Na₂SO₄, filtered and concentrated under reduced pressure. Obtained product was suspended in DCM/TFA mixture (3:1) at 0 °C. Mixture was then stirred at rt for 2 h. Solvents were evaporated. EtOAc was added, washed with 2.5 M NaOH (× 2) and saturated aqueous NaCl solution (× 2). Organic layer was dried over anhydrous Na₂SO₄, filtered and concentrated under reduced pressure to give the desired aniline.

### General procedure G-4: Synthesis of anilines without linker

Aryl amine (1.0 equiv.) was placed in a sealed tube, followed by the corresponding boronic acid (1.5 equiv.), NaOH 2M, tetrakis(triphenylphosphine)palladium (0.02 equiv.) and a mixture of dioxane/H₂O (4:1, v:v). The reaction mixture was flushed with N₂ and submitted to microwave irradiation (150 °C, 150 W) for 20 minutes. After cooling down to rt, a mixture of EtOAc/H₂O (1:1, v:v) was added to stop the reaction. The aqueous layer was extracted with EtOAc (× 3). The organic layer was washed with saturated aqueous NaCl solution (1x) and with water (1x), dried over MgSO₄, filtered and concentrated under reduced pressure. The residue was purified using column chromatography.

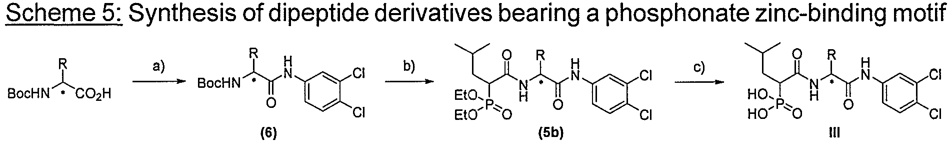

(a) IBCF, NMM, -20 °C THF, 30 min, (b) i) HCl (4 M in dioxane), r.t., 12h, ii) **2d,** TBTU, NMM, DMF, 0 °C - r.t., 12h, c) TMSBr, r.t., 12h, prep HPLC.

### General procedure H: Synthesis of aniline substituted derivatives (6)

The corresponding Boc-protected amino acid (1.0 equiv.) was dissolved in THF (0.1 M) and cooled down to -20 °C. Then NMM (2.5 equiv.) and isobutyl chloroformate (1.0 equiv.) were added dropwise. The reaction mixture was stirred at this temperature for 30 minutes and then the aniline (1.0 equiv.), dissolved in THF (1 M), was added. After the reaction mixture had reached rt, it was diluted with EtOAc. The organic phase was washed with KHSO₄ (1 N) solution, saturated NaHCO₃ solution and saturated aqueous NaCl solution, dried over anhydrous Na₂SO₄, filtered and concentrated under reduced pressure. Column chromatographic purification afforded the corresponding peptide.

### General procedure I: Synthesis of phosphor containing dipeptides (5b)

The Boc-protected peptide (1.0 equiv.) was dissolved in DCM (0.1 M) and treated at 0 °C with HCl (10.0 equiv., 4 M in dioxane). The mixture was warmed up to rt and after complete conversion (TLC), the solvent was removed under reduced pressure with the result that the crystalline hydrochloride remained, which was subsequently dissolved in DMF (0.1 M). 2-(diethoxyphosphoryl)-4-methylpentanoic acid **2d** (1.1 equiv.) was added to this solution and the reaction mixture was cooled to 0 °C. Coupling was achieved by TBTU (1.1 equiv.) and NMM (2.5 equiv.). The reaction mixture was warmed up to rt and after complete conversion (TLC) diluted with EtOAc and washed successively with 1 N KHSO₄ solution, saturated NaHCO₃ solution and saturated aqueous NaCl solution. The organic layer was dried over anhydrous Na₂SO₄, filtered, and the residue was used without further purification for the next step.

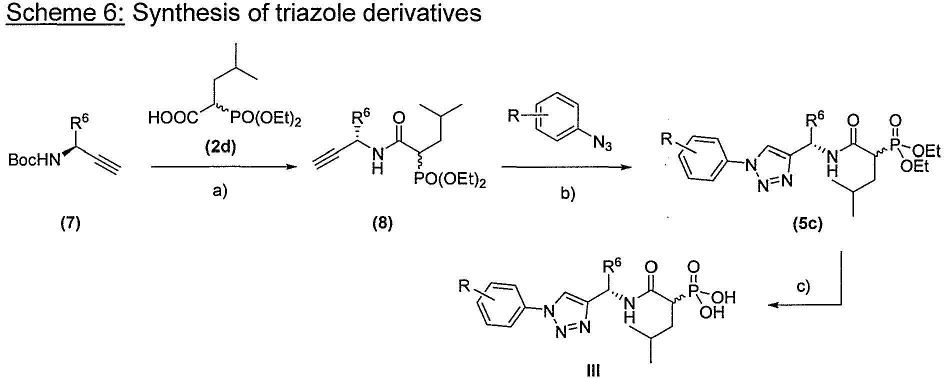

(a) i) HCl (4 M in dioxane), DCM, r.t.; 18 h, ii) TBTU, NMM, DMF 0 °C-rt, 22 h; (b) CuSO₄·5 H₂O, Na ascorbate, *t*BuOH/H₂O/MeOH (2:2:1) r.t., 14 h; (c) TMSBr, DCM, r.t., overnight, prep HPLC.

### General procedure J: Synthesis of alkinyl diethyl phosphonates (8)

Alkyne component **7** (1.0 equiv.), which was synthesized as previously reported (https://doi.org/10.1002/anie.201601564), was dissolved in DCM (10 mL/mmol), and HCl (10.0 equiv., 4 M in dioxane) was added at rt. The mixture was stirred for 18 h and then concentrated under reduced pressure. In the meantime, a mixture of compound **2d** (1.1 equiv.) and TBTU (1.2 equiv.) in DMF (5 mL/mmol) was cooled to 0 °C and NMM (2.5 equiv.) was added. The reaction mixture was stirred for 30 minutes and then Boc-deprotected alkenyl amino acid was dissolved in DMF (5 mL/mmol) and added dropwise at 0 °C. The mixture was stirred for 22 h and allowed to warm to rt. After addition of EtOAc, the organic layer was subsequently washed with saturated aqueous NaHCO₃ solution, 1 M HCl, water and saturated aqueous NaCl solution. The organic layer was dried over Na₂SO₄, filtered and concentrated under reduced pressure. The crude product was purified *via* automated combiflash purification (Teledyne ISCO).

### General procedure K: Synthesis of 1H-1,2,3-triazole containing diethyl phosphonates (5c)

A solution of the corresponding alkenyl diethyl phosphonate (1.0 equiv.) and the azide (1.1 equiv.), which was synthesized according to (https://doi.org/10.1016/j.ejmech.2019.06.007) in *t*BuOH/H₂O/MeOH (2:2:1, 10 mL/mmol) was purged with argon. Sodium ascorbate (20 mol%) and CuSO₄·5 H₂O (10 mol%) were added, and the reaction mixture was stirred at rt for 14 h. Then, saturated EDTA solution was added, and the mixture extracted with EtOAc (x 3), the combined organic layers were washed with saturated aqueous NH₄Cl solution and saturated aqueous NaCl solution. After drying over anhydrous Na₂SO₄ and filtration, the solvent was removed under reduced pressure to yield the title compound, which was used in the next step without further purification.

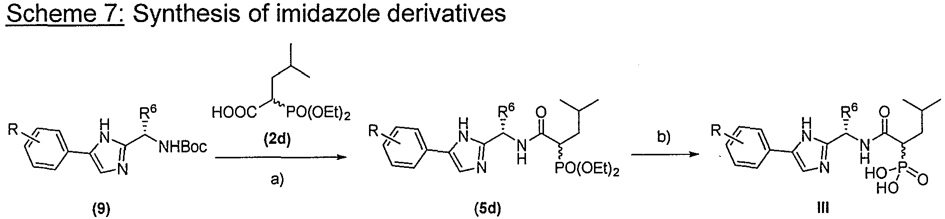

(a) i) HCl (4 M in dioxane), DCM, r.t.; 18 h, ii) TBTU, NMM, DMF 0 °C-rt, 22 h; (b) TMSBr, DCM, r.t., overnight, prep HPLC.

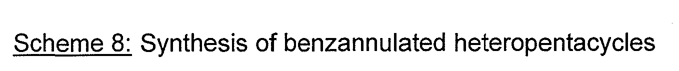

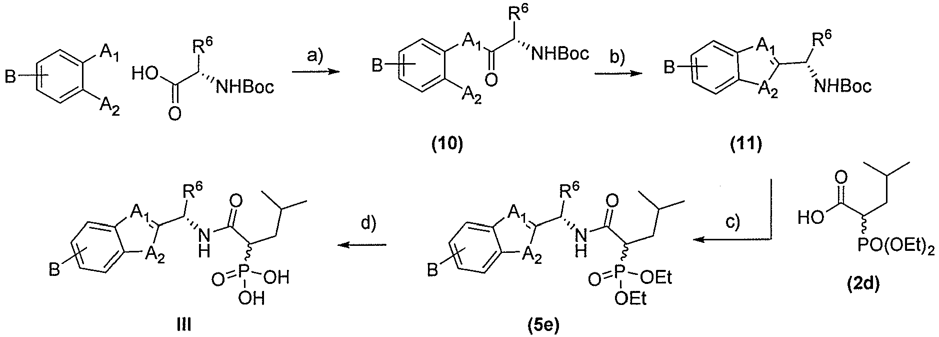

(a) TBTU, NMM, DMF, 0 °C-r.t., 2 d; (b) HOAc/toluene (1:1), 110 °C, 3 h; (c) i) HCl (4 M in 1,4-dioxane), DCM, 18 h, r.t.; ii) TBTU, NMM, DMF, 0 °C-rt, 21 h, d) TMSBr, DCM, r.t., 21 h, prep HPLC.

### General procedure L: Synthesis of benzannulated heteropentacycles (11)

The corresponding Boc-protected amino acid (1.0 equiv.) was dissolved in DMF (10 mL/mmol). After cooling to 0 °C, NMM (1.1 equiv.) and TBTU (1.1 equiv.) were added subsequently. The reaction mixture was stirred for 30 minutes, and the corresponding nucleophile (1.0) was added. After 3 days, saturated aqueous NH₄Cl solution was added, and the mixture extracted with EtOAc (x 3) and subsequently washed with saturated aqueous NaHCO₃ solution and saturated aqueous NaCl solution. The organic layer was dried over Na₂SO₄, filtered and and concentrated under reduced pressure, the crude product was redissolved in toluene (5 mL), and HOAc (5 mL) was added. The mixture was heated under reflux for 3 hours and quenched by the slow addition of saturated aqueous NaHCO₃ solution. After stirring for 20 minutes, the mixture was extracted with EtOAc (x 3) and washed with 1 M HCl. Subsequent washing with saturated aqueous NaHCO₃ solution (x 4) and saturated aqueous NaCl solution afforded the title compound after drying over anhydrous Na₂SO₄, filtration and concentration under reduced pressure, which was used in the next step without further purification.

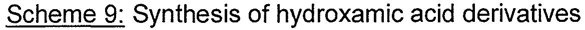

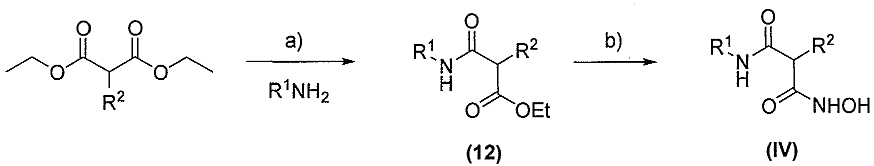

(a) i) NaOH, EtOH/H₂O, r.t., ii) EDC·HCl, DCM, r.t.; b) NH₂OH, KCN, MeOH, r.t.

### General procedure M: Synthesis of ethyl ester derivatives (12)

Diethyl 2-alkylmalonate (1.0 equiv.) was dissolved in EtOH/H₂O (4:1), and NaOH (1.2 equiv.) was added. The reaction was stirred at rt overnight. EtOH was evaporated under reduced pressure, saturated aqueous NaHCO₃ solution was added and extracted with DCM. The organic layer was discarded. The aqueous layer was acidified with 6 M HCl and extracted with DCM. The organic layer was washed with saturated aqueous NaCl solution, dried over anhydrous Na₂SO₄, filtered and evaporated under reduced pressure. The obtained mono-acid (1.2 equiv.) and EDC·HCl (1.2 equiv.) were added to a solution of the corresponding aniline (1.0 equiv.) in DCM. The resultant mixture was stirred at rt, until the starting aniline was consumed (monitored by TLC or LC-MS). The solution obtained was washed with 1 M HCl and saturated aqueous NaCl solution. The organic layer was dried over anhydrous Na₂SO₄, filtered and concentrated under reduced pressure to afford the crude product. The obtained crude product was purified using column chromatography.

### General procedure N: Synthesis of hydroxamic acid derivatives (IV)

Ethyl ester derivative **12** (1.0 equiv.) was dissolved in MeOH. NH₂OH 50 wt % in H₂O (same volume as methanol) was added, followed by KCN (0.2 equiv.). The mixture was stirred at rt overnight. Solvents were concentrated under reduced pressure, and the resultant oil was purified by preparative HPLC.

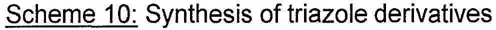

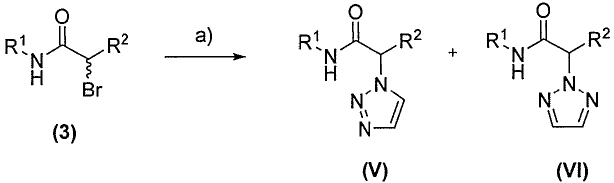

a) 1H-1,2,3-triazole, K₂CO₃, acetone, 70 °C

### General procedure O: Synthesis of 1H-1,2,3-triazole (V) and 2H-1,2,3-triazole (VI) derivatives.

*N*-aryl-2-bromo-2-alkylacetamide derivative **3** (1.0 equiv.) was placed in a crimp vial and dissolved in acetone. 1*H*-1,2,3-triazole (1.1 equiv.) and K₂CO₃ (1.1. equiv.) were added and the mixture heated to 70 °C overnight. EtOAc was added, the organic layer washed with water and saturated aqueous NaCl solution, dried over anhydrous Na₂SO₄, filtered and concentrated under reduced pressure. The crude was purified by preparative HPLC.

### II. Synthesis examples

### Example 1

### 2-Chloro-3-phenylpropanoic acid.

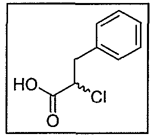

2-Chloro-3-phenylpropanoic acid was prepared according to general procedure A, using D,L-phenylalanine (1.00 g, 6.0 mmol) and sodium nitrite (1.46 g, 21.2 mmol). The crude product was obtained as light-yellow oil (1.05 g, 94%) and used without further purification. ¹H NMR (500 MHz, CDCl₃) δ ppm: 7.37-7.24 (m, 5H), 4.51 (dd, *J* = 7.8, 6.9 Hz, 1H), 3.42 (dd, *J* = 14.0, 6.7 Hz, 1H), 3.21 (dd, *J* = 14.1, 7.9 Hz, 1H). MS (ESI⁻) m/z 183.25 (M-H)⁻, 147.23 (M-H-HCl)⁻.

### 2-Chloro-N,3-diphenylpropanamide.

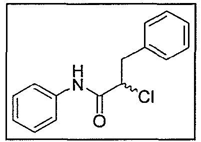

2-Chloro-*N*,3-diphenylpropanamide was prepared according to general procedure B-1 using 2-chloro-3-phenylpropanoic acid (934 mg, 5.06 mmol), EDC·HCl (786 mg, 5.06 mmol) and aniline (385 µL, 4.22 mmol). Purification was done via automated flash chromatography (hexane/EtOAc = 100:0 to 0:100). The product was obtained as white solid (404 mg, 31%). ¹H NMR (500 MHz, DMSO-*d₆*) δ ppm: 7.95 (s, 1H), 7.60-7.52 (m, 2H), 7.38-7.28 (m, 6H), 7.27-7.19 (m, 1H),7.11-7.04 (m, 1H), 4.76 (t, *J* = 7.5 Hz, 1H), 3.41 (dd, *J* = 13.8, 7.2 Hz, 1H), 3.13 (dd, *J* = 13.9, 7.8 Hz, 1H). MS (ESI⁺) m/z 260.08 (M+H)⁺.

### S-(1-Oxo-3-phenyl-1-(phenylamino)propan-2-yl) ethanethioate.

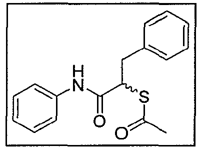

*S*-(1-oxo-3-phenyl-1-(phenylamino)propan-2-yl) ethanethioate was prepared according to general procedure C using 2-chloro-*N*,3-diphenylpropanamide (242 mg, 0.93 mmol) and potassium thioacetate (196 mg, 1.86 mmol). Purification was done via automated flash chromatography (hexane/EtOAc = 100:0 to 0:100). The product was obtained as colorless oil (127 mg, 46%). ¹H NMR (500 MHz, CDCl₃) δ ppm: 7.96 (br s, 1H), 7.46 (d, *J* = 8.2 Hz, 2H), 7.33-7.22 (m, 6H), 7.12-7.07 (m, 1H), 4.30 (t, *J* = 7.7 Hz, 1H), 3.46 (dd, *J* = 14.1, 8.5 Hz, 1H), 3.01 (dd, *J* = 14.1, 7.1 Hz, 1H), 2.38 (s, 3H), 1.59 (s, 3H). ¹³C NMR (126 MHz, CDCl₃) δ ppm: 197.3, 168.3, 137.6, 137.6, 129.2, 128.9, 128.6, 127.0, 124.4, 119.8, 48.5, 35.7, 30.4. MS (ESI⁺) m/z 300.17 (M+H)⁺, 258.10 (M-Ac+2H)⁺.

### 2-Mercapto-N,3-diphenylpropanamide (1).

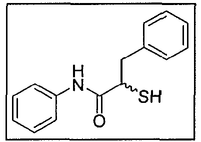

2-Mercapto-*N*,3-diphenylpropanamide was prepared according to general procedure D using *S*-(1-oxo-3-phenyl-1-(phenylamino) propan-2-yl)ethanethioate (127 mg, 0.42 mmol) and NaOH (50 mg, 1.3 mmol). Purification was done via automated flash chromatography (hexane/EtOAc = 100:0 to 0:100). The product was obtained as white solid (46 mg, 43%). ¹H NMR (500 MHz, CDCl₃) δ ppm: 8.02 (*br* s, 1H), 7.46 (d, *J =* 8.1 Hz, 2H), 7.36-7.29 (m, 4H), 7.29-7.23 (m, 4H), 7.14 (t, *J* = 7.6 Hz, 1H), 3.72 (dd, *J* = 14.8, 6.6 Hz, 1H), 3.38 (dd, *J* = 13.8, 6.5 Hz, 1H), 3.24 (dd, *J* = 13.8, 6.8 Hz, 1H), 2.11 (d, *J* = 8.9 Hz, 1H). ¹³C NMR (126 MHz, CDCl₃) δ ppm: 169.5, 137.3, 137.2, 129.4, 129.0, 128.6, 127.1, 124.8, 120.0, 45.9, 41.5. HRMS (ESI⁺) calculated for C₁₅H₁₅NOS [M+H]⁺ 258.0947, found 258.0943.

### Example 2

### S-(4-Methyl-1-oxo-1-(p-tolylamino)pentan-2-yl) ethanethioate.

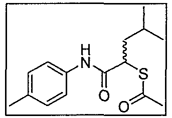

*S*-(4-Methyl-1-oxo-1-(*p*-tolylamino)pentan-2-yl) ethanethioate was synthesized in two steps. The first step was performed according to general procedure B-1, using p-toluidine (80 mg, 0.75 mmol), 2-bromo-4-methylpentanoic acid (175 mg, 0.90 mmol), EDC·HCl (172 mg, 0.90 mmol) and DCM (5 mL). The reaction was stirred at rt for 5 h. The crude product obtained was used in the next step without further purification. The second step was achieved according to general procedure C, using the crude product obtained from the first step, potassium thioacetate (171 mg, 1.49 mmol) and acetone (7 mL). The reaction was stirred at rt for 2.5 h. The crude product was purified using column chromatography (100% DCM). The product was obtained as beige solid (131 mg, 63% (over 2 steps)). ¹H NMR (500 MHz, DMSO-*d*₆) δ ppm: 10.23 (s, 1H), 7.46 (d, *J =* 8.0 Hz, 2H), 7.10 (d, *J =* 8.0 Hz, 2H), 4.27 (*br* t, *J* = 7.5 Hz, 1H), 2.35 (s, 3H), 2.24 (s, 3H), 1.88-1.75 (m, 1H), 1.62-1.40 (m, 2H), 0.95 (d, *J* = 6.5 Hz, 3H), 0.88 (d, *J* = 6.5 Hz, 3H). ¹³C NMR (126 MHz, DMSO-*d*₆) δ ppm: 194.5, 168.6, 136.2, 132.6, 129.1, 119.4, 46.4, 41.7, 30.3, 25.9, 22.5, 22.1, 20.5. HRMS (ESI⁺) calculated for C₁₅H₂₂NO₂S [M+H]⁺ 280.1371, found 280.1358.

### 2-Mercapto-4-methyl-N-(p-tolyl)pentanamide (2).

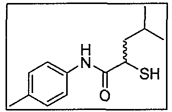

2-Mercapto-4-methyl-*N*-(*p*-tolyl)pentanamide was synthesized according to general procedure D, using *S*-(4-methyl-1-oxo-1-(*p-*tolylamino)pentan-2-yl) ethanethioate (90 mg, 0.32 mmol), NaOH (39 mg, 0.97 mmol) and MeOH (5 mL). The reaction was stirred at rt for 2 h. The crude product was purified using preparative HPLC (H₂O (HCOOH 0.05 %)-CH₃CN (HCOOH 0.05 %): 9.0-1.0 to 0.0-10.0). The product was obtained as beige solid (38 mg, 50%, MP = 90 °C). ¹H NMR (500 MHz, DMSO-*d*₆) δ ppm: 9.99 (s, 1H), 7.47 (d, *J* = 8.0 Hz, 2H), 7.11 (d, *J* = 8.0 Hz, 2H), 3.51 (br t, *J* = 7.8 Hz, 1H), 2.93 (s, 1H), 2.25 (s, 3H), 1.84-1.73 (m, 1H), 1.67-1.56 (m, 1H), 1.54-1.43 (m, 1H), 0.91 (d, *J* = 7.0 Hz, 3H), 0.86 (d, *J* = 6.5 Hz, 3H). ¹³C NMR (126 MHz, DMSO-*d*₆) δ ppm: 170.9, 136.5, 132.4, 129.2, 119.2, 44.4, 39.9, 25.8, 22.2, 22.1, 20.5. HRMS (ESI⁺) calculated for C₁₃H₂₀NOS [M+H]⁺ 238.1266, found 238.1254.

**Table 1: The following further compounds have been prepared according to the procedures described above:**

| 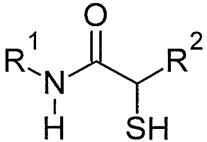 | | |
|---|---|---|
| **Example** | **R¹** | **R²** |
| **4** | 3,4-di-Cl-Ph | Bn |
| **5** | 4-OH-Ph | Bn |
| **6** | 2-Me-Ph | Bn |
| **7** | 3-Me-Ph | Bn |
| **8** | 4-Me-Ph | Bn |
| **9** | 4-NO₂-Ph | Bn |
| **10** | 4-OMe-Ph | Bn |
| **11** | Ph | 4-OH-Bn |
| **12** | Ph | 3-NO₂-4-OH-Bn |
| **13** | Ph | 4-Me-Bn |
| **14** | Ph | Me |
| **15** | 3,4-di-Cl-Ph | Me |
| **16** | 4-OMe-Ph | Me |
| **17** | 4-Ac-Ph | Me |
| **18** | 3,4-di-Cl-Ph | Et |
| **19** | 4-OMe-Ph | Et |
| **20** | 4-Ac-Ph | Et |
| **21** | 3,4-di-Cl-Ph | *i*Pr |
| **22** | 4-OMe-Ph | *i*Pr |
| **23** | 4-Ac-Ph | *i*Pr |
| **24** | Ph | *n*Pr |
| **25** | 4-Me-Ph | *n*Pr |
| **26** | 3,4-di-Cl-Ph | *n*Pr |
| **27** | 4-OMe-Ph | *n*Pr |
| **28** | 4-Ac-Ph | *n*Pr |
| **29** | 3,4-di-Cl-Ph | *n*Bu |
| **30** | 4-OMe-Ph | *n*Bu |
| **31** | 3,4-di-Cl-Ph | *i*Bu |
| **32** | 2-OMe-Ph | *i*Bu |
| **33** | 3-OMe-Ph | *i*Bu |
| **34** | 4-OMe-Ph | *i*Bu |
| **35** | 2,4-di-OMe-Ph | *i*Bu |
| **36** | 3,4-di-OMe-Ph | *i*Bu |
| **38** | 4-Cl-Ph | *i*Bu |
| **39** | 4-Ac-Ph | *i*Bu |
| **40** | 2-OH-Ph | *i*Bu |
| **41** | 4-OH-Ph | *i*Bu |
| 42 | 2-OH-4-Cl-Ph | *i*Bu |
| **43** | 3,4-di-Cl-Ph | *s*Bu |
| **44** | 4-OMe-Ph | *s*Bu |
| **45** | 3,4-di-Cl-Ph | cyclopropylmethyl |
| **46** | 4-OMe-Ph | cyclopropylmethyl |
| **47** | 3,4-di-Cl-Ph | cyclohexylmethyl |
| **48** | 4-OMe-Ph | cyclohexylmethyl |
| **49** | 3,4-di-Cl-Ph | CH₂OCH₃ |
| **50** | 4-OMe-Ph | CH₂OCH₃ |
| **51** | 3,4-di-Cl-Ph | CH₂COOMe |
| **52** | 4-OMe-Ph | CH₂COOMe |
| **53** | 4-Ac-Ph | CH₂COOH |
| **54** | 3,4-di-Cl-Ph | CH₂COOH |
| **55** | 2-benzothiazolyl | Bn |
| **56** | 6-methoxy-2-benzothiazolyl | Bn |
| **57** | 6-chloro-2-benzothiazolyl | Bn |
| **58** | 2-thiazolyl | Bn |
| **59** | methyl 2-aminothiophenyl-3-carboxylate | Bn |
| **60** | pyridin-3-yl | Bn |
| **61** | 2-benzoimidazolyl | Bn |
| **62** | 2-benzothiazolyl | *i*Bu |

### Example 63

### Diethyl (4-methyl-1-oxo-1-(p-tolylamino)pentan-2-yl)phosphonate.

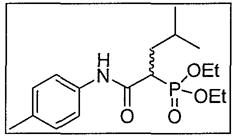

Diethyl (4-methyl-1-oxo-1-(*p*-tolylamino)pentan-2-yl) phosphonate was synthesized over two steps. The first step was performed according to general procedure B-1, using *p-*toluidine (92 mg, 0.85 mmol), 2-bromo-4-methylpentanoic acid (200 mg, 1.02 mmol), EDC·HCl (196 mg, 1.02 mmol) and DCM (15 mL). The reaction was stirred at rt for 5 h. The crude product obtained was used in the next step without further purification. The second step was achieved according to general procedure E, using the crude product obtained from the first step and triethyl phosphite (1.5 mL, 17.1 mmol). The crude product was purified using column chromatography (hexane/EtOAc = 1:1). The product was obtained as white solid (114 mg, 39% (over 2 steps)). ¹H NMR (500 MHz, CDCl₃) δ ppm: 8.41 (s, 1H), 7.39 (d, *J* = 8.4 Hz, 2H), 7.08 (d, *J* = 8.4 Hz, 2H), 4.21-4.08 (m, 4H), 2.97 (ddd, *J =* 22.6, 11.3, 3.5 Hz, 1H), 2.28 (s, 3H), 2.09-1.99 (m, 1H), 1.77-1.68 (m, 1H), 1.61-1.52 (m, 1H), 1.32 (q, *J =* 7.1 Hz, 6H), 0.97-0.91 (m, 6H). ¹³C NMR (126 MHz, CDCl₃) δ ppm: 165.6 (*J* = 1.8 Hz), 135.3, 133.8, 129.4, 119.8, 63.0 (*J* = 7.4 Hz), 62.8 (*J* = 6.4 Hz), 45.2 (*J* = 128.6 Hz), 35.8 (*J* = 4.6 Hz), 26.6 (*J =* 13.8 Hz), 23.2, 21.2, 20.8, 16.4 (*J* = 1.8 Hz), 16.4 (*J* = 2.8 Hz). MS (ESI⁺) m/z 342.2 [M+H]⁺.

### (4-Methyl-1-oxo-1-(p-tolylamino)pentan-2-yl)phosphonic acid (63).

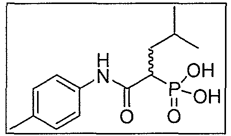

(4-Methyl-1-oxo-1-(*p*-tolylamino)pentan-2-yl)phosphonic acid was synthesized according to general procedure F, using diethyl (4-methyl-1-oxo-1-(*p*-tolylamino)pentan-2-yl)phosphonate (110 mg, 0.32 mmol), bromotrimethylsilane (213 µL, 1.61 mmol) and DCM (6 mL). The reaction was stirred at rt overnight. Then, MeOH (10 mL) was added, the reaction mixture was stirred for additional 30 minutes, and the solvent evaporated under the reduced pressure. The crude product was purified using preparative HPLC (CH₃CN (HCOOH 0.05%)-H₂O (HCOOH 0.05%): 1.0:9.0 to 10.0:0.0). The product was obtained as white solid (56 mg, 71%). ¹H NMR (500 MHz, DMSO-*d₆*) δ ppm: 9.84 (s, 1H), 7.47 (d, *J =* 8.4 Hz, 2H), 7.07 (d, *J =* 8.2 Hz, 2H), 2.95 (ddd, *J* = 22.4, 11.4, 2.9 Hz, 1H), 2.22 (s, 3H), 1.99-1.89 (m, 1H), 1.51-1.34 (m, 2H), 0.87-0.82 (m, 6H). ¹³C NMR (126 MHz, DMSO-*d*₆) δ ppm: 167.6 (*J* = 4.6 Hz), 137.0, 131.8, 129.0, 119.0, 46.0 (*J =* 126.8 Hz), 35.8 (*J =* 3.7 Hz), 26.5 (*J* = 14.7 Hz), 23.3, 21.4, 20.5. ³¹P NMR (202 MHz, DMSO-*d*₆) δ ppm: 20.1. HRMS (ESI⁻) calculated for C₁₃H₁₉NO₄P [M-H]⁻ 284.1057, found 284.1058.

### Example 95

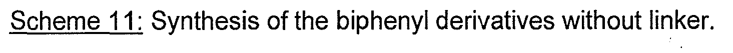

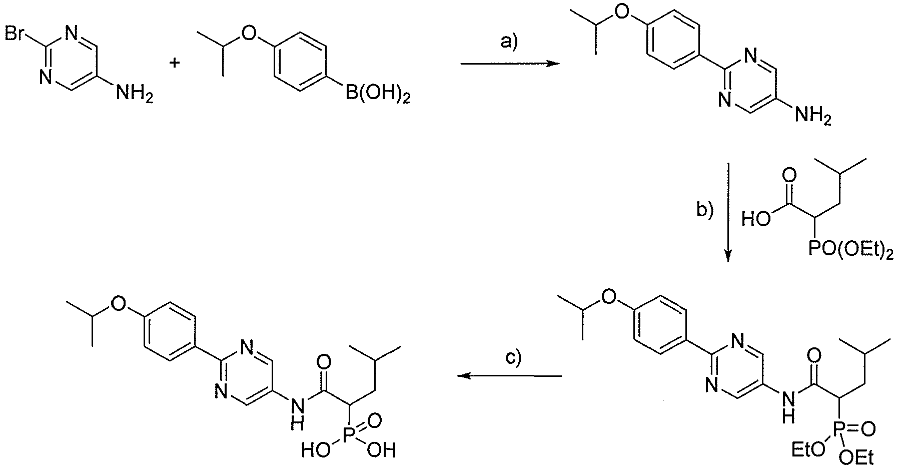

a) Pd(PPh₃)₄, dioxane/H₂O (4:1, v:v, 3 mL), NaOH (2M), microwave (150 °C, 150 W, 20 min); b) EDC·HCl, DCM, r.t., 2 h; c) i) TMSBr, DCM, r.t., overnight; ii) MeOH, r.t.

### Diethyl (1-((2-(4-isopropoxyphenyl)pyrimidin-5-yl)amino)-4-methyl-1-oxopentan-2-yl)phosphonate.

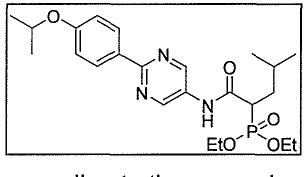

Diethyl (1-((2-(4-isopropoxyphenyl)pyrimidin-5-yl)amino)-4-methyl-1-oxopentan-2-yl)phosphonate was synthesized according to general procedure G, using 2-(4-isopropoxyphenyl)pyrimidin-5-amine synthesized according to the general procedure G-4 (60 mg, 0.26 mmol), **2d** (100 mg, 0.40 mmol), EDC·HCl (100 mg, 0.52 mmol) in DCM (5 mL). The crude product was purified using column chromatography (DCM/MeOH from 0% to 3%). The product was obtained as white solid (84 mg, 69%). ¹H NMR (500 MHz, CDCl₃) δ ppm:10.27 (s, 1H), 8.79 (s, 2H), 7.90 (d, *J* = 8.8 Hz, 2H), 6.69 (d, *J* = 8.8 Hz, 2H), 4.50 (hept, *J* = 6.0 Hz, 1H), 4.32 - 4.17 (m, 2H), 4.10 (p, *J =* 7.2 Hz, 2H), 3.34 (ddd, *J* = 22.8, 11.3, 2.8 Hz, 1H), 2.17 - 2.06 (m, 1H), 1.60 - 1.52 (m, 1H), 1.43 (dtd, *J* = 13.1, 10.2, 2.9 Hz, 1H), 1.31 (ddd, *J* = 19.2, 12.6, 6.2 Hz, 12H), 0.86 (dd, *J =* 13.1, 6.5 Hz, 6H). MS (ESI⁺) m/z 464 [M+H]⁺.

### (1-((2-(4-Isopropoxyphenyl)pyrimidin-5-yl)amino)-4-methyl-1-oxopentan-2-yl)phosphonic acid (95).

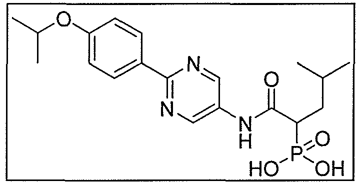

(1-((2-(4-isopropoxyphenyl)pyrimidin-5-yl)amino)-4-methyl-1-oxopentan-2-yl)phosphonic acid (**95**) was synthesized according to general procedure F, using diethyl (1-((2-(4-isopropoxyphenyl)pyrimidin-5-yl)amino)-4-methyl-1-oxopentan-2-yl)phosphonate (65 mg, 0.14 mmol), bromotrimethylsilane (100 µL, 0.72 mmol) and DCM (4 mL). The reaction was stirred at rt overnight. Then, MeOH (4 mL) was added, the reaction mixture was stirred for additional 30 minutes, and the solvent evaporated under the reduced pressure. The crude product was purified using preparative HPLC (CH₃CN (HCOOH 0.05%)-H₂O (HCOOH 0.05%). The product was obtained as white solid (41 mg, 72%). ¹H NMR (500 MHz, DMSO-*d*₆) δ ppm: 10.46 (s, 1H), 9.05 (s, 2H), 8.24 (d, *J* = 8.9 Hz, 2H), 7.01 (d, *J =* 8.9 Hz, 2H), 4.70 (dt, *J =* 12.1, 6.0 Hz, 1H), 3.04 (ddd, *J =* 22.4, 11.1, 2.3 Hz, 1H), 2.00 (ddd, *J* = 15.4, 10.0, 3.7 Hz, 1H), 1.61 - 1.35 (m, 2H), 1.30 (d, *J* = 6.0 Hz, 6H), 0.88 (d, *J* = 6.3 Hz, 6H). ¹³C NMR (126 MHz, DMSO-*d*₆) δ ppm: 169.24, 169.20, 159.76, 158.52, 147.75, 132.71, 129.72, 129.36, 115.87, 69.75, 47.05, 46.05, 36.10, 36.06, 26.98, 26.87, 23.59, 22.28, 21.82. ³¹P NMR (202 MHz, DMSO-*d*₆) δ ppm: 18.93. MS (ESI⁺) m/z 408 [M+H]⁺.

### Example 108

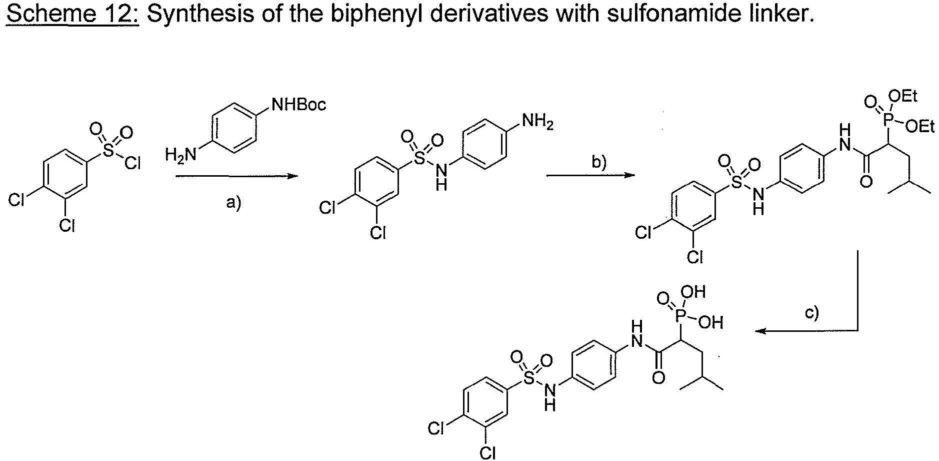

a) i) Et₃N, DCM, 0 °C-r.t., 8 h, ii) TFA, DCM, r.t., 2 h, b) EDC·HCl, DCM, r.t., overnight, c) TMSBr, DCM, r.t., overnight.

### N-(4-Aminophenyl)-3,4-dichlorobenzenesulfonamide.

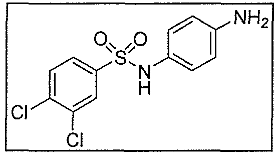

*N*-(4-Aminophenyl)-3,4-dichlorobenzenesulfonamide was synthesized according to general procedure G-2, using tert-butyl (4-aminophenyl)carbamate (300 mg, 1.44 mmol), Et₃N (240 µL, 1.73 mmol) and 3,4-dichlorobenzenesulfonyl chloride (250 µL, 1.58 mmol) in DCM (10 mL). Reaction mixture was stirred at rt for 8 h. Precipitate was filtered and filtrate purified on column chromatography (hexanes/EtOAc = 7/3) giving tert-butyl (4-((3,4-dichlorophenyl)sulfonamido)phenyl)carbamate (316 mg, 52%). Obtained tert-butyl (4-((3,4-dichlorophenyl)sulfonamido)phenyl)carbamate was suspended in 3.5 mL DCM/TFA (3:1) and stirred at rt for 2 h. After the workup, *N*-(4-aminophenyl)-3,4-dichlorobenzenesulfonamide (193 mg, 81%) was obtained as beige solid. ¹H NMR (500 MHz, DMSO-*d*₆) δ ppm: 9.65 (br s, 1H), 7.81 (d, *J* = 8.4 Hz, 1H), 7.77 (d, *J =* 2.0 Hz, 1H), 7.54 (dd, *J* = 8.5, 2.1 Hz, 1H), 6.66 (d, *J* = 8.7 Hz, 2H), 6.40 (d, *J* = 8.7 Hz, 2H), 5.01 (br s, 2H). MS (ESI⁻) m/z 314.99 [M-H]⁻.

### Diethyl (1-((4-((3,4-dichlorophenyl)sulfonamido)phenyl)amino)-4-methyl-1-oxopentan-2-yl)phosphonate.

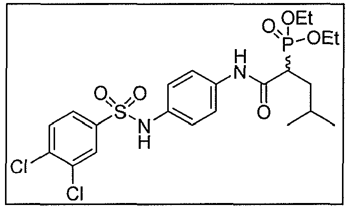

Diethyl (1-((4-((3,4-dichlorophenyl)sulfonamido)phenyl)amino)-4-methyl-1-oxopentan-2-yl)phosphonate was synthesized according to general procedure G, using *N*-(4-aminophenyl)-3,4-dichlorobenzenesulfonamide (84 mg, 0.26 mmol), **2d** (100 mg, 0.40 mmol), EDC·HCl (100 mg, 0.52 mmol), HOBt (80 mg, 0.52 mmol) and DIPEA (110 µL, 0.62 mmol) in DCM (5 mL). The crude product was purified using column chromatography (hexanes/EtOAc = 3/7). The product was obtained as white foam (84 mg, 58%). ¹H NMR (500 MHz, DMSO-*d*₆) δ ppm: 10.27 (br s, 1H), 10.11 (s, 1H), 7.88 (d, *J =* 2.1 Hz, 1H), 7.83 (d, *J =* 8.4 Hz, 1H), 7.61 (dd, *J =* 8.4, 2.1 Hz, 1H), 7.49-7.44 (m, 2H), 7.05-7.00 (m, 2H), 4.06-3.95 (m, 4H), 3.15 (ddd, *J* = 22.6, 11.3, 3.1 Hz, 1H), 1.98-1.88 (m, 1H), 1.49-1.29 (m, 2H), 1.18 (dt, *J =* 9.1, 7.1 Hz, 6H), 0.85 (d, *J* = 6.6 Hz, 6H). MS (ESI⁺) m/z 551.12 [M+H]⁺.

### (1-((4-((3,4-Dichlorophenyl)sulfonamido)phenyl)amino)-4-methyl-1-oxopentan-2-yl)phosphonic acid (108).

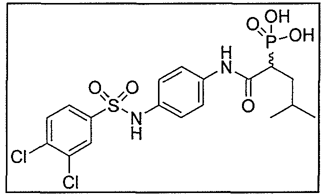

(1-((4-((3,4-Dichlorophenyl)sulfonamido)phenyl)amino)-4-methyl-1-oxopentan-2-yl)phosphonic acid was synthesized according to general procedure F, using diethyl (1-((4-((3,4-dichlorophenyl)sulfonamido)phenyl)amino)-4-methyl-1-oxopentan-2-yl)phosphonate (80 mg, 0.14 mmol), bromotrimethylsilane (100 µL, 0.72 mmol) and DCM (4 mL). The reaction was stirred at rt overnight. Then, MeOH (4 mL) was added, the reaction mixture was stirred for additional 30 minutes and the solvent evaporated under the reduced pressure. The crude product was purified using preparative HPLC (CH₃CN (HCOOH 0.05%)-H₂O (HCOOH 0.05%): 1.0:9.0 to 10.0:0.0). The product was obtained as white solid (48 mg, 70%). ¹H NMR (500 MHz, DMSO-*d*₆) δ ppm: 10.22 (s, 1H), 9.92 (s, 1H), 7.89 (d, *J = 2.1* Hz, 1H), 7.82 (d, *J* = 8.4 Hz, 1H), 7.60 (dd, *J* = 8.4, 2.1 Hz, 1H), 7.51-7.46 (m, 2H), 7.01-6.97 (m, 2H), 2.93 (ddd, J = 22.5, 11.3, 2.8 Hz, 1H), 1.97-1.88 (m, 1H), 1.49-1.34 (m, 2H), 0.83 (d, *J* = 6.4 Hz, 6H). ¹³C NMR (126 MHz, DMSO-*d*₆) δ ppm: 167.8 (d, *J* = 5.5 Hz), 139.66, 136.86, 135.99, 132.15, 131.73, 131.51, 128.40, 126.85, 122.33, 119.82, 46.0 (d, *J* = 126.8 Hz), 35.7 (d, *J* = 3.7 Hz), 26.4 (d, *J* = 14.7 Hz), 23.2, 21.3. ³¹P NMR (202 MHz, DMSO-*d*₆) δ ppm: 19.8. HRMS (ESI⁻) calculated for C₁₈H₂₀Cl₂N₂O₆PS [M-H]⁻ 493.0162, found 493.0156.

### Example 147

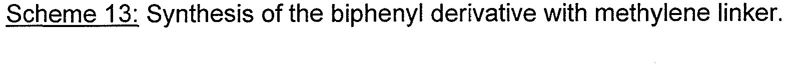

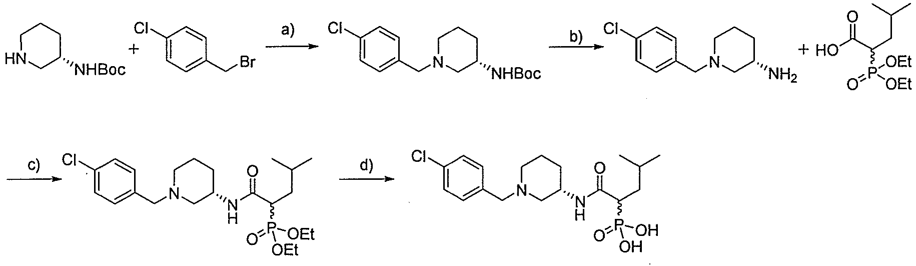

a) Et₃N (3 equiv.), DCM, r.t., 16 h, b) TFA (5 equiv.), DCM, rt, 19h, c) EDC·HCl (1.2 equiv.), HOBt (1.2 equiv.), DIPEA (2.4 equiv.), DMF, r.t., 18 h, d) TMSBr (7 equiv.), DCM, r.t., 23 h.

### Tert-butyl-(S)-(1-(4-chlorobenzyl)piperidin-3-yl)carbamate.

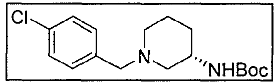

To a heat-dried 50 mL Schlenk tube was added (S)-3-(Boc-amino)piperidine (200.3 mg, 1 mmol, 1 equiv.) and 4-chlorobenzyl bromide (205.5 mg, 1 mmol, 1 equiv.) and dissolved in dry DCM (2.5 mL, 0.4 M) followed by addition of Et₃N (303.6 mg, 418.1 µL, 3 mmol, 3 equiv.) under nitrogen atmosphere. The reaction mixture was stirred at r.t. and after completion of the reaction (LCMS, 16 h) water (5 mL) was added, and the reaction mixture was extracted with DCM (3 × 10 mL). The combined organic phases were dried over anhydrous Na₂SO₄, filtered, and volatiles were removed under reduced pressure to obtain the titled compound as an off-white solid (322 mg), which was used in the next step without further purification.

### (S)-1-(4-Chlorobenzyl)piperidin-3-amine.

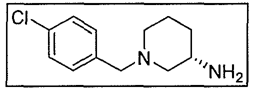

In a 50 mL Schlenk tube, crude tert-butyl-(S)-(1-(4-chlorobenzyl)piperidin-3-yl)carbamate (322 mg, approx. 0.99 mmol, 1 equiv.) was dissolved in DCM (3 mL, 0.4 M). To a resulting solution was added TFA (383 µL, 5 equiv.), and the reaction mixture was kept stirring at rt. After completion of the reaction (LCMS, 19 h) solvent was removed under reduced pressure to obtain an oily residue, which was treated with 2M NaOH solution and extracted with EtOAc (3 × 20 mL). The combined organic phases were dried over anhydrous Na₂SO₄, filtered, and volatiles were removed under reduced pressure to obtain the title compound as an oil (207 mg), which was used in the next step without further purification.

### Diethyl (1-(((S)-1-(4-chlorobenzyl)piperidin-3-yl)amino)-4-methyl-1-oxopentan-2-yl)phosphonate.

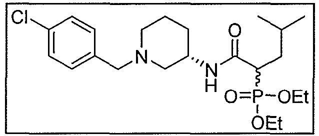

To a 4 mL glass vial were added (S)-1-(4-chlorobenzyl)piperidin-3-amine (50 mg, approx. 0.22 mmol, 1 equiv.), 2-(diethoxyphosphoryl)-4-methylpentanoic acid 2d (84.2 mg, 0.33 mmol, 1.5 equiv.), HOBt·H₂O (68.2 mg, 0.44 mmol, 2 equiv.) and dissolved in DMF (1.5 mL). To the resulting solution was added EDC·HCl (85.3 mg, 0.44 mmol, 2 equiv.) and DIPEA (93 µL, 0.53 mmol, 2.4 equiv.) and the reaction was kept stirring at rt. After complete conversion (LCMS, 18 h), water (5 mL) and EtOAc (5 mL) were added to the reaction. The organic phase was removed, and the aqueous phase was extracted with EtOAc (3 × 10 mL). The combined organic phases were passed through a pad of anhydrous Na₂SO₄, filtered and concentrated under reduced pressure to obtain the title compound (55 mg), which was used in the next step without further purification.

### (1-(((S)-1-(4-Chlorobenzyl)piperidin-3-yl)amino)-4-methyl-1-oxopentan-2-yl)phosphonic acid (147).

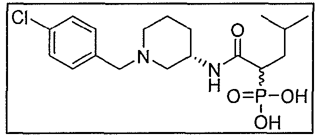

To a heat-dried 25-mL Schlenk tube were added crude diethyl (1-(((S)-1-(4-chlorobenzyl)piperidin-3-yl)amino)-4-methyl-1-oxopentan-2-yl)phosphonate (53 mg, 0.115 mmol, 1 equiv.) and dry DCM (1 mL) under argon. To the resulting solution was added dropwise bromotrimethylsilane (107 µL, 0.81 mmol, 7 equiv.), and the reaction kept stirring at r.t.. After completion of the reaction (LCMS, 23h), MeOH (2 mL) was added and stirred at r.t. for 30 min. The volatiles were removed under reduced pressure and the crude was purified on preparative HPLC to obtain the title compound as white amorphous solid (21 mg, 0.052 mmol, 45%).

### Mixture of diastereomers:

Major diastereomer: ¹H NMR (500 MHz, DMSO-ds) δ ppm: 10.05 (s, 1H), 7.72-7.24 (m, 4H), 4.43-4.18 (m, 2H), 4.08-3.92 (m, 1H), 3.44-3.04 (m, 2H), 3.00-2.30 (m, 3H), 1.98-1.80 (m, 2H), 1.80-1.62 (m, 2H), 1.54-1.27 (m, 3H), 0.81 (dd, *J* = 6.4, 5.8 Hz, 6H). ¹³C NMR (126 MHz, DMSO-*d*₆) δ 169.4, 158.6 (dd, *J* = 31.2, 30.6 Hz), 134.8, 133.8, 133.7, 129.3, 58.7, 54.3, 51.0, 45.9 (d, *J* = 124.0 Hz), 43.9, 36.0, 26.9 (t, *J* = 14.5 Hz), 23.6, 21.8. ³¹P NMR (202 MHz, DMSO-*d*₆) δ ppm: 19.7.

Minor diastereomer: ¹H NMR (500 MHz, DMSO-*d*₆) δ ppm: 10.05 (s, 1H), 8.39-7.77 (m, 4H), 4.47-4.18 (m, 3H), 3.44-3.04 (m, 2H), 3.00-2.30 (m, 3H), 1.98-1.80 (m, 2H), 1.80-1.62 (m, 2H), 1.54-1.27 (m, 3H), 0.81 (dd, *J* = 6.4, 5.8 Hz, 6H). ¹³C NMR (126 MHz, DMSO-*d*₆) δ 169.7, 158.6 (dd, *J* = 31.2, 30.6 Hz), 134.9, 133.8, 133.7, 129.2, 58.7, 54.3, 51.5, 45.6 (d, *J* = 125.0 Hz), 43.9, 27.8 (dd, *J* = 33.1, 15.6 Hz), 23.6, 21.8. ³¹P NMR (202 MHz, DMSO-*d*₆) δ ppm: 19.6.

HRMS (ESI+) calculated for C₁₈H₂₉ClN₂O₄P [M+1]⁺ 403.1553, found 403.1537.

### Example 151

### Tert-butyl (3-((3,4-dichlorophenyl)carbamoyl)bicyclo[1.1.1]pentan-1-yl)carbamate.

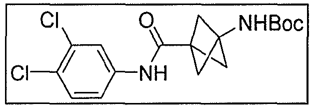

3-((*tert*-butoxycarbonyl)amino)bicyclo[1.1.1]pentane-1-carboxylic acid (46.6 mg, 0.205 mmol, 1.0 equiv.) was prepared as previously described (https://doi.org/10.1002/ejoc.201701296) and dissolved in DMF (2 mL), cooled to 0 °C and TBTU (73.0 mg, 0.23 mmol, 1.1 equiv.) was added, followed by addition of NMM (24 µL, 0.23 mmol, 1.1 equiv.). The reaction mixture was stirred at the indicated temperature for 1 hour and then 3,4-dichloroaniline (33 mg, 0.205 mmol) was added. After stirring 16 hours and warming up to rt, EtOAc was added and subsequently washed with saturated NaHCO₃ solution, 1 M HCl, water and saturated aqueous NaCl solution. The organic layer was dried over Na₂SO₄, filtered and concentrated under reduced pressure to afford the title compound as a colorless solid (42 mg, 0.113 mmol, 55%), which was used in the next step without further purification.. ¹H NMR (CDCl₃, 500 MHz) δ ppm: 7.77-7.76 (m, 1 H), 7.38-7.37 (m, 2 H), 7.12 (bs, 1 H), 5.00 (bs, 1 H), 2.37 (s, 6 H), 1.47 (s, 9 H). ¹³C NMR (CDCl₃, 126 MHz) δ ppm: 167.4, 136.8, 132.9, 130.6, 121.4, 118.8, 53.8, 45.1, 28.4. MS (ESI+): m/z [M+H]⁺ = 372

### Diethyl (1-((3-((3,4-dichlorophenyl)carbamoyl)bicyclo[1.1.1]pentan-1-yl)amino)-4-methyl-1-oxopentan-2-yl)phosphonate.

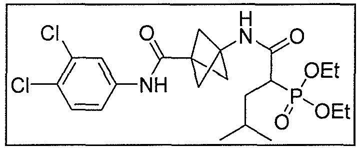

The title compound was prepared according to general procedure I. *Tert-butyl* (3-((3,4-dichlorophenyl)carbamoyl)bicyclo[1.1.1]pentan-1-yl)carbamate (40 mg, 0.108 mmol) and HCl (0.27 mmol, 1.08 mmol, 4 M in dioxane) were used for the deprotection. 2-(diethoxyphosphoryl)-4-methylpentanoic acid 2d (30 mg, 0.119 mmol), NMM (31 µL, 0.298 mmol) and TBTU (43 mg, 0.131 mmol) were used in the peptide coupling to afford the title compound as a yellow oil (36.7 mg, 0.073 mmol, 67%), which was used in the next step without further purification. MS (ESI+): m/z [M+H]⁺ = 506.

### (1-((3-((3,4-Dichlorophenyl)carbamoyl)bicyclo[1.1.1]pentan-1-yl)amino)-4-methyl-1-oxopentan-2-yl)phosphonic acid (151).

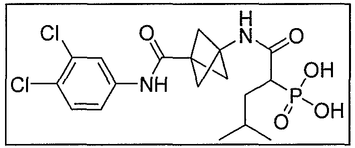

The title compound was prepared according to general procedure F. Diethyl (1-((3-((3,4-dichlorophenyl)carbamoyl)bicyclo[1.1.1]pentan-1-yl)amino)-4-methyl-1-oxopentan-2-yl)phosphonate (36 mg, 0.071 mmol) and bromotrimethylsilane (47 µL, 0.356 mmol) were used to afford the title compound as a colorless solid (8.6 mg, 0.019 mmol, 27%) after purification *via* preperative HPLC.
Mixture of diastereomeres ¹H NMR (500 MHz, acetone-*d*₆) δ ppm: 8.05 (d, *J =* 2.3 Hz, 1 H), 7.62 (dd, *J* = 8.9 Hz, *J* = 2.3 Hz, 1 H), 7.44 (d, *J* = 8.9 Hz, 1 H), 3.02-2.94 (m, 1 H), 2.40 (s, 6 H), 2.01-1.98 (m, 1 H), 1.65-1.61 (m, 1 H), 1.59-1.53 (m, 1 H), 0.92 (d, *J = 6.3* Hz, 6 H). ¹³C NMR (126 MHz, acetone-*d*₆) δ ppm: 169.9, 167.8, 138.9, 131.6, 130.4, 125.6, 121.0, 119.3, 53.8, 45.8, 45.0, 44.8, 38.2, 35.7, 26.7, 22.6, 21.0. ³¹P NMR (202 MHz, acetone-*d*₆) δ ppm: 24.7, 24.6. HRMS (ESI+) calculated for C₁₈H₂₄Cl₂N₂O₃P [M+H]⁺ 449.0794, found: 449.0798.

### Example 152

### Tert-Butyl (S)-(1-((3,4-dichlorophenyl)amino)-3-methyl-1-oxobutan-2-yl)carbamate.

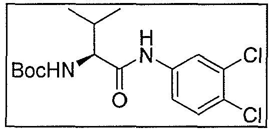

(Tert-butoxycarbonyl)-L-valine (434 mg, 2.0 mmol, 1.0 equiv.) was dissolved in THF (20 mL, 0.1 M) and cooled down to -20 °C. Then NMM (0.55 ml, 2.5 equiv.) and isobutyl chloroformate (0.259 mL, 1.0 equiv.) were added dropwise. The reaction mixture was stirred at this temperature for 30 minutes and then the aniline (324 mg, 2 mmol, 1.0 equiv.), dissolved in THF (1 M), was added. After the reaction mixture had reached rt, it was diluted with EtOAc. The organic phase was washed with KHSO₄ (1 N) solution, saturated aqueous NaHCO₃ solution and saturated aqueous NaCl solution, dried over Na₂SO₄, filtered and the solvent was removed under reduced pressure. Purification by column chromatography (SiO₂, hexanes/EtOAc 9:1) afforded the corresponding tert-butyl (S)-(1-((3,4-dichlorophenyl)amino)-3-methyl-1-oxobutan-2-yl)carbamate (523.8 mg, 1.44 mmol, 72% yield). ¹H NMR (500 MHz, CDCl₃) δ ppm: 8.85 (br s, 1H), 7.70 (br s, 1H), 7.2-7.3 (m, 2H), 5.27 (br d, 1H, *J* = 8.2 Hz), 4.06 (br t, 1H, *J* = 7.6 Hz), 2.15 (br d, 1H, *J* = 6.1 Hz), 1.47 (s, 9H), 1.03 (dd, 6H, *J* = 2.7, 6.7 Hz). ¹³C NMR (126 MHz, CDCl₃,) δ ppm: 170.7, 137.2, 132.5, 130.2, 121.2, 118.6, 61.1, 30.5, 28.3, 19.3, 18.4. HRMS (ESI+) calculated for C₁₆H₂₃Cl₂N₂O₃ [M+H]⁺ 361.1080, found 361.1080.

### (1-(((S)-1-((3,4-Dichlorophenyl)amino)-3-methyl-1-oxobutan-2-yl)amino)-4-methyl-1-oxopentan-2-yl)phosphonic acid (152).

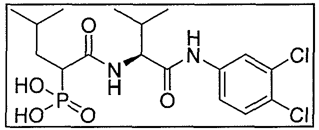

*Tert-butyl* (S)-(1-((3,4-dichlorophenyl)amino)-3-methyl-1-oxobutan-2-yl)carbamate (100.0 mg, 0.28 mmol, 1.0 equiv.) was dissolved in DCM (0.1 M) and treated at 0 °C with HCl (0.69 mL, 10.0 equiv., 4 M in dioxane). The mixture was warmed up to rt and after complete conversion (TLC), the solvent was removed under reduced pressure with the result that the crystalline hydrochloride remained, which was subsequently dissolved in DMF (2.8 mL, 0.1 M). 2-(diethoxyphosphoryl)-4-methylpentanoic acid 2d (77.7 mg, 0.308 mmol, 1.1 equiv.) was added to this solution, and the reaction mixture was cooled to 0 °C. Coupling was achieved by TBTU (98.9 mg, 0.308 mmol, 1.1 equiv.) and NMM (0.08 mL, 2.5 equiv.). The reaction mixture was warmed up to rt and after complete reaction (TLC) diluted with EtOAc and washed successively with 1N KHSO₄ solution, saturated NaHCO₃ solution and saturated aqueous NaCl solution. After drying over Na₂SO₄ and removing the solvent under reduced pressure, residue diethyl (1-(((S)-1-((3,4-dichlorophenyl)amino)-3-methyl-1-oxobutan-2-yl)amino)-4-methyl-1-oxopentan-2-yl)phosphonate (136.7 mg, 0.28 mmol, quant.) was used without further purification for the next step. To a solution of diethyl phosphonate dipeptides (136.7 mg, 0.28 mmol) in DCM (0.1 M), bromotrimethylsilane (0.26 mL, 1.93 mmol) was added dropwise over a period of 15 minutes. The reaction mixture was stirred at rt overnight. Then MeOH was added and stirred for 30 minutes at rt to cleave the previously formed TMS ester. The solvents were removed under reduced pressure and the crude product was purified *via* a Waters Autopurifier System (APS) with a Phenomenex Gemini C18 column (250 × 4.6 mm, particle size 5 µm) using mass trigger detection to afford dipeptide 152 (51.7 mg, 0.12 mmol, 43%) as a white amorphous solid.

### Mixture of diastereomers:

Major diastereomer: ¹H NMR (500 MHz, MeOH-*d*₄,) δ ppm: 8.04 (d, 1H, *J* = 2.4 Hz), 7.62 (dd, 1H, *J* = 2.4, 8.9 Hz), 7.42 (d, 1H, *J* = 8.9 Hz), 4.45 (d, 1H, *J = 5.0* Hz), 3.24 (ddd, 1H, *J* = 2.7, 11.6, 23.3 Hz), 2.42 (qd, 1H, *J* = 6.9, 12.1 Hz), 1.47-1.61 (m, 3H), 0.9-1.1 (m, 19H), 0.99 (d, 3H, *J* = 7.02 Hz), 0.98 (d, 3H, *J* = 6.87 Hz), 0.95 (d, 6H, *J =* 6.56 Hz). ¹³C NMR (126 MHz, MeOH-*d*₄) δ ppm: 172.7, 172.5 (d, *J* = 4.6 Hz), 139.6, 133.2, 131.5, 128.2, 123.5, 121.6, 60.7, 47.1, 46.0, 36.3 (d, *J* = 4.6 Hz), 31.2, 28.4 (d, *J* = 15.6 Hz), 23.7, 21.8, 19.9, 17.8 ³¹P NMR (202 MHz, MeOH-*d*₄) δ ppm: 22.7.

Minor diastereomer: ¹H NMR (500 MHz, MeOH-*d*₄) δ ppm: 7.92 (dd, 1H, *J* = 0.61, 1.83 Hz), 7.44-7.46 (m, 1H), 4.27 (d, 1H, *J* = 7.48 Hz), 3.24 (ddd, 1H, *J = 2.7,* 11.9, 22.4 Hz), 1.95-2.20 (m, 2H), 1.28-1.35 (m, 2H), 1.05 (d, 3H, *J* = 6.71 Hz), 1.00 (d, 3H, *J =* 6.71 Hz), 0.92 (d, 3H, *J* = 6.10 Hz), 0.90 (d, 3H, *J* = 6.26 Hz). ¹³C NMR (126 MHz, MeOH-*d*₄) δ ppm: 172.6, 172.0 (d, *J* = 4.6 Hz), 139.7, 133.5, 131.7, 128.1, 122.8, 120.8, 61.5, 46.8, 45.8, 37.3 (d, *J* = 4.6 Hz), 32.3, 28.1 (d, *J* = 15.6 Hz), 23.8, 21.9, 19.1. ³¹P NMR (202 MHz, MeOH-*d*₄) δ ppm: 22.4. HRMS (ESI+) calculated for C₁₇H₂₆Cl₂N₂O₅P [M+H]⁺ 439.0956, 439.0935.

### Example 170

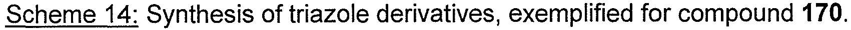

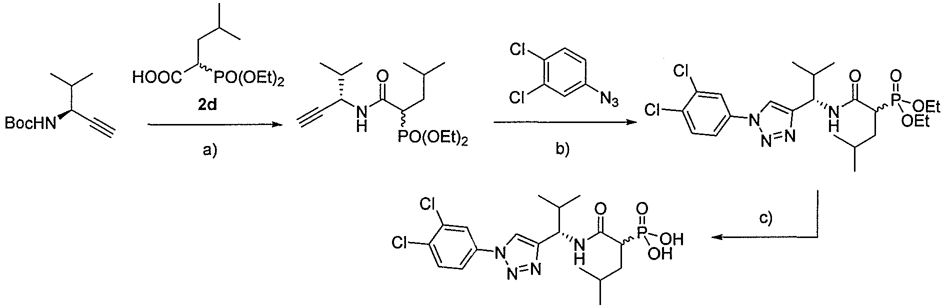

_(a) i) HCl (4 M in dioxane), DCM, r.t., 18 h ; ii) TBTU, NMM, DMF 0 °C-r.t., 22 h, 76% (over 2 steps); (b) CuSO₄·5 H₂O, Na ascorbate, tBuOH/H₂O/MeOH (2:2:1) r.t., 14 h, 84%; (c) TMSBr, DCM, r.t., 23 h, prep HPLC, 26%.

### Diethyl (4-methyl-1-(((S)-4-methylpent-1-yn-3-yl)amino)-1-oxopentan-2-yl)phosphonate.

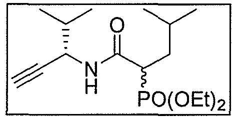

Compound tert-butyl (S)-(4-methylpent-1-yn-3-yl)carbamate (282 mg, 1.43 mmol), which was synthesized as previously reported (https://doi.org/10.1002/anie.201601564), was dissolved in DCM (11 mL) and HCl (, 2.85 mL, 11.4 mmol, 4 M in dioxane) was added at rt to afford the corresponding Boc-deprotected alkinyl amine hydrochloride. The mixture was stirred for 18 h and then concentrated under reduced pressure. In the meantime, a mixture of compound **2d** (396 mg, 1.57 mmol) and TBTU (562 mg, 1.75 mmol) in DMF (7.5 mL) was cooled to 0 °C, and NMM (0.91 mL, 3.58 mmol) was added. The reaction mixture was stirred for 30 min and then previously prepared Boc-deprotected alkinyl amine hydrochloride was dissolved in DMF (7.5 mL) and added dropwise at 0 °C. The mixture was stirred for 22 h and allowed to warm to rt. After addition of EtOAc, the organic layer was subsequently washed with saturated aqueous NaHCO₃ solution, 1 M HCl, water and saturated aqueous NaCl solution. The organic layer was dried over Na₂SO₄ and the solvent removed under reduced pressure. The crude product was purified *via* automated combiflash purification (Teledyne ISCO) and yielded 359 mg of diethyl (4-methyl-1-(((S)-4-methylpent-1-yn-3-yl)amino)-1-oxopentan-2-yl)phosphonate (1.08 mmol, 76% over 2 steps). ¹H NMR (500 MHz, CDCl₃) δ ppm: 6.68-6.58 (m, 1 H), 4.67-4.64 (m, 1 H), 4.18-4.09 (m, 4 H), 2.87-2.80 (m, 1 H), 2.24-2.23 (m, 1 H), 1.99-1.93 (m, 2 H), 1.70-1.61 (m, 1 H), 1.56-1.52 (m, 1 H), 1.34-1.30 (m, 6 H), 1.02-1.00 (m, 6 H), 0.95-0.90 (m, 6 H). MS (ESI+): m/z [M+H]⁺ = 332.

### Diethyl (1-(((S)-1-(1-(3,4-dichlorophenyl)-1H-1,2,3-triazol-4-yl)-2-methylpropyl)amino)-4-methyl-1-oxopentan-2-yl)phosphonate.

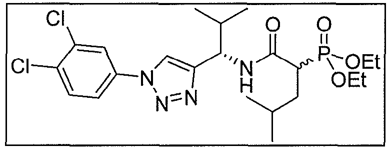

A solution of (4-methyl-1-(((S)-4-methylpent-1-yn-3-yl)amino)-1-oxopentan-2-yl)phosphonate (293 mg, 0.89 mmol) and 4-azido-1,2-dichlorobenzene (170 mg, 0.904 mmol), which was synthesized according to literature (https://doi.org/10.1016/j.ejmech.2019.06.007) in 2 mL *t*BuOH/H₂O/MeOH (2:2:1) was purged with argon. Na ascorbate (20 mol%) and CuSO₄·5 H₂O (10 mol%) were added, and the reaction mixture was stirred for 14 h at rt. Then, saturated EDTA solution was added and the mixture extracted with EtOAc (x 3), the combined organic layers were washed with saturated aqueous NH₄Cl solution and saturated aqueous NaCl solution. After drying over Na₂SO₄ and filtration, the solvent was evaporated to yield the title compound (394 mg, 0.759 mmol, 84%, mixture of diastereomers) which was used in the next step without further purification. MS (ESI+): m/z [M+H]⁺ = 520.

### (1-(((S)-1-(1-(3,4-dichlorophenyl)-1H-1,2,3-triazol-4-yl)-2-methylpropyl)amino)-4-methyl-1-oxopentan-2-yl)phosphonic acid (170).

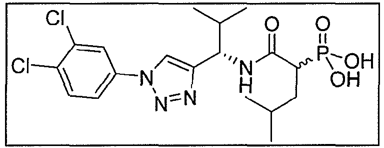

The title compound was prepared according to general procedure F. 52 mg (0.100 mmol) of compound diethyl (1-(((*S*)-1-(1-(3,4-dichlorophenyl)-1*H*-1,2,3-triazol-4-yl)-2-methylpropyl)amino)-4-methyl-1-oxopentan-2-yl)phosphonate was used and yielded the title compound after purification *via* preparative HPLC (12 mg, 0.026 mmol, 26%, Mixture of diastereomers:
Major diastereomer: ¹H NMR (500 MHz, MeOH-*d*₄) δ ppm: 8.44 (s, 1 H), 8.10-8.09 (m, 1 H), 7.83-7.80 (m, 1 H), 7.75-7.73 (m, 1H), 4.99-4.97 (m, 1 H), 3.03-2.96 (m, 1 H), 2.35-2.30 (m, 1 H), 2.13-2.06 (m, 1 H), 1.49-1.43 (m, 2 H), 1.06-0.98 (m, 6 H),

0.87-0.84 (m, 6 H). ³¹P NMR (202 MHz, MeOH-*d*₄) δ ppm: 22.3.Minor diastereomer: ¹H NMR (500 MHz, MeOH-*d*₄) δ ppm: 8.58 (s, 1 H), 8.10-8.09 (m, 1 H), 7.83-7.80 (m, 1 H), 7.73-7.70 (m, 1H), 5.08-5.07 (m, 1 H), 3.17-3.12 (m, 1 H), 2.42-2.35 (m, 1 H), 2.02-1.98 (m, 1 H), 1.61-1.51 (m, 2 H), 0.98-0.95 (m, 6 H). ³¹P NMR (202 MHz, MeOH-*d*₄,) δ ppm: 22.3. HRMS (ESI+) calculated for C₁₈H₂₆Cl₂N₄O₄P [M+H]⁺ 463.1063, found: 463.1065.

### Example 171

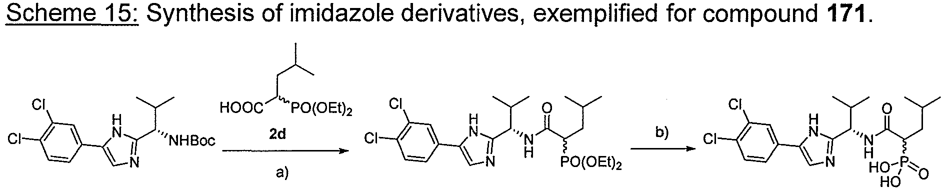

(a) i) HCl (4 M in dioxane), DCM, r.t., 18h; ii) TBTU, NMM, DMF 0 °C-rt, 22 h; (b) TMSBr, DCM, r.t., overnight, prep HPLC. 26%.

### Diethyl (1-(((S)-1-(5-(3,4-dichlorophenyl)-1H-imidazol-2-yl)-2-methylpropyl)amino)-4-methyl-1-oxopentan-2-yl)phosphonate.

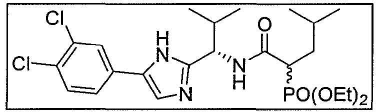

The corresponding imidazolyl amino acid derivative *tert-butyl (S)-(1-(5-(3,4-dichlorophenyl)-1H-*imidazol-2-yl)-2-methylpropyl)carbamate was synthesized as previously reported in the literature (https://doi.org/10.1016/j.ejmech.2016.08.070). *Tert-butyl* (S)-(1-(5-(3,4-dichlorophenyl)-1H-imidazol-2-yl)-2-methylpropyl)carbamate (77 mg, 0.200 mmol) was dissolved in DCM (2 mL) and HCl (0.25 mL, 1.00 mmol, 4 M in dioxane) was added. After full consumption of the starting material (LCMS) the solvent was evaporated to yield (S)-1-(5-(3,4-dichlorophenyl)-1H-imidazol-2-yl)-2-methylpropan-1-amine hydrochloride, which was used in the coupling step without further purification. The title compound was synthesized using general procedure I using aforementioned (*S*)-1-(5-(3,4-dichlorophenyl)-1*H*-imidazol-2-yl)-2-methylpropan-1-amine hydrochloride, compound **2d** (51 mg, 0.200 mmol), TBTU (70.6 mg, 0.220 mmol), NMM (53 µL, 0.500 mmol) in DMF (2 mL). Automated combiflash purification (Teledyne ISCO) yielded the title compound (22 mg, 0.042 mmol, 21%) as a mixture of diastereomers. MS (ESI+): m/z [M+H]⁺ = 519.

### Mixture of diastereomers:

Major diastereomer: ¹H NMR (500 MHz, CDCl₃) δ ppm: 7.88 (bs, 1 H), 7.54 (dd, *J* = 8.2 Hz, *J* = 1.8 Hz, 1 H), 7.39 (d, *J =* 8.4 Hz, 1 H), 7.25 (bs, 1 H), 5.26-5.23 (m, 1 H), 4.20-4.09 (m, 4 H), 3.00-2.93 (m, 1 H), 2.76-2.68 (m, 1 H), 2.18-2.11 (1 H), 1.70-1.61 (m, 1H), 1.52-1.44 (m, 1H), 1.34 (dd, *J* = 6.10 Hz, 3H), 1.30 (dd, *J* = 7.1 Hz, 3 H), 1.02 (d, *J* = 6.8 Hz, 3 H), 0.94 (dd, *J* = 7.1 Hz, 6 H), 0.90 (d, *J* = 6.7 Hz, 3 H). ³¹P NMR (202 MHz, CDCl₃) δ ppm: 26.3.

Minor diastereomer (selected signals): ¹H NMR (500 MHz, CDCl₃) δ ppm: 7.93 (d. *J* = 1.8 Hz, 1 H), 7.64 (dd, *J* = 8.4 Hz, *J* = 1.8 Hz, 1 H), 7.41 (d, *J = 8.4* Hz, 1 H), 4.08-4.04 (m, 4 H), 3.09-3.03 (m, 1 H), 2.58-2.51 (m, 1 H). ³¹P NMR (202 MHz, CDCl₃) δ ppm: 26.5.

### (1-(((S)-1-(5-(3,4-dichlorophenyl)-1H-imidazol-2-yl)-2-methylpropyl)amino)-4-methyl-1-oxopentan-2-yl)phosphonic acid (171)

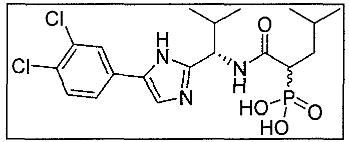

The title compound was prepared according to general procedure F. Diethyl (1-(((*S*)-1-(5-(3,4-dichlorophenyl)-1*H*-imidazol-2-yl)-2-methylpropyl)amino)-4-methyl-1-oxopentan-2-yl)phosphonate (22 mg, 0.042 mmol) and bromotrimethylsilane (28 µL, 0.212 mmol) in DCM (0.5 mL) were used. Purification *via* preperative HPLC afforded the title compound as a colorless solid_(5.2 mg, 0.011 mmol, 26%, mixture of diastereomeres). Mixture of diastereomers:
Major diastereomer: ¹H NMR (500 MHz, MeOH-*d*₄) δ ppm: 8.09 (d, *J* = 1.8 Hz, 1 H), 7.88 (s, 1 H), 7.79 (dd, *J* = 8.4 Hz, *J* = 1.7 Hz, 1 H), 7.62 (d,J = 8.64 Hz, 1 H), 5.21 (d, *J* = 5.0 Hz, ), 3.33-3.25 (m, 1 H), 2.52-2.46 (m, 1 H), 2.10-2.04 (m, 1 H), 1.62-1.53 (m, 2 H), 1.09 (d, *J = 6.9* Hz, 3 H), 1.01 (d, *J = 6.9* Hz, 3 H), 0.95 (dd, *J* = 5.7 Hz, 6 H). ¹³C NMR (126 Mhz, MeOH-*d*₄) δ ppm: 174.3, 151.1, 134.5, 132.9, 132.5, 131.1, 128.9, 126.8, 117.6, 61.7, 54.1, 47.6 36.0, 32.3, 28.8, 23.5, 22.1, 19.4, 17.5, 14.6. ³¹P NMR (202 MHz, MeOH-*d*₄) δ ppm: 19.7.
Minor diastereomer (selected signals): ¹H NMR (500 MHz, MeOH-*d*₄) δ ppm: 8.04 (d, *J* = 2.1 Hz, 1 H), 7.97 (d, *J = 1.8* Hz, 1 H), 7.89 (bs, 1 H), 7.63 (d, *J = 7.6* Hz, 1 H), 3.18-3.16 (m, 1 H), 2.42-2.38 (m, 1 H), 1.54-1.53 (m, 2 H), 1.13 (d, *J* =6.7 Hz, 3 H), 0.92 (d, *J = 6.4* Hz, 3 H), 0.90 (d, *J* = 6.4 Hz, 3 H). ¹³C NMR (126 MHz, MeOH-*d*₄) δ ppm: 134.6, 132.7, 128.8, 126.9, 54.9, 32.7, 23.6, 22.0, 19.7.

### Example 172

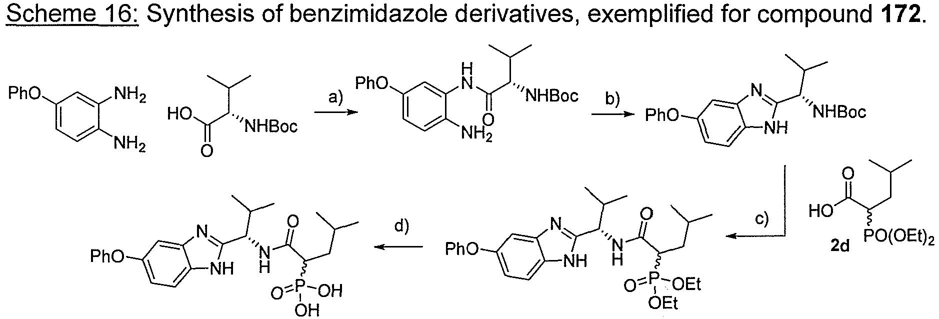

(a) TBTU, NMM, DMF, 0 °C-r.t., 2 d, quantitative; (b) HOAc/toluene (1:1), 110 °C, 3 h; (c) i) HCl (4 M in 1,4-dioxane), DCM, r.t., 18 h; ii) TBTU, NMM, DMF, 0 °C - rt, 21 h, 97% (over 3 steps); d) TMSBr, DCM, r.t., 21 h, prep HPLC, 1.2%.

### Tert-butyl (S)-(1-((2-amino-5-phenoxyphenyl)amino)-3-methyl-1-oxobutan-2-yl)carbamate.

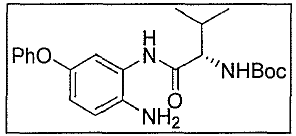

The title compound was synthesized using general procedure L. Boc-Val-OH (543 mg, 2.50 mmol) was dissolved in DMF (25 mL) and NMM (302 µL, 2.75 mmol) followed TBTU (894 mg, 2.75 mmol) were added at 0 °C. The reaction mixture was stirred at this temperature for 30 min and 4-phenoxybenzene-1,2-diamine (500 mg, 2.5 mmol) was added. After warming up to r.t. overnight, the reaction was quenched with saturated aqueous NaHCO₃ solution and extracted with EtOAc (x 3). The combined organic layers were subsequently washed with 1 M HCl, water and saturated aqueous NaCl solution, dried over Na₂SO₄, filtered and concentrated *in vacuo.* The crude product was obtained as a brown foam (1.00 g, 2.50 mmol, quantitative) and used in the next step without further purification.¹H NMR (500 MHz, CDCl₃) δ ppm: 7.62 (bs, 1 H), 7.34-7.31 (m, 2 H), 7.11-7.08 (m, 2 H), 7.02-7.00 (m, 2 H), 6.42-6.39 (m, 2 H), 5.11 (bs, 1 H), 3.99-3.97 (m, 1 H), 2.31-2.23 (m, 1 H), 1.46 (s, 9 H), 1.07 (dd, *J* = 6.71 Hz, 3 H), 1.04 (dd, *J* = 6.71 Hz, 3 H). MS (ESI+): m/z [M+H]⁺ = 400.

### Tert-butyl (S)-(2-methyl-1-(5-phenoxy-1H-benzordlimidazol-2-yl)propyl)carbamate.

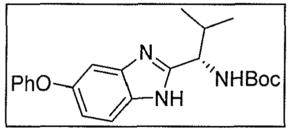

The title compound was synthesized using general procedure L. *Tert-butyl* (*S*)-(1-((2-amino-5-phenoxyphenyl)amino)-3-methyl-1-oxobutan-2-yl)carbamate (200 mg, 1.00 mmol) was dissolved in 5 mL toluene/HOAc (1:1) and heated under reflux (pre-heated oil bath) for 3 h. After cooling to r.t. saturated aqueous NaHCO₃ solution was added carefully until pH = 9. The solution was then stirred for 20 min and extracted with EtOAc (x 3), washed with saturated aqueous NaHCO₃ solution (x 4), water (x 2) and saturated aqueous NaCl solution. The organic layer was dried over Na₂SO₄, filtered and concentrated under reduced pressure to afford the title compound as an orange solid (191 mg, 0.500 mmol, quantitative), which was used in the next step without further purification. MS (ESI+): m/z [M+H]⁺ = 382.

### Diethyl (4-methyl-1-(((S)-2-methyl-1-(5-phenoxy-1H-benzo[d]imidazol-2-yl)propyl)amino)-1-oxopentan-2-yl)phosphonate.

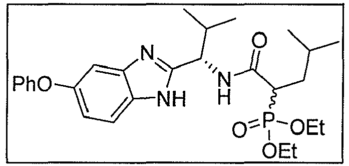

*Tert-butyl* (*S*)-(2-methyl-1-(5-phenoxy-1*H-*benzo[d]imidazol-2-yl)propyl)carbamate (159 mg, 0.417 mmol) was dissolved in DCM (4 mL) and then HCl (4 M in 1,4-dioxane, 1.04 mL, 4.17 mmol) was added. The reaction mixture was stirred for 18 h at r.t. and then concentrated under reduced pressure. In the meantime, a mixture of 2-(diethoxyphosphoryl)-4-methylpentanoic acid **2d** (116 mg, 0.459 mmol) and TBTU (181 mg, 0.505 mmol) in DMF (2.5 mL) was cooled to 0 °C, and NMM (121 µL, 1.15 mmol) was added. The reaction mixture was stirred for 30 minutes and then the Boc-deprotected benzimidazole amino acid derivative, dissolved in DMF (2.5 mL) was added dropwise at 0 °C. After stirring for 21 h EtOAc and 1 M HCl was added, and the aqueous layer was extracted with EtOAc (x 2). The combined organic layers were washed with water and saturated aqueous NaCl solution. After drying over Na₂SO₄ and filtration, the solvent was removed under reduced pressure, and the title compound was obtained as a brownish resin (208 mg, 0.405 mmol, 97%). The title compound was used in the next step without any further purification. MS (ESI+): m/z [M+H]⁺ = 516.

### (4-Methyl-1-(((S)-2-methyl-1-(5-phenoxy-1H-benzo[d]imidazol-2-yl)propyl)amino)-1-oxopentan-2-yl)phosphonic acid (172).

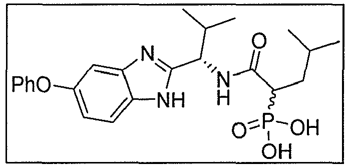

The title compound was prepared according to general procedure F. 166 mg (0.322 mmol) of diethyl (4-methyl-1-(((*S*)-2-methyl-1-(5-phenoxy-1*H-*benzo[d]imidazol-2-yl)propyl)amino)-1-oxopentan-2-yl)phosphonate were used and yielded the title compound after purification *via* preparative HPLC (1.74 mg, 0.004 mmol, 1.2%).

### Mixture of diastereomers:

Major diastereomer: ¹H NMR (500 MHz, DMSO-*d*₆,) δ ppm: 8.14-8.12 (m, 1 H), 7.53-7.51 (m, 1 H), 7.37-7.33 (m, 2 H), 7.13-7.05 (m, 2 H), 6.97-6.95 (m, 2 H), 4.94-4.91 (m, 1 H), 3.28-3.22 (m, 1 H), 2.32-2.26 (m, 1 H), 1.87-1.81 (m, 1 H) 1.50-1.37 (m, 2 H), 0.98-0.96 (m, 3 H), 0.93-0.92 (m, 3 H), 0.87-0.86 (m, 6 H). ¹³C NMR (126 MHz, DMSO-*d*₆) δ ppm: 169.8, 158.6, 156.2, 151.9, 130.5, 123.1, 118.1, 60.2, 53.7, 36.5, 32.4, 27.3 (d, *J* = 14.7 Hz), 23.5, 22.0, 19.8, 18.8. ³¹P NMR (202 MHz, DMSO-*d*₆) δ ppm: 20.7.Minor diastereomer (selected signals): ¹H NMR (500 MHz, DMSO-*d*₆) δ ppm: 8.45-8.43 (m, 1 H), 7.49-7.48 (m, 1 H), 6.92-6.89 (m, 2 H), 5.13-5.11 (m, 1 H), 1.96-1.92 (m, 1 H), 0.78-0.76 (m, 6 H). ¹³C NMR (126 MHz, DMSO-*d*₆) δ ppm: 170.71, 158.5, 157.4, 152.1, 123.4, 118.2, 115.1, 53.2, 34.9, 30.6, 26.8 (d, *J* = 14.7 Hz), 23.4, 21.9, 19.7, 17.4. ³¹P NMR (202 MHz, DMSO-*d*₆) δ ppm: 20.9.

HRMS (ESI+) calculated for [M+H]⁺: 460.1996, found: 460.1982.

### Example 173

### Ethyl 4-methyl-2-(p-tolylcarbamoyl)pentanoate.

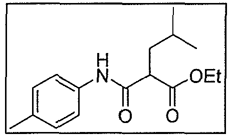

Ethyl 4-methyl-2-(*p*-tolylcarbamoyl)pentanoate was synthesized according to general procedure M, using diethyl 2-alkylmalonate (645 mg, 2.98 mmol), EtOH/H₂O (30 mL, 4:1) and NaOH (143 mg, 3.58 mmol). The reaction was stirred at rt overnight. The obtained mono-acid (505 mg, 2.68 mmol) and EDC·HCl (515 mg, 2.68 mmol) were added to a solution of *p*-toluidine (240 mg, 2.23 mmol) in DCM (20 mL). The resultant mixture was stirred at rt overnight. After the workup, the obtained crude product was purified using column chromatography (Hex/EtOAc=8/2). The product was obtained as orange crystals (441 mg, 71%). ¹H NMR (500 MHz, CDCl₃) δ ppm: 8.45 (br s, 1H), 7.42 (d, *J =* 8.1 Hz, 2H), 7.13 (d, *J =* 8.1 Hz, 2H), 4.31-4.17 (m, 2H), 3.43 (t, *J =* 7.7 Hz, 1H), 2.32 (s, 3H), 1.94-1.80 (m, 2H), 1.69-1.61 (m, 1H), 1.35-1.28 (m, 3H), 0.96 (d, *J* = 6.6 Hz, 6H). ¹³C NMR (126 MHz, CDCl₃) δ ppm: 173.2, 166.4, 135.0, 134.0, 129.4, 119.8, 61.7, 52.4, 40.8, 26.4, 22.5, 22.0, 20.9, 14.1. MS (ESI+): m/z [M+H]⁺ = 278

### N¹-hydroxy-2-isobutyl-N³-(p-tolyl)malonamide (173).

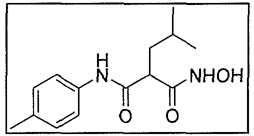

*N*¹-hydroxy-2-isobutyl-*N*³-(p-tolyl)malonamide was synthesized according to general procedure N, using ethyl 4-methyl-2-(p-tolylcarbamoyl)pentanoate (100 mg, 0.36 mmol), MeOH (2 mL), NH₂OH 50 wt % in H₂O (2 mL) and KCN (4.7 mg, 0.07 mmol). The mixture was stirred at rt overnight. Solvents were concentrated in vacuo and the resultant oil was purified by preparative HPLC (CH₃CN (HCOOH 0.05%)-H₂O (HCOOH 0.05%): 1.0:9.0 to 10.0:0.0). The product was obtained as white solid (49 mg, 52%). ¹H NMR (500 MHz, DMSO-*d*₆) δ ppm: 10.54 (s, 1H), 9.66 (s, 1H), 9.00 (s, 1H), 7.45 (d, *J* = 8.4 Hz, 2H), 7.10 (d, *J =* 8.4 Hz, 2H), 3.18 (t, *J =* 7.6 Hz, 1H), 2.24 (s, 3H), 1.67 (t, *J* = 7.2 Hz, 2H), 1.47 (dquin, J = 13.4, 6.7, 6.7, 6.7, 6.7 Hz, 1H), 0.87 (br d, *J* = 6.6 Hz, 3H), 0.87 (br d, *J =* 6.6 Hz, 3H). ¹³C NMR (126 MHz, DMSO-*d*₆) δ ppm: 167.6, 166.5, 136.3, 132.4, 129.2, 119.4, 49.9, 38.1, 25.8, 22.5, 22.3, 20.5. HRMS (ESI⁺) calculated for C₁₄H₂₁N₂O₃ [M+H]⁺ 265.1547, found 265.1545.

### Example 174 and Example 177

### 4-Methyl-N-(p-tolyl)-2-(1H-1,2,3-triazol-1-yl)pentanamide (174) and 4-methyl-N-(p-tolyl)-2-(2H-1,2,3-triazol-2-yl)pentanamide (177).

4-Methyl-*N*-(p-tolyl)-2-(1H-1,2,3-triazol-1-yl)pentanamide **(174)** and 4-methyl-*N*-(*p-*tolyl)-2-(2*H*-1,2,3-triazol-2-yl)pentanamide **(177)** were synthesized according to general procedure O, using 2-bromo-4-methyl-*N*-(*p*-tolyl)pentanamide (70 mg, 0.25 mmol) (synthesized according to general procedure B-1), acetone (7 mL), 1*H*-1,2,3-triazole (18.7 mg, 0.27 mmol) and K₂CO₃ (37.4 mg, 0.27 mmol). The crude product was purified by preparative HPLC (CH₃CN (HCOOH 0.05%)-H₂O (HCOOH 0.05%): 1.0:9.0 to 10.0:0.0), giving products **174** (20.3 mg, 30%) and **177** (30 mg, 45%) as white solids.

### 4-Methyl-N-(p-tolyl)-2-(1H-1,2,3-triazol-1-yl)pentanamide (174).

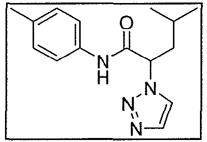

¹H NMR (500 MHz, DMSO-*d*₆) δ ppm: 10.53 (s, 1H), 8.30 (d, *J =* 0.8 Hz, 1H), 7.77 (d, *J* = 0.6 Hz, 1H), 7.47 (d, *J* = 8.4 Hz, 2H), 7.16 (d, *J* = 8.2 Hz, 2H), 5.61 (dd, *J* = 9.8, 6.1 Hz, 1H), 2.25 (s, 3H), 2.16-2.06 (m, 1H), 2.01-1.93 (m, 1H), 1.31-1.20 (m, 1H), 0.93 (d, *J* = 6.8 Hz, 3H), 0.90 (d, *J* = 6.6 Hz, 3H). ¹³C NMR (126 MHz, DMSO-*d*₆) δ ppm: 166.6, 135.7, 133.3, 133.1, 129.3, 123.9, 119.5, 61.6, 40.4, 24.5, 22.4, 21.5, 20.5. HRMS (ESI⁺) calculated for C₁₅H₂₁N₄O [M+H]⁺ 273.1710, found 273.1708.

### 4-Methyl-N-(p-tolyl)-2-(2H-1,2,3-triazol-2-yl)pentanamide (177).

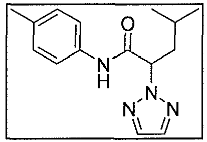

¹H NMR (500 MHz, DMSO-*d*₆) δ ppm: 10.35 (s, 1H), 7.83 (s, 2H), 7.45 (d, *J* = 8.4 Hz, 2H), 7.11 (d, *J* = 8.4 Hz, 2H), 5.46 (dd, *J* = 9.3, 6.0 Hz, 1H), 2.34-2.28 (m, 1H), 2.24 (s, 3H), 1.99 (ddd, *J* = 13.8, 7.8, 6.2 Hz, 1H), 1.47-1.36 (m, 1H), 0.91 (t, *J* = 6.2 Hz, 6H). ¹³C NMR (126 MHz, DMSO-*d*₆) δ ppm: 166.2, 135.9, 134.5, 132.9, 129.2, 119.4, 65.8, 24.5, 22.5, 21.7, 20.5. HRMS (ESI⁺) calculated for C₁₅H₂₁N₄O [M+H]⁺ 273.1710, found 273.1708.

### III. Biological evaluation

### Activity against LasB:

The activities of the compounds of the present invention were determined according to the procedures described in Kany, A. M.; Sikandar, A.; Haupenthal, J.; Yahiaoui, S.; Maurer, C. K.; Proschak, E.; Köhnke, J.; Hartmann, R. W. ACS Infect. Dis. 2018, 4, 988-997.

### α-Benzylated derivatives:

**Table 2: Activities of α-benzylmercaptoacetamides against LasB.**

| 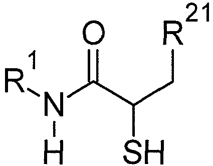 | | | |
|---|---|---|---|
| **Example** | **R¹** | **R²¹** | **IC₅₀ [µM]** |
| **1** | Ph | Ph | 1.2 ± 0.1 |
| **4** | 3,4-di-Cl-Ph | Ph | 2.7 ± 0.3 |
| **5** | 4-OH-Ph | Ph | 0.59 ± 0.04 |
| **6** | 2-CH₃-Ph | Ph | 2.4 ± 1.0 |
| **7** | 3-CH₃-Ph | Ph | 0.98 ± 0.43 |
| **8** | 4-CH₃-Ph | Ph | 0.48 ± 0.04 |
| **9** | 4-NO₂-Ph | Ph | 0.97 ± 0.10 |
| **10** | 4-OCH₃-Ph | Ph | 0.73 ± 0.03 |
| **11** | Ph | 4-OH-Ph | 7.3 ± 0.5 |
| **12** | Ph | 3-NO₂-4-OH-Ph | 2.5 ± 0.1 |
| **13** | Ph | 4-CH₃-Ph | 2.8 ± 0.3 |

### Activity of enantiomers:

To elucidate whether the configuration of the stereocenter has an influence on activity, the enantiomers (E1 and E2, labeled according to their elution order from the chiral column) of the compounds of examples **1** and **4** were separated using a chiral column with a preparative HPLC and independently examined. Although both enantiomers were active, a difference in activity between the two configurations was observed (Table 3). For both compounds, the E2 enantiomer was more active.

**Table 3. Activity of the racemic mixtures and pure enantiomers for examples 1 and 4.**

| **Example** | | **IC₅₀ [µM]** |
|---|---|---|
| **1** | E1 | 4.8 ± 0.7 |
| | E2 | 1.0 ± 0.1 |
| | Rac | 1.2 ± 0.1 |
| **4** | E1 | 5.2 ± 0.6 |
| | E2 | 2.0 ± 0.4 |
| | Rac | 2.7 ± 0.3 |

In order to ensure that no racemization occurs during the assays, the configurational stability in methanol and aqueous buffer (50 m_{M} Tris, pH 7.2, 2.5 mm CaCl₂) was examined. The CD spectra were unchanged over one hour indicating that racemization does not occur during this period.

### Selectivity:

The inhibition of zinc-containing human enzymes is described frequently for LasB inhibitors and poses a serious difficulty in the development of selective compounds. Particularly, the inhibition of matrix metalloproteases (MMPs) should be avoided. To further investigate this issue, three derivatives (the compounds of examples **1, 5** and **8)** have been tested for their selectivity against several human off-targets, including six MMPs, ADAM17 (TACE), HDAC-3 and HDAC-8 (Table 4). The selectivity of the compounds is particularly high for MMPs and HDACs, whereas the inhibition of ADAM17 was considerably stronger.

**Table 4. Selectivity of examples 1, 5 and 8 against off-targets. (n.i. = <10% inhibition).**

| | **conc. [µM]** | **Inhibition [%]** | | |
|---|---|---|---|---|
| | | **1** | **5** | **8** |
| MMP-1 | 100 | n.i. | n.i. | n.i. |
| MMP-2 | 100 | n.i. | n.i. | n.i. |
| MMP-3 | 100 | n.i. | n.i. | n.i. |
| MMP-7 | 100 | n.i. | n.i. | n.i. |
| MMP-8 | 100 | 34 ± 11 | 19 ± 4 | n.i. |
| MMP-14 | 100 | n.i. | n.i. | n.i. |

| | | **IC₅₀ [µM]** | | |
|---|---|---|---|---|
| ADAM17 | | 2.2 ± 0.1 | 2.3 ± 1.4 | 4.8 ± 1.2 |
| HDAC-3 | | >100 | >250 | >100 |
| HDAC-8 | | >100 | >250 | >100 |

### Cytotoxicity:

The compounds of examples **1** and **5** were not toxic against the cell lines HepG2, HEK293 and A549 (Table 5). Additionally, the inhibitory effect against *P. aeruginosa* PA14 was evaluated to exclude an antibacterial effect of the compounds of the present invention. This is important as it was the aim to target virulence and not viability of the bacteria. The results show that for both compounds MIC values on PA14 as well as cytotoxicities (IC₅₀ values) in the cell lines were greater than 100 µM and therefore unproblematic.

**Table 5. Cytotoxicity data and PA14 inhibition by examples 1 and 5.**

| | **1 [µM]** | **5 [µM]** |
|---|---|---|
| HepG2 IC₅₀ | > 100 | > 100 µM |
| HEK293 IC₅₀ | > 100 | > 100 |
| A549 IC₅₀ | > 100 | > 100 |
| MIC PA14 | > 100 | > 100 |

### α-Alkylated derivatives:

Compounds bearing alkyl substituents in C_{α}-position are highly favorable for activity, leading to submicromolar IC₅₀ values (Tables 6 and 7). Among these, the compound of example **2** with a 4-Me substituent on the aromatic core and *iso*-butyl chain proved to be one of the most promising ones, and was therefore further explored regarding selectivity and cytotoxicity (Table 8).

**Table 6. α-alkylated derivatives and their corresponding activities towards LasB.**

| 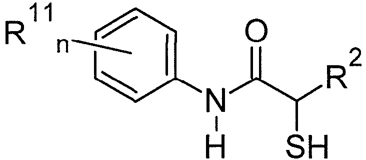 | | |
|---|---|---|
| **R¹¹** (n = 1 or 2) | **R²** | **IC₅₀ [µM]** |
| 3,4-diCl¹ | H | 6.6 ± 0.3 |
| 3,4-diCl **(15)** | methyl | 4.5 ± 0.7 |
| 4-MeO **(16)** | methyl | 45 ± 1 |
| 4-Ac **(17)** | methyl | 89 ± 9 |
| 3,4-diCl **(18)** | ethyl | 2.4 ± 0.4 |
| 4-MeO **(19)** | ethyl | 4.2 ± 0.6 |
| 4-Ac **(20)** | ethyl | 10 ± 2 |
| 3,4-diCl **(21)** | *i*-propyl | 2.5 ± 0.3 |
| 4-MeO **(22)** | *i*-propyl | 16 ± 1 |
| 4-Ac **(23)** | *i*-propyl | 22 ± 0 |
| H **(24)** | *n*-propyl | 4.8 ± 0.4 |
| 4-Me **(25)** | *n*-propyl | 2.0 ± 0.2 |
| 3,4-diCl **(26)** | *n*₋propyl | 4.0 ± 0.6 |
| 4-MeO **(27)** | *n*-propyl | 2.4 ± 0.6 |
| 4-Ac **(28)** | *n*-propyl | 2.6 ± 0.5 |
| 3,4-diCl **(29)** | *n*-butyl | 6.8 ± 1.1 |
| 4-OMe **(30)** | *n*-butyl | 2.8 ± 0.3 |
| 3,4-diCl **(43)** | *sec*-butyl | 7.5 ± 0.2 |
| 4-MeO **(44)** | *sec-*butyl | 17 ± 2 |
| 3,4-diCl **(45)** | cyclopropylmethyl | 6.3 ± 1.2 |
| 4-MeO **(46)** | cyclopropylmethyl | 3.9 ± 0.6 |
| 3,4-diCl **(47)** | cyclohexylmethyl | 12 ± 3 |
| 4-MeO **(48)** | cyclohexylmethyl | 2.6 ± 1.2 |
| 3,4-diCl **(49)** | -CH₂OCH₃ | 2.4 ± 0.6 |
| 4-MeO **(50)** | -CH₂OCH₃ | 17 ± 1 |

| | | |
|---|---|---|
| ¹ Compound disclosed in Kany *et al.* | | |

**Table 7. Compounds bearing an iso-butyl group in α-position and their corresponding activities against LasB.**

| 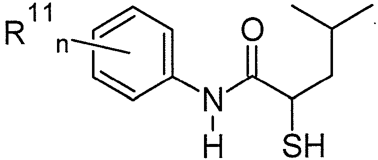 | |
|---|---|
| **R11** (n = 1 or 2) | **IC₅₀ [µM]** |
| 3,4-diCl **(31)** | 2.6 ± 0.2 |
| 2-OMe **(32)** | 0.70 ± 0.04 |
| 3-OMe **(33)** | 0.56 ± 0.03 |
| 4-OMe **(34)** | 0.36 ± 0.11 |
| 3,4-diOMe **(36)** | 0.73 ± 0.10 |
| 4-Me **(2)** | 0.40 ± 0.13 |
| 4-Cl **(38)** | 0.84 ± 0.24 |
| 4-Ac **(39)** | 0.69 ± 0.34 |
| 2-OH **(40)** | 1.4 ± 0.2 |

**Table 8. Selectivity and cytotoxicity data for the compound of example 2. n.i. = inhibition < 10%.**

| | | | |
|---|---|---|---|
| | **% inhibition at 100 µM** | **MMP-1** | n.i. |
| | | **MMP-2** | n.i. |
| | | **MMP-3** | n.i. |
| **Selectivity** | | **MMP-7** | n.i. |
| | | **MMP-8** | n.i. |
| | | **MMP-14** | n.i. |
| | **IC₅₀ [µM]** | **HDAC-3** | >100 |
| | | **HDAC-8** | >100 |
| | | **TACE** | 4.5 ± 1.8 |
| **Cytotoxicity IC₅₀ [µM]** | | **HepG2** | >100 |
| | | **HEK293** | >50 |
| | | **A549** | >100 |

As the compound of example **2** has shown an impressive activity in the *in vitro* LasB inhibition assay, high selectivity over a broad spectrum of human enzymes and no signs of cytotoxicity *in vitro,* it has been subjected to a more advanced safety screening (Table 9). The IC₅₀ value regarding the inhibition of the hERG potassium channel was determined to be > 10 µM. Furthermore, it was of particular importance to determine the effect of the compound of example **2** on five human CYP450 isoforms, demonstrating weak or no inhibition. In addition, the compound of example **2** was analyzed using the mini-Ames reverse mutation assay, where no genotoxicity was observed up to 125 µg/mL.

**Table 9. Advanced safety profile of the compound of example 2: hERG/CYP inhibition and mini-Ames test.**

| | **hERG** | **CYP1A** | **CYP2C9** | **CYP3A4** | **CYP2C19** | **CYP2D6** | **Mini-Ames** |
|---|---|---|---|---|---|---|---|
| IC₅₀ [µm] | >10 | >25 | >25 | 15 | 1.0 | 22.3 | No genotoxicity |

Moreover, the compound of example **2** was subjected to pharmacokinetic (PK) studies in mice (Table 10). Injected intravenously (i.v.) at a dose of 10 mg/kg, it is detectable in blood for 2 h. Preliminary results indicate high clearance and low overall exposure, but the volume of distribution would account for good tissue penetration.

**Table 10. PK parameters for example 2.**

| | |
|---|---|
| Cₘₐₓ [ng/mL] | 200 |
| Tₘₐₓ [min] | 15* |
| T_{1/2} [min] | 50 |
| CL/F [mL/min/kg] | 505 ± 119 |
| AUC₀₋ₜ [ng/mL*h] | 241 ± 22 |
| V/F [L/kg] | 45.5 ± 2.8 |

| | |
|---|---|
| * First measuring point | |

### α-Carboxymethyl derivatives:

The compound of Example **54** (Table 1) showed the following activity against LasB: IC₅₀ = 3.9 ± 0.4 µM.

### Heterocyclic derivatives:

**Table 11. Heterocyclic derivatives and their corresponding activities against LasB.**

| **Example** | **Structure** | **IC₅₀ [µM]** |
|---|---|---|
| **55** | 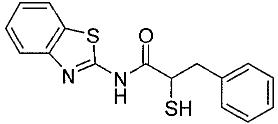 | 0.75 ± 0.07 |
| **56** | 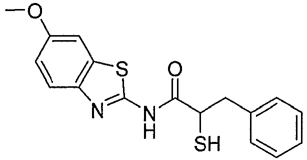 | 0.95 ± 0.08 |
| **57** | 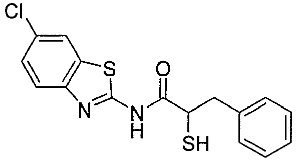 | 2.4 ± 0.2 |
| **58** | 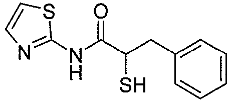 | 1.6 ± 0.1 |
| **59** | 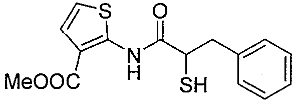 | 8 ± 1 |
| **60** | 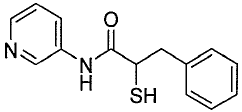 | 1.2 ± 0.1 |
| **61** | 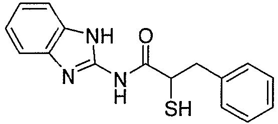 | 7.7 ± 1.4 |
| **62** | 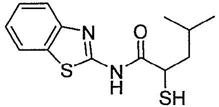 | 0.65 ± 0.14 |

### Phosphonic acid derivatives:

**Table 12. Phosphonic acid derivatives and their corresponding activities against LasB. Examples 63 to 89 were prepared according to procedures described above.**

| **Example** | **Structure** | **IC₅₀ [nM]** |
|---|---|---|
| **63** | 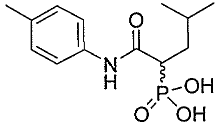 | 51 ± 7 |
| **64** | 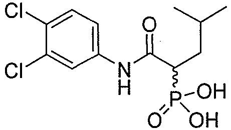 | 26 ± 4 |
| **65** | 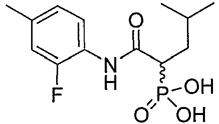 | 116 ± 16 |
| **66** | 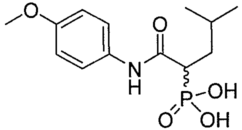 | 52 ± 10 |
| **67** | 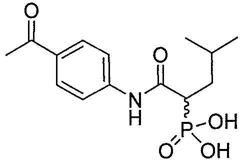 | 40 ± 12 |
| **68** | 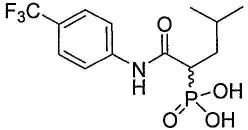 | 26 ± 8 |
| **69** | 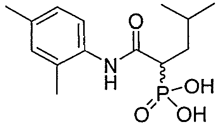 | 84 ± 3 |
| **70** | 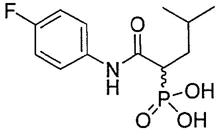 | 93 ± 22 |
| **71** | 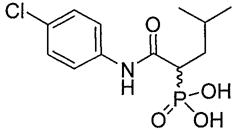 | 48 ± 4 |
| **72** | 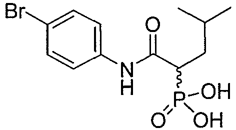 | 49 ± 2 |
| **73** | 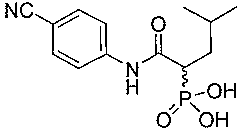 | 25 ± 1 |
| **74** | 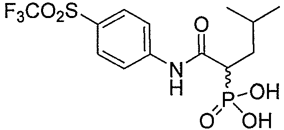 | 28 ± 6 |
| **75** | 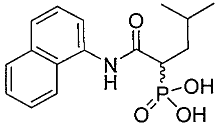 | 69 ± 8 |
| **76** | 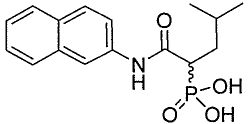 | 64 ± 10 |
| **77** | 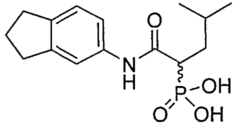 | 44 ± 4 |
| **78** | 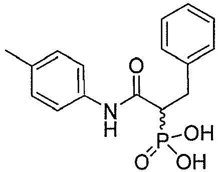 | 137 ± 33 |
| **79** | 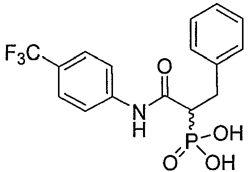 | 102 ± 1 |
| **80** | 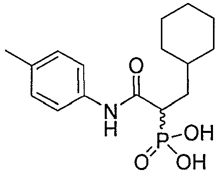 | 2030 ± 110 |
| **81** | 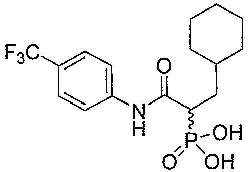 | 1870 ± 30 |
| **82** | 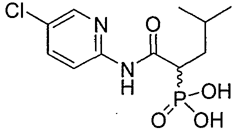 | 501 ± 39 |
| **83** | 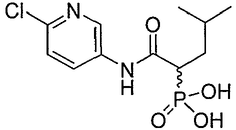 | 1130 ± 20 |
| **84** | 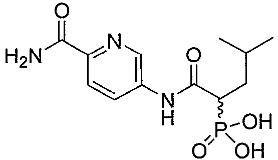 | 223 ± 16 |
| **85** | 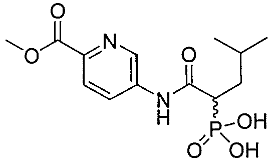 | 194 ± 11 |
| **86** | 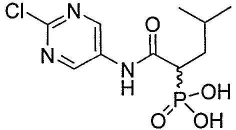 | 498 ± 25 |
| **87** | 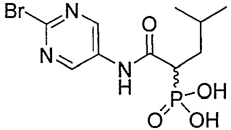 | 384 ± 2 |
| **88** | 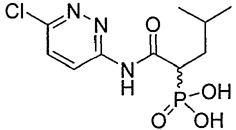 | 18800 ± 740 |
| **89** | 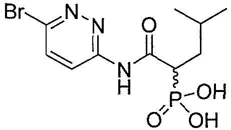 | 7910 ± 390 |

**Table 13. Further phosphonic acid derivatives and their corresponding activities against LasB. Examples 90 to 151 were prepared according to procedures described above.**

| **Example** | **Structure** | **IC₅₀ [nM]** |
|---|---|---|
| **90** | 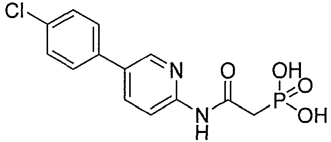 | 31% inhibition @ 50 µM |
| **91** | 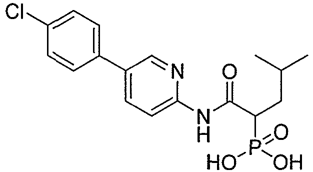 | 191 ± 5 |
| **92** | 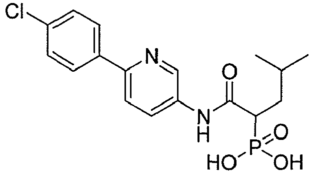 | 9.5 ± 0.4 |
| **93** | 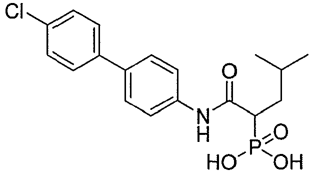 | 8.5 ± 0.4 |
| **94** | 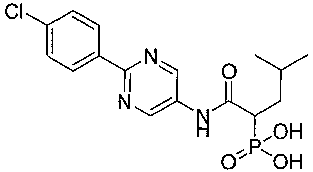 | 29 ± 2 |
| **95** | 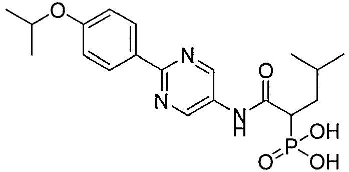 | 15 ± 1 |
| **96** | 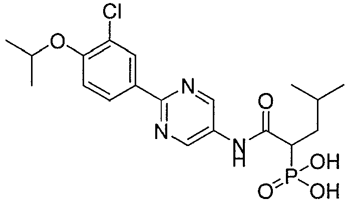 | 22 ± 1 |
| **97** | 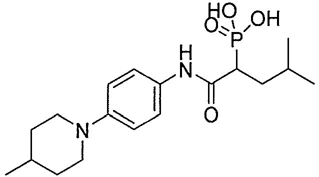 | 38 ± 1 |
| **98** | 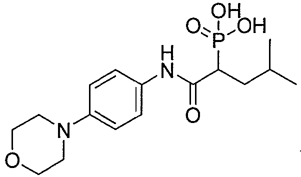 | 49 ± 2 |
| **99** | 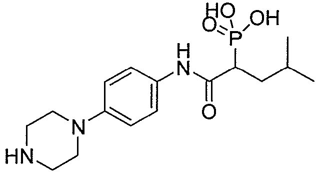 | 174 ± 10 |
| **100** | 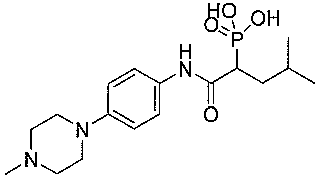 | 110 ± 8 |
| **101** | 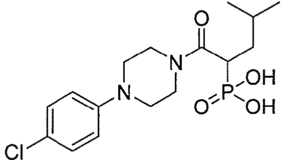 | 1450 ± 30 |
| **102** | 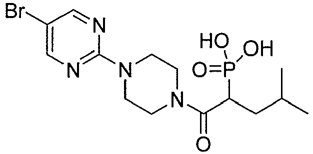 | 35840 ± 1750 |
| **103** | 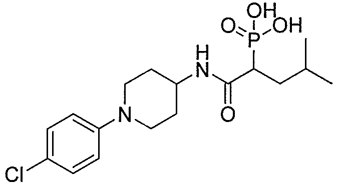 | 1910 ± 110 |
| **104** | 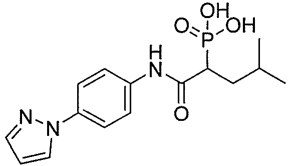 | 49 ± 2 |
| **105** | 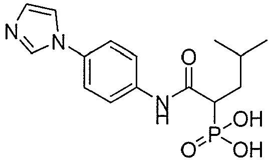 | 37 ± 2 |
| **106** | 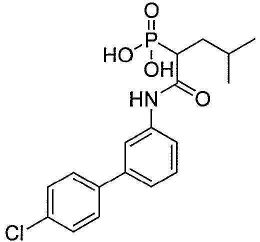 | 21 ± 1 |
| **107** | 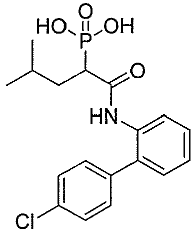 | 2723 ± 166 |
| **108** | 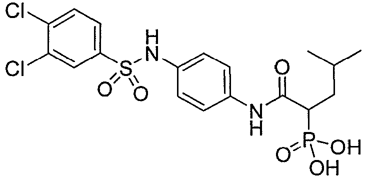 | 13 ± 0 |
| **109** | 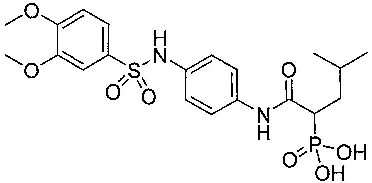 | 52 ± 2 |
| **110** | 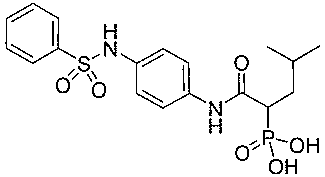 | 31 ± 1 |
| **111** | 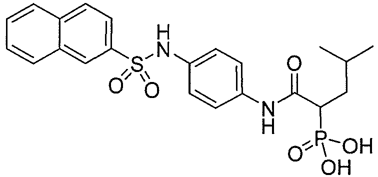 | 38 ± 1 |
| **112** | 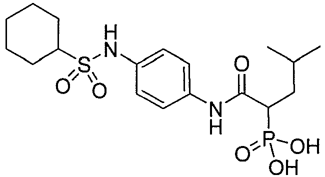 | 31 ± 1 |
| **113** | 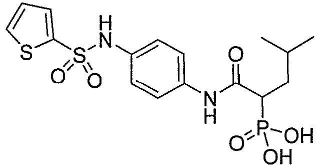 | 26 ± 2 |
| **114** | 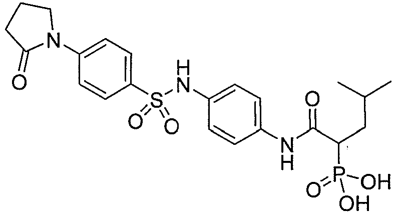 | 66 ± 3 |
| **115** | 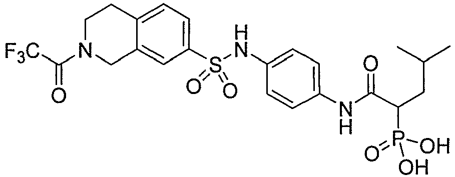 | 57 ± 3 |
| **116** | 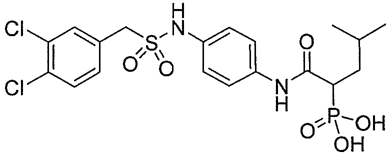 | 21 ± 1 |
| **117** | 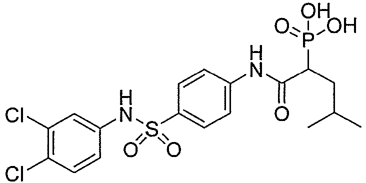 | 16 ± 1 |
| **118** | 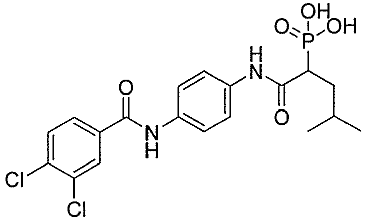 | 25 ± 1 |
| **119** | 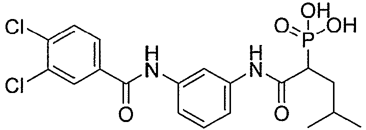 | 265 ± 11 |
| **120** | 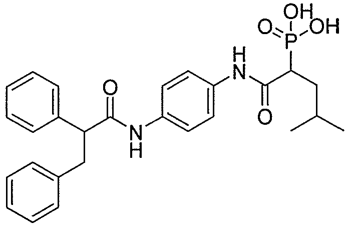 | 30 ± 1 |
| **121** | 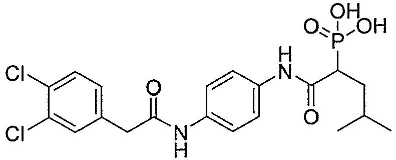 | 24 ± 1 |
| **122** | 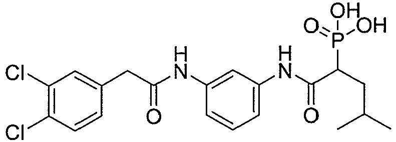 | 107 ± 4 |
| **123** | 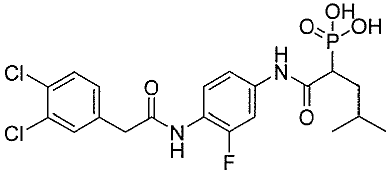 | 38 ± 1 |
| **124** | 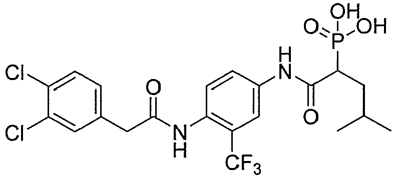 | 112 ± 6 |
| **125** | 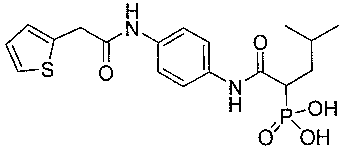 | 64 ± 3 |
| **126** | 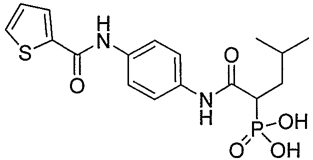 | 40 ± 2 |
| **127** | 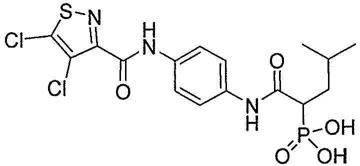 | 46 ± 4 |
| **128** | 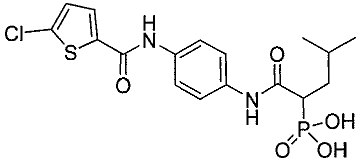 | 50 ± 1 |
| **129** | 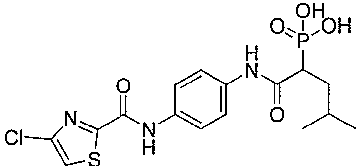 | 49 ± 2 |
| **130** | 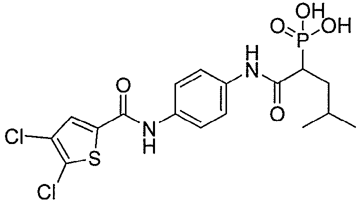 | 40 ± 2 |
| **131** | 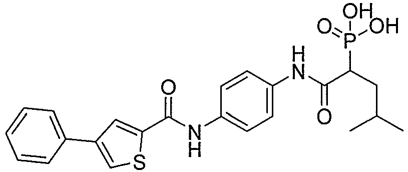 | 30 ± 2 |
| **132** | 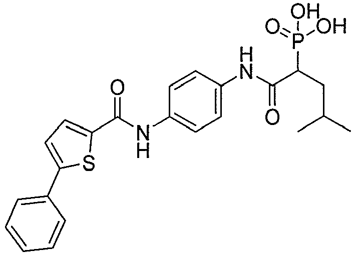 | 23 ± 1 |
| **133** | 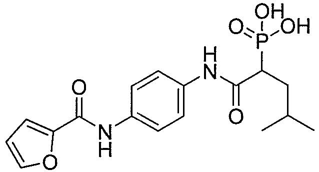 | 31 ± 2 |
| **134** | 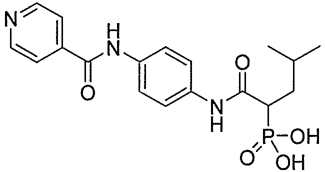 | 68 ± 4 |
| **135** | 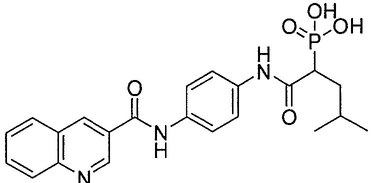 | 29 ± 2 |
| **136** | 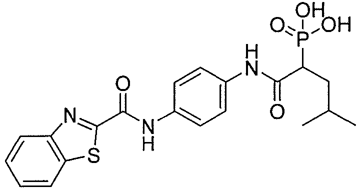 | 29 ± 1 |
| **137** | 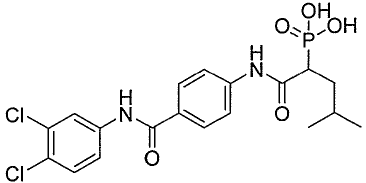 | 41 ± 3 |
| **138** | 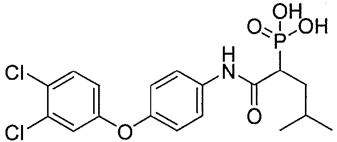 | 17 ± 1 |
| **139** | 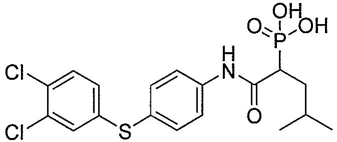 | 16 ± 1 |
| **140** | 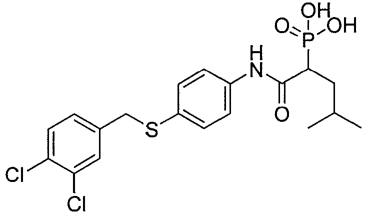 | 21 ± 1 |
| **141** | 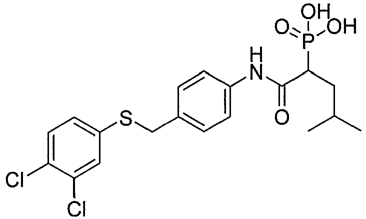 | 16 ± 1 |
| **142** | 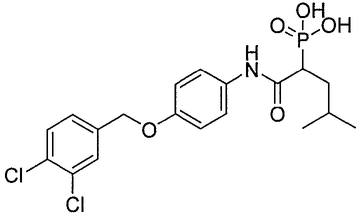 | 13 ± 0 |
| **143** | 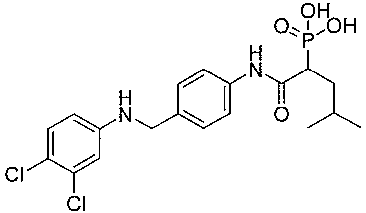 | 16 ± 1 |
| **144** | 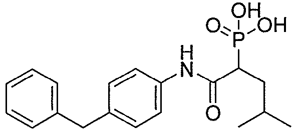 | 24 ± 1 |
| **145** | 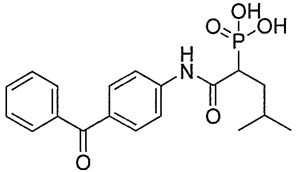 | 46 ± 1 |
| **146** | 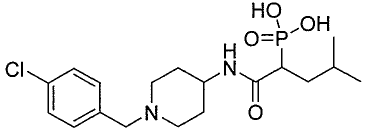 | 2270 ± 70 |
| **147** | 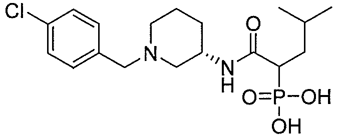 | 5180 ± 530 |
| **148** | 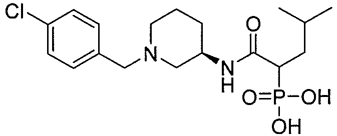 | 8360 ± 260 |
| **149** | 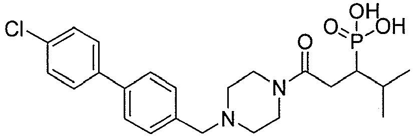 | 7280 ± 960 |
| **150** | 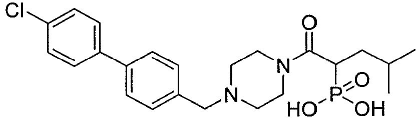 | 8660 ± 1110 |
| **151** | 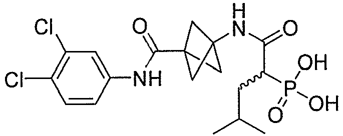 | 347 ± 12 |

**Table 14: α-Phosphonate dipeptides and their corresponding activities against LasB. Examples 152 to 169 were prepared according to procedures described above.**

| **Compound** | **Structure** | **IC₅₀ [nM]** |
|---|---|---|
| **152** | 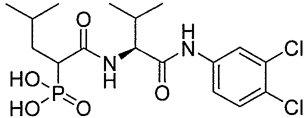 | 5.1 ± 0.2 |
| **153** | 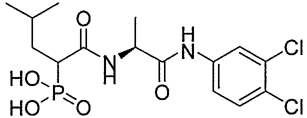 | 62 ± 2 |
| **154** | 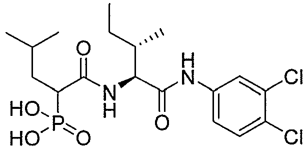 | 13 ± 1 |
| **155** | 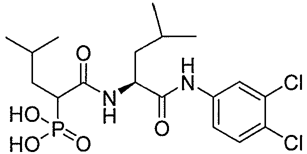 | 60 ± 2 |
| **156** | 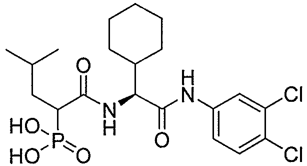 | 12 ± 0 |
| **157** | 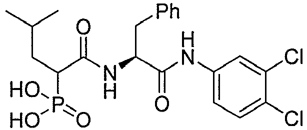 | 427 ± 48 |
| **158** | 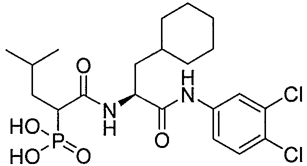 | 242 ± 21 |
| **159** | 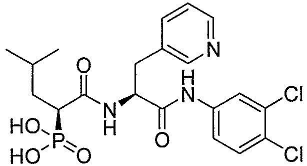 | 1500 ± 60 |
| **160** | 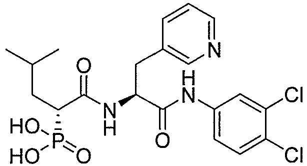 | 573 ± 16 |
| **161** | 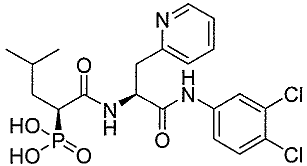 | 1360 ± 50 |
| **162** | 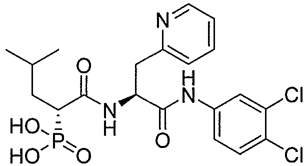 | 416 ± 23 |
| **163** | 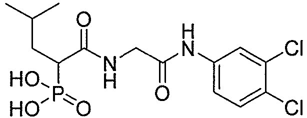 | 1756 ± 3 |
| **164** | 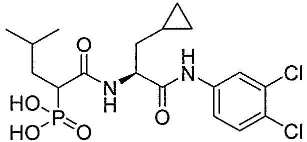 | 27 ± 1 |
| **165** | 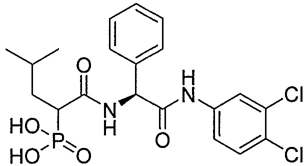 | 100 ± 3 |
| **166** | 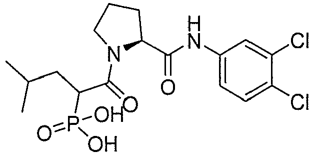 | 2539 ± 69 |
| **167** | 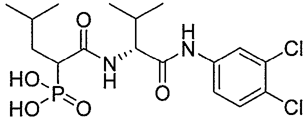 | 11 ± 0 |
| **168** | 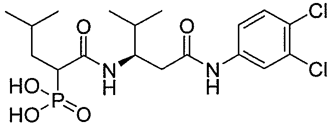 | 1756 ± 30 |
| **169** | 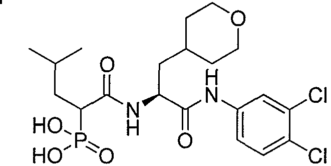 | 378 ± 8 |

All phosphonates are showing excellent selectivity and cytotoxicity profile, as well as no inhibition of PA14 bacterial growth (Tables 15, 17 and 18).

**Table 15. Selectivity, cytotoxicity and PA14 inhibition for the compounds of examples 63, 92, 108 and 152. n.i. = inhibition < 10%; n.d.= not determined**

| | | | Example **63** | Example **92** | Example **108** | Example **152** |
|---|---|---|---|---|---|---|
| | **% inhibition at 100 µM** | **MMP-1** | n.i. | n.i. | 12 ± 1 | 13±1 |
| | | **MMP-2** | n.i. | 18 ± 0 | n.i. | 7 ± 9 |
| | | **MMP-3** | n.i. | n.i. | n.i. | n.i. |
| | | **MMP-7** | n.i. | n.d. | n.d. | n.d. |
| **Selectivity** | | **MMP-8** | 10 ± 0 | n.d. | n.d. | n.d. |
| | | **MMP-14** | 12 ± 7 | n.d. | n.d. | n.d. |
| | **IC₅₀ (µM)** | **HDAC-3** | >100 | n.d. | n.d. | n.d. |
| | | **HDAC-8** | >100 | n.d. | n.d. | n.d. |
| | | **TACE** | >100 | >100 | >100 | >100 |
| | | **COX-1** | >100 | >100 | >100 | >100 |
| **Cytotoxicity IC₅₀ (µM)** | | **HepG2** | >100 | >100 | >100 | >100 |
| | | **HEK293** | >100 | >100 | >100 | >100 |
| | | **A549** | >100 | >100 | >100 | >100 |
| **MIC PA14 (µM)** | | | >100 | >100 | >100 | >100 |

**Table 16: Triazoles, imidazoles and benzimidazoles as examples for heteropentacycles and benzannulated heteropentacycles. Compounds 170 and 172 were prepared according to procedures described above.**

| **Example** | **Structure** | **IC₅₀ [µM]** |
|---|---|---|
| **170** | 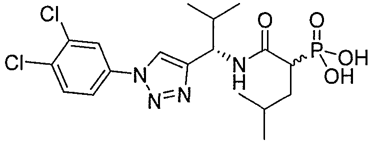 | 60 ± 3 |
| **171** | 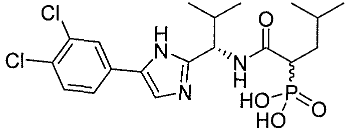 | 6 ± 0.2 |
| **172** | 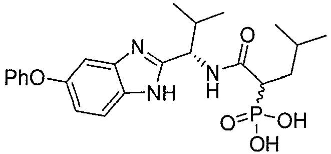 | 12±1 |

**Table 17. Selectivity of examples 170 and 172 against off-targets. (n.i. = <10% inhibition; n.d. = not determined).**

| | **conc. [µM]** | **inhibition [%]** | |
|---|---|---|---|
| | | **170** | **172** |
| MMP-1 | 100 | 11 ± 0 | 18 ± 4 |
| MMP-2 | 100 | n.i. | 12 ± 2 |
| MMP-3 | 100 | n.i. | 18 ± 5 |
| MMP-7 | 100 | n.d. | n.d. |
| MMP-8 | 100 | n.d. | n.d. |
| MMP-14 | 100 | n.d. | n.d. |
| ADAM17 (TACE) | 100 | n.i. | n.i. |
| HDAC-3 | 100 | n.d. | n.d. |
| HDAC-8 | 100 | n.d. | n.d. |
| COX-1 | 100 | n.i. | n.d. |

**Table 18. Cytotoxicity data and PA14 inhibition by examples 170 and 172**

| | **170 [µM]** | **172 [µM]** |
|---|---|---|
| HepG2 IC₅₀ | > 100 | > 100 |
| HEK293 IC₅₀ | > 100 | > 100 |
| A549 IC₅₀ | > 100 | > 100 |
| MIC PA14 | > 100 | > 100 |

As compounds of examples **63** and **170** showed an impressive activity in the *in vitro* LasB assay, high selectivity over a broad spectrum of human enzymes and no signs of cytotoxicity *in vitro,* they were subjected to a more advanced safety screening (SafetyScreen44 panel, performed by Eurofins CEREP). This screening comprises 44 different targets including GPCRs, transporters, ion channels, nuclear receptors, kinases and other non-kinase enzymes. Compounds of examples **63** and **170** demonstrated no inhibition of control specific binding (< 22% inhibition at a compound concentration of 1.0E-0.5 M) of all of the targets tested.

### Hydroxamic acid derivatives:

**Table 19. Hydroxamic acid derivatives and their corresponding activities against LasB. Example 173 was prepared according to procedures described above.**

| **Example** | **Structure** | **IC₅₀ [nM]** |
|---|---|---|
| **173** | 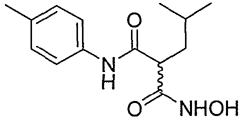 | 14 ± 1 |

**Table 20. Selectivity, cytotoxicity and PA14 inhibition for the compound of example 173.**

| | | | |
|---|---|---|---|
| **Selectivity** | **% inhibition at 100 µM** | **MMP-1** | 29 ± 3 |
| | | **MMP-2** | 26 ± 2 |
| | | **MMP-3** | 16 ± 7 |
| | **IC₅₀ (µM)** | **HDAC-3** | >100 |
| | | **HDAC-8** | >100 |
| | | **TACE** | 16 ± 2 |
| | | **COX-1** | >100 |
| **Cytotoxicity IC₅₀ (µM)** | | **HepG2** | >100 |
| | | **HEK293** | >100 |
| | | **A549** | >100 |
| **MIC PA14 (µM)** | | | >100 |

### Triazole derivatives:

**Table 21. Triazole derivatives and their corresponding activities against LasB. Examples 174 to 178 were prepared according to procedures described above.**

| **Example** | **Structure** | **IC₅₀ [µM]** |
|---|---|---|
| **174** | 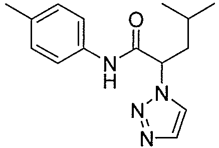 | 2.8 ± 0.1 |
| **175** | 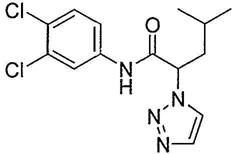 | 4.3 ± 0.2 |
| **176** | 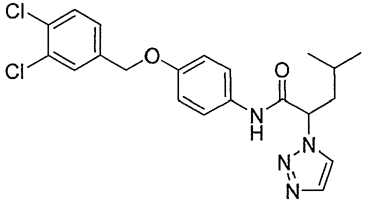 | 45.6* |
| **177** | 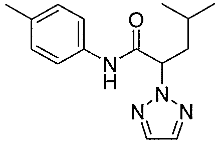 | 5.3 ± 0.2 |
| **178** | 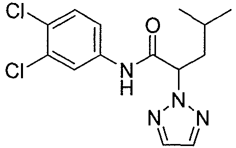 | 5.5 ± 0.2 |
| **179** | 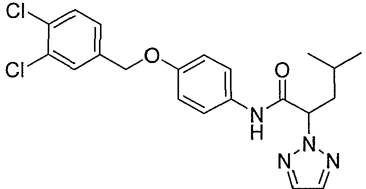 | 30.0* |

| | | |
|---|---|---|
| * n = 1 | | |

**Table 22. Selectivity, cytotoxicity and PA14 inhibition for the compounds of examples 174 and 177. n.i. = inhibition < 10%; n.d.= not determined**

| | | | **174** | **177** |
|---|---|---|---|---|
| **Selectivity** | **% inhibition at 100 µM** | **MMP-1** | n.i. | 7 ± 4 |
| | | **MMP-2** | n.i. | n.i. |
| | | **MMP-3** | -3 ± 17 | n.i. |
| | **IC₅₀ (µM)** | **HDAC-3** | >100 | >100 |
| | | **HDAC-8** | >100 | >100 |
| | | **TACE** | >100 | >100 |
| | | **COX-1** | >100 | >100 |
| **Cytotoxicity IC₅₀ (µM)** | | **HepG2** | >100 | >100 |
| | | **HEK293** | >100 | >100 |
| | | **A549** | >100 | >100 |
| **MIC PA14 (µM)** | | | >100 | >100 |

## Claims

1. A compound of formula (la): 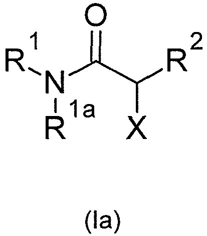 wherein
X is a group of formula -PO(OH)₂, -SH, -C(=O)-NH-OH, an optionally substituted triazolyl group, -SR³, -PO(OH)(OR⁴) or -PO(OR⁴)(OR⁵);
R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group; or a group of formula -CH(R⁶)-C(=O)-NH-R⁷, or a group of formula -C(Me)₂-CH₂-C(=O)-NH-R⁷, or a group of formula -CH(R⁶)-CH₂-C(=O)-NH-R⁷, or a group of formula -CH(R⁶)-R⁸;
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R³ is a group of formula -COR^{3a} or -CON(R^{3b})₂; wherein R^{3a} is an alkyl group, an optionally substituted phenyl group or an optionally substituted benzyl group and R^{3b} is independently selected from hydrogen or an alkyl group, an optionally substituted phenyl group or an optionally substituted benzyl group;
R⁴ is an alkyl group, an optionally substituted phenyl group or an optionally substituted benzyl group;
R⁵ is an alkyl group, an optionally substituted phenyl group or an optionally substituted benzyl group;
R⁶ is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R⁷ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group;
R⁸ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group; and
R^{1a} is hydrogen, or, if R¹ is a group of formula -CH(R⁶)-C(=O)-NH-R⁷, R¹⁸ and R⁶ together may be a group of formula -(CH₂)₃- or -(CH₂)₄-;
or a pharmaceutically acceptable salt thereof,
for use in the treatment of bacterial infections caused by P. *aeruginosa.*

2. The compound for use according to claim 1, wherein the compound is a compound of formula (I) 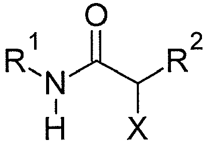 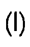 wherein X, R¹ and R² are as defined in claim 1, or a pharmaceutically acceptable salt thereof.

3. The compound for use according to claim 1, wherein the compound is a compound of formula (II), (III), (IV), (V) or (VI): 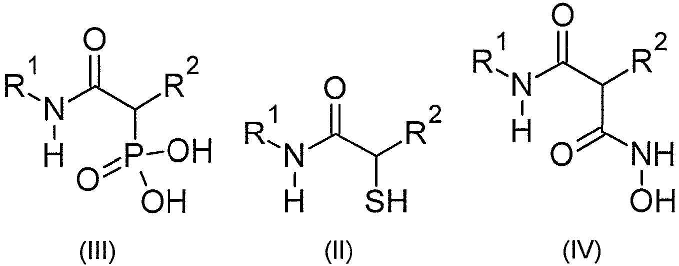 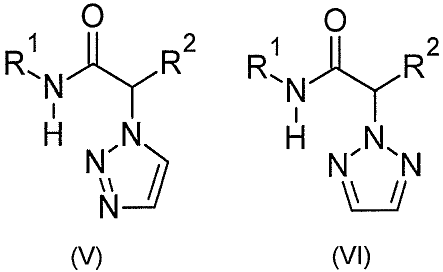 wherein R¹ and R² are as defined in claim 1, or a pharmaceutically acceptable salt thereof.

4. The compound for use according to any one of claims 1 to 3, wherein R² is a C₁₋₆ alkyl group; a heteroalkyl group containing from 1 to 6 carbon atoms and 1, 2, 3 or 4 heteroatoms selected from O, S and N; a C₄₋₁₀ alkylcycloalkyl group; or a C₇₋₁₂ aralkyl group; all of which may optionally be substituted;
especially wherein R² is an optionally substituted benzyl group, or a group of formula -CH₂CH(CH₃)₂.

5. The compound for use according to any one of claims 1 to 4, wherein R¹ is an optionally substituted aryl group or an optionally substituted heteroaryl group; especially wherein R¹ is an optionally substituted phenyl group, an optionally substituted naphthyl group or an optionally substituted heteroaryl group containing one or two rings and from 5 to 10 ring atoms selected from C, O, N and S;
further especially wherein R¹ is an optionally substituted phenyl group.

6. The compound for use according to any one of claims 1 to 4, wherein R¹ is a group of formula -Cy¹-L-Cy², wherein Cy¹ is an optionally substituted cycloalkylene group containing 1 or 2 rings and from 3 to 7 carbon ring atoms, an optionally substituted heterocycloalkylene group containing 1 or 2 rings and from 3 to 7 ring atoms selected from C, N, O and S, an optionally substituted phenylene group, or an optionally substituted heteroarylene group containing 5 or 6 ring atoms selected from C, N, O and S; Cy² is a cycloalkyl, heterocycloalkyl, alkylcycloalkyl, heteroalkylcycloalkyl, aryl, heteroaryl, aralkyl or heteroaralkyl group, all of which may optionally be substituted; and L is a bond or -O-, -S-, -NH-, -CH₂-, -CO-, -NHCO-, -CO-NH-, -CH₂-CO-NH-, -NH-CO-CH₂-, -CH₂-O-CO-NH-, -NH-CO-O-CH₂-, -O-CO-NH-, -NH-CO-O-, -NHSO₂-, - SO₂NH-, -CH₂-SO₂-NH-, -NH-SO₂-CH₂-, -S-CH₂-, -CH₂-S-, -NH-CH₂-, -CH₂-NH-, -O-CH₂- or-CH₂-O-;
especially wherein Cy² is an optionally substituted phenyl group, an optionally substituted biphenyl group, an optionally substituted naphthyl group, an optionally substituted heteroaryl group containing one or two rings and 5, 6, 9 or 10 ring atoms selected from C, O, N and S, an optionally substituted cycloalkyl group containing from 3 to 7 ring atoms, an optionally substituted heterocycloalkyl group containing from 3 to 7 ring atoms selected from C, N, O and S, an optionally substituted heterocycloalkylaryl group containing 9 or 10 ring atoms selected from C, N, S and O, or a group of formula -CH(CH₂Ph)Ph; and/or wherein L is a bond or -NHCO-, -CO-NH-, -CH₂-CO-NH-, -NH-CO-CH₂-, -NHSO₂- or -SO₂NH-; and/or wherein Cy¹ is a 1,4-phenylene group.

7. The compound for use according to any one of claims 1 to 4, wherein R¹ is a group of formula -CH(R⁶)-C(=O)-NH-R⁷ or a group of formula -CH(R⁶)-R⁸.

8. The compound for use according to claim 7, wherein R⁶ is hydrogen or a C₁₋₆ alkyl group, a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O, or a group of formula -CH₂-R^{6a}, wherein R^{6a} is a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O;
especially wherein R⁶ is a group of formula -CH(CH₃)₂.

9. The compound for use according to claim 7 or 8, wherein R⁷ is an optionally substituted phenyl group or an optionally substituted C₃₋₇ cycloalkyl group; and/or wherein R⁸ is an optionally substituted benzimidazole group or an optionally substituted triazole group or an optionally substituted imidazole group.

10. A compound of formula (III): 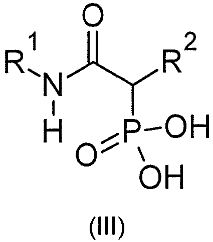 wherein
R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group; or a group of formula -CH(R⁶)-C(=O)-NH-R⁷, or a group of formula -C(Me)₂-CH₂-C(=O)-NH-R⁷, or a group of formula -CH(R⁶)-CH₂-C(=O)-NH-R⁷, or a group of formula -CH(R⁶)-R⁸;
R² is a group of formula -CH₂CH(CH₃)₂;
R⁶ is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R⁷ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group; and
R⁸ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group;
or a pharmaceutically acceptable salt thereof.

11. The compound according to claim 10, wherein R¹ is an optionally substituted aryl group or an optionally substituted heteroaryl group;
especially wherein R¹ is an optionally substituted phenyl group, an optionally substituted naphthyl group or an optionally substituted heteroaryl group containing one or two rings and from 5 to 10 ring atoms selected from C, O, N and S;
further especially wherein R¹ is an optionally substituted phenyl group.

12. The compound according to claim 10, wherein R¹ is a group of formula -Cy¹-L-Cy², wherein Cy¹ is an optionally substituted cycloalkylene group containing 1 or 2 rings and from 3 to 7 carbon ring atoms, an optionally substituted heterocycloalkylene group containing 1 or 2 rings and from 3 to 7 ring atoms selected from C, N, O and S, an optionally substituted phenylene group, or an optionally substituted heteroarylene group containing 5 or 6 ring atoms selected from C, N, O and S; Cy² is a cycloalkyl, heterocycloalkyl, alkylcycloalkyl, heteroalkylcycloalkyl, aryl, heteroaryl, aralkyl or heteroaralkyl group, all of which may optionally be substituted; and L is a bond or -O-, -S-, -NH-, -CH₂-, -CO-, - NHCO-, -CO-NH-, -CH₂-CO-NH-, -NH-CO-CH₂-, -CH₂-O-CO-NH-, -NH-CO-O-CH₂-, -O-CO-NH-, -NH-CO-O-, -NHSO₂-, -SO₂NH-, -CH₂-SO₂-NH-, -NH-SO₂-CH₂-, -S-CH₂-, -CH₂-S-, -NH-CH₂-, -CH₂-NH-, -O-CH₂- or -CH₂-O-;
especially wherein Cy² is an optionally substituted phenyl group, an optionally substituted biphenyl group, an optionally substituted naphthyl group, an optionally substituted heteroaryl group containing one or two rings and 5, 6, 9 or 10 ring atoms selected from C, O, N and S, an optionally substituted cycloalkyl group containing from 3 to 7 ring atoms, an optionally substituted heterocycloalkyl group containing from 3 to 7 ring atoms selected from C, N, O and S, an optionally substituted heterocycloalkylaryl group containing 9 or 10 ring atoms selected from C, N, S and O, or a group of formula -CH(CH₂Ph)Ph; and/or wherein L is a bond or -NHCO-, -CO-NH-, -CH₂-CO-NH-, -NH-CO-CH₂-, -NHSO₂- or -SO₂NH-; and/or wherein Cy¹ is a 1,4-phenylene group.

13. The compound according to claim 10, wherein R¹ is a group of formula -CH(R⁶)-C(=O)-NH-R⁷ or a group of formula -CH(R⁶)-R⁸;
especially wherein R⁶ is hydrogen or a C₁₋₆ alkyl group, a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O, or a group of formula -CH₂-R^{6a}, wherein R^{6a} is a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O; and/or wherein R⁷ is an optionally substituted phenyl group or an optionally substituted C₃₋₇ cycloalkyl group;
and/or wherein R⁸ is an optionally substituted benzimidazole group or an optionally substituted triazole group or an optionally substituted imidazole group.

14. Pharmaceutical composition comprising a compound according to anyone of the preceding claims 10 to 13 and optionally one or more carrier substances and/or one or more adjuvants and/or one or more further antibacterial compounds.

15. Compound according to any one of claims 10 to 13 or pharmaceutical composition according to claim 14 for use in the treatment of bacterial infections.

## Patentansprüche

1. Verbindung der Formel (la): 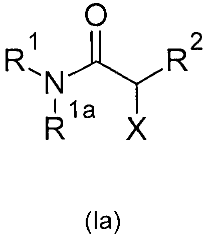 wobei
X eine Gruppe der Formel -PO(OH)₂, -SH, -C(=O)-NH-OH, optional eine substituierte Triazolylgruppe, -SR³, -PO(OH)(OR⁴) oder -PO(OR⁴(OR⁵) ist;
R¹ eine optional substituierte Cycloalkylgruppe, eine optional substituierte Heterocycloalkylgruppe, eine optional substituierte Arylgruppe oder eine optional substituierte Heteroarylgruppe oder eine optional substituierte Aralkylgruppe oder eine optional substituierte Heteroaralkylgruppe; oder eine Gruppe der Formel -CH(R⁶)-C(=O)-NH-R⁷ oder eine Gruppe der Formel - C(Me)₂ -CH₂ -C(=O)-NH-R⁷ oder eine Gruppe der Formel -CH(R⁶)-CH₂-C(=O)-NH-R⁷ oder eine Gruppe der Formel -CH(R⁶ )-R⁸ ist;
R² eine Alkyl-, Alkenyl-, Alkinyl-, Heteroalkyl-, Cycloalkyl-, Heterocycloalkyl-, Alkylcycloalkyl-, Heteroalkylcycloalkyl-, Aryl-, Heteroaryl-, Aralkyl- oder Heteroaralkylgruppe ist, die alle optional substituiert sein können;
R³ eine Gruppe der Formel -COR^{3a} oder -CON(R³⁶)₂ ist; wobei R^{3a} eine Alkylgruppe, eine optional substituierte Phenylgruppe oder eine optional substituierte Benzylgruppe ist und R^{3b} unabhängig aus Wasserstoff oder einer Alkylgruppe, einer optional substituierten Phenylgruppe oder einer optional substituierten Benzylgruppe ausgewählt ist;
R⁴ eine Alkylgruppe, eine optional substituierte Phenylgruppe oder eine optional substituierte Benzylgruppe ist;
R⁵ eine Alkylgruppe, eine optional substituierte Phenylgruppe oder eine optional substituierte Benzylgruppe ist;
R⁶ Wasserstoff oder eine Alkyl-, Alkenyl-, Alkinyl-, Heteroalkyl-, Cycloalkyl-, Heterocycloalkyl-, Alkylcycloalkyl-, Heteroalkylcycloalkyl-, Aryl-, Heteroaryl-, Aralkyl- oder Heteroaralkylgruppe ist, die alle optional substituiert sein können;
R⁷ eine optional substituierte Cycloalkylgruppe, eine optional substituierte Heterocycloalkylgruppe, eine optional substituierte Arylgruppe, eine optional substituierte Heteroarylgruppe, eine optional substituierte Aralkylgruppe oder eine optional substituierte Heteroaralkylgruppe ist;
R⁸ eine optional substituierte Cycloalkylgruppe, eine optional substituierte Heterocycloalkylgruppe, eine optional substituierte Arylgruppe, eine optional substituierte Heteroarylgruppe, eine optional substituierte Aralkylgruppe oder eine optional substituierte Heteroaralkylgruppe ist; und
R^{1a} Wasserstoff ist oder, wenn R¹ eine Gruppe der Formel -CH(R⁶)-C(=O)-NH-R⁷ ist, R^{1a} und R⁶ zusammen eine Gruppe der Formel -(CH₂)₃- oder -(CH₂ )₄-sein können;
oder ein pharmazeutisch akzeptables Salz davon,
zur Verwendung bei der Behandlung von bakteriellen Infektionen, die durch P. *aeruginosa* verursacht werden.

2. Verbindung zur Verwendung gemäß Anspruch 1, wobei die Verbindung eine Verbindung der Formel (I) ist 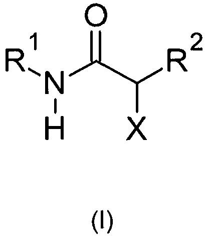 wobei X, R¹ und R² wie in Anspruch 1 definiert sind, oder ein pharmazeutisch akzeptables Salz davon.

3. Verbindung zur Verwendung gemäß Anspruch 1, wobei die Verbindung eine Verbindung der Formel (II), (III), (IV), (V) oder (VI) ist: 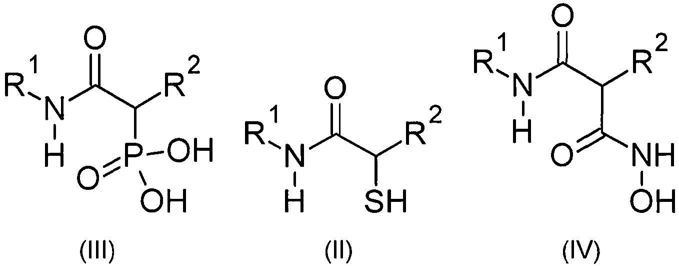 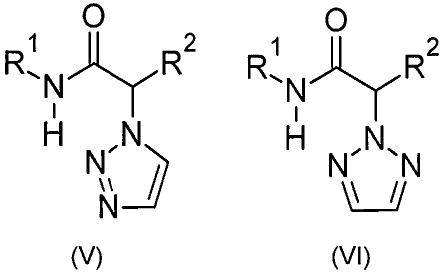 wobei R¹ und R² wie in Anspruch 1 definiert sind, oder ein pharmazeutisch akzeptables Salz davon.

4. Verbindung zur Verwendung gemäß einem der Ansprüche 1 bis 3, wobei R² eine C₁₋₆-Alkylgruppe; eine Heteroalkylgruppe, die 1 bis 6 Kohlenstoffatome und 1, 2, 3 oder 4 Heteroatome enthält, ausgewählt aus O, S und N; eine C₄₋₁₀)-Alkylcycloalkylgruppe; oder eine C₇₋₁₂-Aralkylgruppe ist; wobei alle optional substituiert sein können;
insbesondere wobei R² eine optional substituierte Benzylgruppe oder eine Gruppe der Formel -CH₂CH(CH₃)₂ ist.

5. Verbindung zur Verwendung gemäß einem der Ansprüche 1 bis 4, wobei R¹ eine optional substituierte Arylgruppe oder eine optional substituierte Heteroarylgruppe ist;
insbesondere wobei R¹ eine optional substituierte Phenylgruppe, eine optional substituierte Naphthylgruppe oder eine optional substituierte Heteroarylgruppe mit einem oder zwei Ringen und 5 bis 10 Ringatomen, ausgewählt aus C, O, N und S, ist;
weiter insbesondere, wobei R¹ eine optional substituierte Phenylgruppe ist.

6. Verbindung zur Verwendung gemäß einem der Ansprüche 1 bis 4, wobei R¹ eine Gruppe der Formel -Cy¹-L-Cy² ist, wobei Cy¹ eine optional substituierte Cycloalkylengruppe mit 1 oder 2 Ringen und 3 bis 7 Kohlenstoffringatomen, eine optional substituierte Heterocycloalkylengruppe mit 1 oder 2 Ringen und 3 bis 7 Ringatomen, ausgewählt aus C, N, O und S, eine optional substituierte Phenylengruppe oder eine optional substituierte Heteroarylengruppe mit 5 oder 6 Ringatomen, ausgewählt aus C, N, O und S, enthält; Cy² eine Cycloalkyl-, Heterocycloalkyl-, Alkylcycloalkyl-, Heteroalkylcycloalkyl-, Aryl-, Heteroaryl-, Aralkyl- oder Heteroaralkylgruppe ist, die alle optional substituiert sein können; und L eine Bindung oder -O-, -S-, -NH-, -CH₂ -, -CO-, -NHCO-, -CO-NH-, -CH₂ -CO-NH-, -NH-CO-CH₂-, -CH₂-O-CO-NH-, -NH-CO-O-CH₂-, -O-CO-NH-, -NH-CO-O-, -NHSO₂-, -SO₂NH-, -CH₂-SO₂-NH-, -NH-SO₂-CH₂-, -S-CH₂-, -CH₂-S-, - NH-CH₂-, -CH₂-NH-, -O-CH₂ - oder -CH₂ -O- ist;
insbesondere wobei Cy² eine optional substituierte Phenylgruppe, eine optional substituierte Biphenylgruppe, eine optional substituierte Naphthylgruppe, eine optional substituierte Heteroarylgruppe mit einem oder zwei Ringen und 5, 6, 9 oder 10 Ringatomen, ausgewählt aus C, O, N und S, eine optional substituierte Cycloalkylgruppe mit 3 bis 7 Ringatomen, eine optional substituierte Heterocycloalkylgruppe mit 3 bis 7 Ringatomen, ausgewählt aus C, N, O und S, eine optional substituierte Heterocycloalkylarylgruppe mit 9 oder 10 Ringatomen, ausgewählt aus C, N, S und O, oder eine Gruppe der Formel - CH(CH₂ Ph)Ph; und/oder wobei L eine Bindung oder -NHCO-, -CO-NH-, -CH₂-CO-NH-, -NH-CO-CH₂ -, -NHSO₂- oder -SO₂ NH- ist; und/oder wobei Cy¹ eine 1,4-Phenylengruppe ist.

7. Verbindung zur Verwendung gemäß einem der Ansprüche 1 bis 4, wobei R¹ eine Gruppe der Formel -CH(R⁶)-C(=O)-NH-R⁷ oder eine Gruppe der Formel - CH(R⁶)-R⁸ ist.

8. Verbindung zur Verwendung gemäß Anspruch 7, wobei R⁶ Wasserstoff oder eine C₁₋₆-Alkylgruppe, eine C₃₋₇-Cycloalkylgruppe, eine Heterocycloalkylgruppe mit 3 bis 7 Ringatomen, ausgewählt aus C, N, O und S, eine Phenylgruppe oder eine Heteroarylgruppe mit 5 oder 6 Ringatomen, ausgewählt aus C, N, S und O, oder eine Gruppe der Formel -CH₂-R⁶⁸, wobei R^{6a} eine C₃₋₇-Cycloalkylgruppe, eine Heterocycloalkylgruppe mit 3 bis 7 Ringatomen, ausgewählt aus C, N, O und S, eine Phenylgruppe oder eine Heteroarylgruppe mit 5 oder 6 Ringatomen, ausgewählt aus C, N, S und O, ist;
wobei insbesondere R⁶ eine Gruppe der Formel -CH(CH₃)₂ ist.

9. Verbindung zur Verwendung gemäß Anspruch 7 oder 8, wobei R⁷ eine optional substituierte Phenylgruppe oder eine optional substituierte C₃₋₇-Cycloalkylgruppe ist; und/oder wobei R⁸ eine optional substituierte Benzimidazolgruppe oder eine optional substituierte Triazolgruppe oder eine optional substituierte Imidazolgruppe ist.

10. Verbindung der Formel (III): 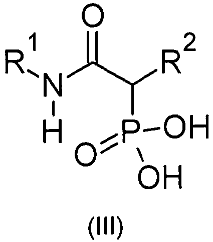 wobei
R¹ eine optional substituierte Cycloalkylgruppe, eine optional substituierte Heterocycloalkylgruppe, eine optional substituierte Arylgruppe oder eine optional substituierte Heteroarylgruppe oder eine optional substituierte Aralkylgruppe oder eine optional substituierte Heteroaralkylgruppe; oder eine Gruppe der Formel -CH(R⁶)-C(=O)-NH-R⁷ oder eine Gruppe der Formel - C(Me)₂-CH₂-C(=O)-NH-R⁷ oder eine Gruppe der Formel -CH(R⁶)-CH₂-C(=O)-NH-R⁷ oder eine Gruppe der Formel -CH(R⁶)-R⁸ ist;
R² eine Gruppe der Formel -CH₂CH(CH₃)₂ ist;
R⁶ Wasserstoff oder eine Alkyl-, Alkenyl-, Alkinyl-, Heteroalkyl-, Cycloalkyl-, Heterocycloalkyl-, Alkylcycloalkyl-, Heteroalkylcycloalkyl-, Aryl-, Heteroaryl-, Aralkyl- oder Heteroaralkylgruppe ist, die alle optional substituiert sein können;
R⁷ eine optional substituierte Cycloalkylgruppe, eine optional substituierte Heterocycloalkylgruppe, eine optional substituierte Arylgruppe, eine optional substituierte Heteroarylgruppe, eine optional substituierte Aralkylgruppe oder eine optional substituierte Heteroaralkylgruppe ist; und
R⁸ eine optional substituierte Cycloalkylgruppe, eine optional substituierte Heterocycloalkylgruppe, eine optional substituierte Arylgruppe, eine optional substituierte Heteroarylgruppe, eine optional substituierte Aralkylgruppe oder eine optional substituierte Heteroaralkylgruppe ist;
oder ein pharmazeutisch akzeptables Salz davon.

11. Verbindung gemäß Anspruch 10, wobei R¹ eine optional substituierte Arylgruppe oder eine optional substituierte Heteroarylgruppe ist;
insbesondere wobei R¹ eine optional substituierte Phenylgruppe, eine optional substituierte Naphthylgruppe oder eine optional substituierte Heteroarylgruppe mit einem oder zwei Ringen und 5 bis 10 Ringatomen, ausgewählt aus C, O, N und S, ist;
weiter insbesondere, wobei R¹ eine optional substituierte Phenylgruppe ist.

12. Verbindung nach Anspruch 10, wobei R¹ eine Gruppe der Formel -Cy¹-L-Cy² ist, wobei Cy¹ eine optional substituierte Cycloalkylengruppe mit 1 oder 2 Ringen und 3 bis 7 Kohlenstoffringatomen, eine optional substituierte Heterocycloalkylengruppe mit 1 oder 2 Ringen und 3 bis 7 Ringatomen, ausgewählt aus C, N, O und S, eine optional substituierte Phenylengruppe oder eine optional substituierte Heteroarylengruppe mit 5 oder 6 Ringatomen, ausgewählt aus C, N, O und S ist; Cy² eine Cycloalkyl-, Heterocycloalkyl-, Alkylcycloalkyl-, Heteroalkylcycloalkyl-, Aryl-, Heteroaryl-, Aralkyl- oder Heteroaralkylgruppe ist, die alle optional substituiert sein können; und L eine Bindung oder -O-, -S-, -NH-, -CH₂ -, -CO-, -NHCO-, -CO-NH-, -CH₂-CO-NH-, - NH-CO-CH₂ -, -CH₂-O-CO-NH-, -NH-CO-O-CH₂ -, -O-CO-NH-, -NH-CO-O-, - NHSO₂-, -SO₂NH-, -CH₂-SO₂-NH-, -NH-SO₂-CH₂ -, -S-CH₂ -, -CH₂-S-, -NH-CH₂-, -CH₂-NH-, -O-CH₂- oder -CH₂-O- ist;
insbesondere wobei Cy² eine optional substituierte Phenylgruppe, eine optional substituierte Biphenylgruppe, eine optional substituierte Naphthylgruppe, eine optional substituierte Heteroarylgruppe mit einem oder zwei Ringen und 5, 6, 9 oder 10 Ringatomen, ausgewählt aus C, O, N und S, eine optional substituierte Cycloalkylgruppe mit 3 bis 7 Ringatomen, eine optional substituierte Heterocycloalkylgruppe mit 3 bis 7 Ringatomen, ausgewählt aus C, N, O und S, eine optional substituierte Heterocycloalkylarylgruppe mit 9 oder 10 Ringatomen, ausgewählt aus C, N, S und O, oder eine Gruppe der Formel - CH(CH₂Ph)Ph ist; und/oder wobei L eine Bindung oder -NHCO-, -CO-NH-, - CH₂-CO-NH-, -NH-CO-CH₂-, -NHSO₂- oder -SO₂NH- ist; und/oder wobei Cy¹ eine 1,4-Phenylengruppe ist.

13. Verbindung gemäß Anspruch 10, wobei R¹ eine Gruppe der Formel -CH(R⁶)-C(=O)-NH-R⁷ oder eine Gruppe der Formel -CH(R⁶)-R⁸ ist;
insbesondere wobei R⁶ Wasserstoff oder eine C₁₋₆ Alkylgruppe, eine C₃₋₇ Cycloalkylgruppe, eine Heterocycloalkylgruppe mit 3 bis 7 Ringatomen, ausgewählt aus C, N, O und S, eine Phenylgruppe oder eine Heteroarylgruppe mit 5 oder 6 Ringatomen, ausgewählt aus C, N, S und O, oder eine Gruppe der Formel -CH-R^{6a}, wobei R^{6a} eine C₃₋₇-Cycloalkylgruppe, eine Heterocycloalkylgruppe mit 3 bis 7 Ringatomen, ausgewählt aus C, N, O und S, eine Phenylgruppe oder eine Heteroarylgruppe mit 5 oder 6 Ringatomen, ausgewählt aus C, N, S und O ist; und/oder wobei R⁷ eine optional substituierte Phenylgruppe oder eine optional substituierte C₃₋₇-Cycloalkylgruppe ist; und/oder wobei R⁸ eine optional substituierte Benzimidazolgruppe oder eine optional substituierte Triazolgruppe oder eine optional substituierte Imidazolgruppe ist.

14. Pharmazeutische Zusammensetzung, umfassend eine Verbindung gemäß einem der vorstehenden Ansprüche 10 bis 13 und optional eine oder mehrere Trägersubstanzen und/oder ein oder mehrere Hilfsstoffe und/oder eine oder mehrere weitere antibakterielle Verbindungen.

15. Verbindung gemäß einem der Ansprüche 10 bis 13 oder pharmazeutische Zusammensetzung gemäß Anspruch 14 zur Verwendung bei der Behandlung von bakteriellen Infektionen.

## Revendications

1. Composé de formule (la): 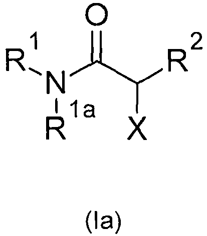 dans laquelle
X est un groupe de formule -PO(OH)₂, -SH, -C(=O)-NH-OH, un groupe triazolyle éventuellement substitué, -SR³, -PO(OH)(OR⁴) ou -PO(OR⁴)(OR⁵);
R¹ est un groupe cycloalkyle éventuellement substitué, un groupe hétérocycloalkyle éventuellement substitué, un groupe aryle éventuellement substitué ou un groupe hétéroaryle éventuellement substitué ou un groupe aralkyle éventuellement substitué ou un groupe hétéroaralkyle éventuellement substitué; ou un groupe de formule -CH(R⁶)-C(=O)-NH-R⁷, ou un groupe de formule -C(Me)₂-CH₂-C(=O)-NH-R⁷, ou un groupe de formule -CH(R⁶)-CH₂-C(=O)-NH-R⁷, ou un groupe de formule -CH(R⁶)-R⁸;
R² est un groupe alkyle, alcényle, alcynyle, hétéroalkyle, cycloalkyle, hétérocycloalkyle, alkylcycloalkyle, hétéroalkylcycloalkyle, aryle, hétéroaryle, aralkyle ou hétéroaralkyle, qui peuvent tous être éventuellement substitués;
R³ est un groupe de formule -COR^{3a} ou -CON(R^{3b})₂; où R^{3a} est un groupe alkyle, un groupe phényle éventuellement substitué ou un groupe benzyle éventuellement substitué et R^{3b} est indépendamment choisi parmi l'hydrogène ou un groupe alkyle, un groupe phényle éventuellement substitué ou un groupe benzyle éventuellement substitué;
R⁴ est un groupe alkyle, un groupe phényle éventuellement substitué ou un groupe benzyle éventuellement substitué;
R⁵ est un groupe alkyle, un groupe phényle éventuellement substitué ou un groupe benzyle éventuellement substitué;
R⁶ est un atome d'hydrogène ou un groupe alkyle, alcényle, alcynyle, hétéroalkyle, cycloalkyle, hétérocycloalkyle, alkylcycloalkyle, hétéroalkylcycloalkyle, aryle, hétéroaryle, aralkyle ou hétéroaralkyle, qui peuvent tous être éventuellement substitués;
R⁷ est un groupe cycloalkyle éventuellement substitué, un groupe hétérocycloalkyle éventuellement substitué, un groupe aryle éventuellement substitué, un groupe hétéroaryle éventuellement substitué, un groupe aralkyle éventuellement substitué ou un groupe hétéroaralkyle éventuellement substitué;
R⁸ est un groupe cycloalkyle éventuellement substitué, un groupe hétérocycloalkyle éventuellement substitué, un groupe aryle éventuellement substitué, un groupe hétéroaryle éventuellement substitué, un groupe aralkyle éventuellement substitué ou un groupe hétéroaralkyle éventuellement substitué; et
R^{1a} est un atome d'hydrogène, ou, si R¹ est un groupe de formule -CH(R⁶)-C(=O)-NH-R⁷, R^{1a} et R⁶ peuvent former ensemble un groupe de formule -(CH₂)₃- ou -(CH₂)₄-;
ou un sel pharmaceutiquement acceptable de celui-ci,
destiné à être utilisé dans le traitement des infections bactériennes causées par *P. aeruginosa.*

2. Composé destiné à être utilisé selon la revendication 1, dans lequel le composé est un composé de formule (I) 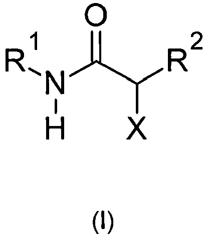 dans laquelle X, R¹ et R² sont tels que définis dans la revendication 1, ou un sel pharmaceutiquement acceptable de celui-ci.

3. Composé destiné à être utilisé selon la revendication 1, dans lequel le composé est un composé de formule (II), (III), (IV), (V) ou (VI): 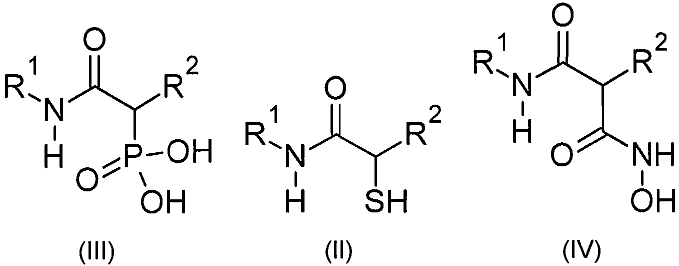 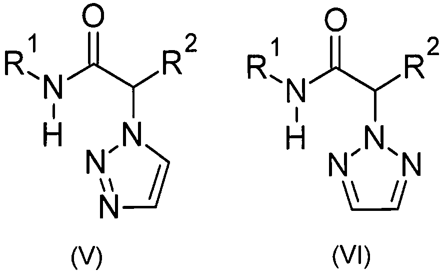 dans laquelle R¹ et R² sont tels que définis dans la revendication 1, ou un sel pharmaceutiquement acceptable de celui-ci.

4. Composé destiné à être utilisé selon l'une quelconque des revendications 1 à 3, dans lequel R² est un groupe alkyle C₁₋₆; un groupe hétéroalkyle contenant de 1 à 6 atomes de carbone et 1, 2, 3 ou 4 hétéroatomes choisis parmi O, S et N; un groupe alkylcycloalkyle; ou un groupe aralkyle en C₇₋₁₂; tous pouvant éventuellement être substitués;
en particulier dans lequel R² est un groupe benzyle éventuellement substitué, ou un groupe de formule -CH₂ CH(CH₃)₂.

5. Composé destiné à être utilisé selon l'une quelconque des revendications 1 à 4, dans lequel R¹ est un groupe aryle éventuellement substitué ou un groupe hétéroaryle éventuellement substitué;
en particulier dans lequel R¹ est un groupe phényle éventuellement substitué, un groupe naphtyle éventuellement substitué ou un groupe hétéroaryle éventuellement substitué contenant un ou deux cycles et de 5 à 10 atomes cycliques choisis parmi C, O, N et S;
en particulier dans lequel R¹ est un groupe phényle éventuellement substitué.

6. Composé destiné à être utilisé selon l'une quelconque des revendications 1 à 4, dans lequel R¹ est un groupe de formule -Cy¹-L-Cy² , dans lequel Cy¹ est un groupe cycloalkylène éventuellement substitué contenant 1 ou 2 cycles et de 3 à 7 atomes de carbone cycliques, un groupe hétérocycloalkylène éventuellement substitué contenant 1 ou 2 cycles et de 3 à 7 atomes cycliques choisis parmi C, N, O et S, un groupe phénylène éventuellement substitué, ou un groupe hétéroarylène éventuellement substitué contenant 5 ou 6 atomes cycliques choisis parmi C, N, O et S; Cy² est un groupe cycloalkyle, hétérocycloalkyle, alkylcycloalkyle, hétéroalkylcycloalkyle, aryle, hétéroaryle, aralkyle ou hétéroaralkyle, qui peuvent tous être éventuellement substitués; et L est une liaison ou -O-, -S-, -NH-, -CH₂-, -CO-, -NHCO-, -CO-NH-, -CH₂-CO-NH-, -NH-CO-CH₂-, -CH₂-O-CO-NH-, -NH-CO-O-CH₂-, -O-CO-NH-, -NH-CO-O-, -NHSO₂-, -SO₂NH-, -CH₂-SO₂-NH-, -NH-SO₂-CH₂-, -S-CH₂-, -CH₂-S, -NH-CH₂-, -CH₂-NH-, -O-CH₂- ou -CH₂-O-;
en particulier dans lequel Cy² est un groupe phényle éventuellement substitué, un groupe biphényle éventuellement substitué, un groupe naphtyle éventuellement substitué, un groupe hétéroaryle éventuellement substitué contenant un ou deux cycles et 5, 6, 9 ou 10 atomes cycliques choisis parmi C, O, N et S, un groupe cycloalkyle éventuellement substitué contenant de 3 à 7 atomes cycliques, un groupe hétérocycloalkyle éventuellement substitué contenant de 3 à 7 atomes cycliques choisis parmi C, N, O et S, un groupe hétérocycloalkylaryle éventuellement substitué contenant 9 ou 10 atomes cycliques choisis parmi C, N, S et O, ou un groupe de formule -CH(CH₂ Ph)Ph; et/ou dans lequel L est une liaison ou -NHCO-, -CO-NH-, -CH₂-CO-NH-, -NH-CO-CH₂-, -NHSO₂- ou -SO₂NH-; et/ou dans lequel Cy¹ est un groupe 1,4-phénylène.

7. Composé destiné à être utilisé selon l'une des revendications 1 à 4, dans lequel R¹ est un groupe de formule -CH(R⁶)-C(=O)-NH-R⁷ ou un groupe de formule - CH(R⁶)-R⁸.

8. Composé destiné à être utilisé selon la revendication 7, dans lequel R⁶ est un atome d'hydrogène ou un groupe alkyle en C₁₋₆ , un groupe cycloalkyl en C₃₋₇, un groupe hétérocycloalkyle contenant de 3 à 7 atomes cycliques choisis parmi C, N, O et S, un groupe phényle ou un groupe hétéroaryle contenant 5 ou 6 atomes cycliques choisis parmi C, N, S et O, ou un groupe de formule -CH₂-R^{6a}, dans lequel R^{6a} est un groupe cycloalkyle en C₃₋₇, un groupe hétérocycloalkyle contenant de 3 à 7 atomes cycliques choisis parmi C, N, O et S, un groupe phényle ou un groupe hétéroaryle contenant 5 ou 6 atomes cycliques choisis parmi C, N, S et O;
en particulier dans lequel R⁶ est un groupe de formule -CH(CH₃)₂.

9. Composé destiné à être utilisé selon la revendication 7 ou 8, dans lequel R⁷ est un groupe phényle éventuellement substitué ou un groupe cycloalkyle en C₃₋₇ éventuellement substitué; et/ou dans lequel R⁸ est un groupe benzimidazole éventuellement substitué ou un groupe triazole éventuellement substitué ou un groupe imidazole éventuellement substitué.

10. Composé de formule (III): 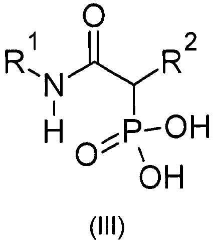 dans laquelle
R¹ est un groupe cycloalkyle éventuellement substitué, un groupe hétérocycloalkyle éventuellement substitué, un groupe aryle éventuellement substitué ou un groupe hétéroaryle éventuellement substitué ou un groupe aralkyle éventuellement substitué ou un groupe hétéroaralkyle éventuellement substitué; ou un groupe de formule -CH(R⁶)-C(=O)-NH-R⁷, ou un groupe de formule -C(Me)₂-CH₂-C(=O)-NH-R⁷, ou un groupe de formule -CH(R⁶)-CH₂-C(=O)-NH-R⁷, ou un groupe de formule -CH(R⁶)-R⁸;
R² est un groupe de formule -CH₂CH(CH₃)₂;
R⁶ est un atome d'hydrogène ou un groupe alkyle, alcényle, alcynyle, hétéroalkyle, cycloalkyle, hétérocycloalkyle, alkylcycloalkyle, hétéroalkylcycloalkyle, aryle, hétéroaryle, aralkyle ou hétéroaralkyle, qui peuvent tous être éventuellement substitués;
R⁷ est un groupe cycloalkyle éventuellement substitué, un groupe hétérocycloalkyle éventuellement substitué, un groupe aryle éventuellement substitué, un groupe hétéroaryle éventuellement substitué, un groupe aralkyle éventuellement substitué ou un groupe hétéroaralkyle éventuellement substitué; et
R⁸ est un groupe cycloalkyle éventuellement substitué, un groupe hétérocycloalkyle éventuellement substitué, un groupe aryle éventuellement substitué, un groupe hétéroaryle éventuellement substitué, un groupe aralkyle éventuellement substitué ou un groupe hétéroaralkyle éventuellement substitué;
ou un sel pharmaceutiquement acceptable de celui-ci.

11. Composé selon la revendication 10, dans lequel R¹ est un groupe aryle éventuellement substitué ou un groupe hétéroaryle éventuellement substitué; en particulier dans lequel R¹ est un groupe phényle éventuellement substitué, un groupe naphtyle éventuellement substitué ou un groupe hétéroaryle éventuellement substitué contenant un ou deux cycles et de 5 à 10 atomes cycliques choisis parmi C, O, N et S;
en particulier dans lequel R¹ est un groupe phényle éventuellement substitué.

12. Composé selon la revendication 10, dans lequel R¹ est un groupe de formule - Cy¹-L-Cy², dans lequel Cy¹ est un groupe cycloalkylène éventuellement substitué contenant 1 ou 2 cycles et de 3 à 7 atomes de carbone cycliques, un groupe hétérocycloalkylène éventuellement substitué contenant 1 ou 2 cycles et de 3 à 7 atomes cycliques choisis parmi C, N, O et S, un groupe phénylène éventuellement substitué, ou un groupe hétéroarylène éventuellement substitué contenant 5 ou 6 atomes cycliques choisis parmi C, N, O et S; Cy² est un groupe cycloalkyle, hétérocycloalkyle, alkylcycloalkyle, hétéroalkylcycloalkyle, aryle, hétéroaryle, aralkyle ou hétéroaralkyle, qui peuvent tous être éventuellement substitués; et L est une liaison ou -O-, -S-, -NH-, -CH₂ -, -CO-, -NHCO-, -CO-NH-, -CH₂-CO-NH-, -NH-CO-CH₂ -, -CH₂-O-CO-NH-, -NH-CO-O-CH₂-, -O-CO-NH-, -NH-CO-O-, -NHSO₂ -, - SO₂NH-, -CH₂-SO₂ -NH-, -NH-SO₂ -CH₂-, -S-CH₂- , -CH₂-S-, -NH-CH₂-, -CH₂ -NH-, -O-CH₂- ou -CH₂-O ;
en particulier dans lequel Cy² est un groupe phényle éventuellement substitué, un groupe biphényle éventuellement substitué, un groupe naphtyle éventuellement substitué, un groupe hétéroaryle éventuellement substitué contenant un ou deux cycles et 5, 6, 9 ou 10 atomes cycliques choisis parmi C, O, N et S, un groupe cycloalkyle éventuellement substitué contenant de 3 à 7 atomes cycliques, un groupe hétérocycloalkyle éventuellement substitué contenant de 3 à 7 atomes cycliques choisis parmi C, N, O et S, un groupe hétérocycloalkylaryle éventuellement substitué contenant 9 ou 10 atomes cycliques choisis parmi C, N, S et O, ou un groupe de formule -CH(CH₂ Ph)Ph; et/ou dans lequel L est une liaison ou -NHCO-, -CO-NH-, -CH₂-CO-NH-, -NH-CO-CH₂-, -NHSO₂- ou -SO₂NH-; et/ou dans lequel Cy¹ est un groupe 1,4-phénylène.

13. Composé selon la revendication 10, dans lequel R¹ est un groupe de formule - CH(R⁶)-C(=O)-NH-R⁷ ou un groupe de formule -CH(R⁶)-R⁸;
en particulier dans lequel R⁶ est un atome d'hydrogène ou un groupe alkyle en C₁₋₆, un groupe cycloalkyle en C₃₋₇, un groupe hétérocycloalkyle contenant de 3 à 7 atomes cycliques choisis parmi C, N, O et S, un groupe phényle ou un groupe hétéroaryle contenant 5 ou 6 atomes cycliques choisis parmi C, N, S et O, ou un groupe de formule -CH₂-R^{6a}, dans lequel R^{6a} est un groupe cycloalkyle en C₃₋₇, un groupe hétérocycloalkyle contenant de 3 à 7 atomes cycliques choisis parmi C, N, O et S, un groupe phényle ou un groupe hétéroaryle contenant 5 ou 6 atomes cycliques choisis parmi C, N, S et O; et/ou dans lequel R⁷ est un groupe phényle éventuellement substitué ou un groupe cycloalkyle en C₃₋₇ éventuellement substitué; et/ou dans lequel R⁸ est un groupe benzimidazole éventuellement substitué ou un groupe triazole éventuellement substitué ou un groupe imidazole éventuellement substitué.

14. Composition pharmaceutique comprenant un composé selon l'une quelconque des revendications 10 à 13 précédentes et éventuellement une ou plusieurs substances vectrices et/ou un ou plusieurs adjuvants et/ou un ou plusieurs autres composés antibactériens.

15. Composé selon l'une quelconque des revendications 10 à 13 ou composition pharmaceutique selon la revendication 14 destinée à être utilisée dans le traitement d'infections bactériennes.
